# EUROPÄISCHE PATENTSCHRIFT

(11) **EP 1 487 255 B1**
(45) Veröffentlichungstag und Bekanntmachung des Hinweises auf die Patenterteilung: **29.12.2010**
(21) Anmeldenummer: 03744375.1
(22) Anmeldetag: 18.03.2003
(51) Int. Cl.: A01H 1/06

(54) **POPULATION TRANSGENER PFLANZEN, DAVON ABGELEITETES BIOLOGISCHES MATERIAL, ENTSPRECHENDE PLASMIDKOLLEKTION UND POPULATION TRANSFORMIERTER WIRTSORGANISMEN, SOWIE DEREN VERWENDUNG UND VERFAHREN ZU DEREN ERZEUGUNG**
POPULATION OF TRANSGENIC PLANTS, BIOLOGICAL MATERIAL DERIVED THEREFROM, CORRESPONDING PLASMID COLLECTION AND POPULATION OF TRANSFORMED HOST ORGANISMS, USE THEREOF AND METHODS FOR PRODUCING THE SAME
POPULATION DE PLANTES TRANSGENIQUES, ELEMENT BIOLOGIQUE DERIVE DE CES PLANTES, COLLECTION DE PLASMIDES CORRESPONDANTE, POPULATION D'ORGANISMES HOTES TRANSFORMES, UTILISATION ET PROCEDE DE PRODUCTION DE CES POPULATIONS

(30) Priorität: 19.03.2002 DE 10212158
(43) Veröffentlichungstag der Anmeldung: 22.12.2004
(73) Patentinhaber: Metanomics GmbH & Co. KGaA, 10589 Berlin-Charlottenburg (DE)
(72) Erfinder: BLAU, Astrid, 14532 Stahnsdorf (DE); KLEIN, Mathieu, 12159 Berlin (DE); WENDEL, Birgit, 12163 Berlin (DE)
(74) Vertreter: Heistracher, Elisabeth
(86) Internationale Anmeldenummer: PCT/EP2003/002815
(87) Internationale Veröffentlichungsnummer: WO 2003/077642

(56) Entgegenhaltungen:
- WO-A-01/07600
- WO-A-99/36516
- WO-A1-01/35725
- WO-A1-01/35725
- WO-A2-01/26459
- WO-A2-01/26459
- US-A1- 2003 003 585
- MCCUBBIN ANDREW G ET AL: "Construction of a binary bacterial artificial chromosome library of Petunia inflata and the isolation of large genomic fragments linked to the self-incompatibility (S-) locus" GENOME, Bd. 43, Nr. 5, Oktober 2000 (2000-10), Seiten 820-826, XP001156802 ISSN: 0831-2796
- HAMILTON ET AL.: "Stable transfer of intact high molecular weight DNA into plant chromosomes" PROC. NAT. ACAD. SCI. USA, Bd. 93, September 1996 (1996-09), Seiten 9975-9979, XP002263681
- FRARY ANNE ET AL: "Efficiency and stability of high molecular weight DNA transformation: An analysis in tomato" TRANSGENIC RESEARCH, Bd. 10, Nr. 2, April 2001 (2001-04), Seiten 121-132, XP009022796 ISSN: 0962-8819
- LAZO G R ET AL: "A DNA TRANSFORMATION-COMPETENT ARABIDOPSIS GENOMIC LIBRARY IN AGROBACTERIUM" BIO/TECHNOLOGY, NATURE PUBLISHING CO. NEW YORK, US, vol. 9, no. 10, 1 October 1991 (1991-10-01), pages 963-967, XP008062132 ISSN: 0733-222X
- LAZO G R ET AL: "A DNA TRANSFORMATION-COMPETENT ARABIDOPSIS GENOMIC LIBRARY IN AGROBACTERIUM" BIO/TECHNOLOGY, NATURE PUBLISHING CO. NEW YORK, US, vol. 9, no. 10, 1 October 1991 (1991-10-01), pages 963-967, XP008062132 ISSN: 0733-222X

## Beschreibung

Die vorliegende Erfindung betrifft Populationen transgener Pflanzen, die einen wesentlichen Teil sämtlicher kodogener Genabschnitte eines Spenderorganismus umfassen, sowie davon abgeleitetes biologisches transgenes Material, Plasmidkollektionen und Populationen transformierter Wirtsorganismen, mit denen Pflanzen in entsprechender Weise transformiert werden können. Verfahren zur Erzeugung der Pflanzen und des Materials sowie die Verwendung der Pflanzen und des Materials für funktionelle Untersuchungen werden ebenfalls beschrieben.

Aufgrund der wachsenden Weltbevölkerung und sinkender Anbauflächen besteht ein Bedarf an nachhaltiger Produktion. Hier können genomische Informationen zur Optimierung von Produktionsprozessen, insbesondere in der Chemie, Lebensmittelproduktion und Landwirtschaft genutzt werden. Bereits jetzt sind riesige Mengen an genomischer Information verfügbar. Dabei handelt es sich allerdings überwiegend um Sequenzinformationen ohne oder nur mit indirekter Funktionszuordnung.

Es gab bereits Versuche, einzelne Gene aus verschiedenartigen Organismen zu unterschiedlichen Zwecken in Pflanzen zu exprimieren. Ziel dieser Versuche war es, die Funktion eines bestimmten Gens in der Pflanze und dessen Auswirkung auf die pflanzliche Physiologie zu untersuchen.

In WO 01/26459 und WO 01/35725 sind Untersuchungen der Firma Mendel Biotechnology, Inc. zur Einführung von Transkriptionsfaktoren in *Arabidopsis* beschrieben. In Lazo G., et al., BioTechnology 9, 1991, 963-967, ist eine aus 21.600 Klonen bestehende Bank beschreiben. Bei den Klonen handelt es sich um Agrobakterien, die mit in einem Cosmidvektor klonierten Fragmenten genomischer DNA des *Arabidopsis-*Genoms transfiziert wurden. Dabei soll jeder kodogene Genabschnitt in der Bank im Durchschnitt viermal vorkommen.

Ein Ansatz zur transienten Expression mehrerer Gene eines bestimmten Spenderorganismus in einer Modellpflanze wird beispielsweise in WO 99/36516 und WO 01/07600 beschrieben. Dort wird eine vom Spenderorganismus abgeleitete cDNA-Bank in einen geeigneten Vektor eines Pflanzenvirus eingefügt, wodurch nach Infektion der Wirtspflanze mit besagtem Vektor eine schnelle und starke Expression der cDNA erzielt werden kann. Dabei können allerdings nur solche cDNAs in den Pflanzen zur Expression gebracht werden, die in der Ausgangsbibliothek vorhanden sind. Signifikante Anteile der Gene eines Genoms werden jedoch, wenn überhaupt, nur sehr schwach oder nur unter sehr spezifischen Bedingungen exprimiert und daher mit einem solchen Ansatz nicht erfasst. Nachteilig an dieser Vorgehensweise ist auch, dass durch die Infektion Effekte auftreten. Diese können die Ergebnisse physiologischer Untersuchungen an den infizierten Pflanzen beeinflussen. Eine ebenfalls der transienten Expression immanente Schwäche ist die lediglich vorübergehende Verfügbarkeit des transfizierten Materials. So können in analytischen Untersuchungen nur verhältnismäßig kurze Zeiträume betrachtet werden. Entwicklungsrelevante und über längere Zeiträume wirksame Einflüsse werden nicht erkannt. Überdies ist bei Verwendung viraler Vektoren wegen der notwendigen Verpackung der Sequenzen in die Virushülle die Größe einbaubarer Sequenzen limitiert.

Aufgabe der vorliegenden Erfindung war es daher, Pflanzen zur Verfügung zu stellen, an denen funktionelle Untersuchungen über die Funktion fremder Gene systematisch über den gesamten Entwicklungszyklus der Pflanze und gewünschtenfalls sogar über mehrere Generationen durchgeführt werden können.

Gelöst wird die Aufgabe durch den Gegenstand der vorliegenden Erfindung, nämlich einer Population transgener Pflanzen, die eine Gruppe transgener Pflanzen umfasst, wobei in das Genom jeder transgenen Pflanze der Gruppe ein kodogener Genabschnitt eines Spenderorganismus, nicht aber ein anderer kodogener Genabschnitt des Spenderorganismus, integriert ist, die Population erhältlich ist, indem man:
a) einen kodogenen Genabschnitt des Spenderorganismus bereitstellt;
b) diesen kodogenen Genabschnitt im Genom einer Pflanze integriert; und
c) die Schritte a) und b) für wenigstens 50% sämtlicher kodogener Genabschnitte des Spenderorganismus an einer entsprechenden Anzahl von Pflanzen durchführt, so dass jede dieser Pflanzen einen, nicht aber einen anderen der kodogenen Genabschnitte aufweist,
und der Spenderorganismus ausgewählt ist unter Acetobacter (subgen. Acetobacter) aceti; Acidithiobacillus ferrooxidans; Acinetobacter sp.; Actinobacillus sp; Aeromonas salmonicida; Agrobacterium tumefaciens; Aquifex aeolicus; Arcanobacterium pyogenes; Aster yellows phytoplasma; Bacillus sp.; Bifidobacterium sp.; Borrelia burgdorferi; Brevibacterium linens; Brucella melitensis; Buchnera sp.; Butyrivibrio fibrisolvens; Campylobacter jejuni; Caulobacter crescentus; Chlamydia sp.; Chlamydophila sp.; Chlorobium limicola; Citrobacter rodentium; Clostridium sp.; Comamonas testosteroni; Corynebacterium sp.; Coxiella burnetii; Deinococcus radiodurans; Dichelobacter nodosus; Edwardsiella ictaluri; Enterobacter sp.; Erysipelothrix rhusiopathiae; Escherichia coli; Flavobacterium sp.; Francisella tularensis; Frankia sp. Cpl1; Fusobacterium nucleatum; Geobacillus stearothermophilus; Gluconobacter oxydans; Haemophilus sp.; Helicobacter pylori; Klebsiella pneumoniae; Lactobacillus sp.; Lactococcus lactis; Listeria sp.; Mannheimia haemolytica; Mesorhizobium loti; Methylophaga thalassica; Microcystis aeruginosa; Microscilla sp. PRE1; Moraxella sp. TA144;

Mycobacterium sp.; Mycoplasma sp.; Neisseria sp.; Nitrosomonas sp.; Nostoc sp. PCC 7120; Novosphingobium aromaticivorans; Oenococcus oeni; Pantoea citrea; Pasteurella multocida; Pediococcus pentosaceus; Phormidium foveolarum; Phytoplasma sp.; Plectonema boryanum; Prevotella ruminicola; Propionibacterium sp.; Proteus vulgaris; Pseudomonas sp.; Ralstonia sp.; Rhizobium sp.; Rhodococcus equi; Rhodothermus marinus; Rickettsia sp.; Riemerella anatipestifer; Ruminococcus flavefaciens; Saccharomyces cerevisiae; Salmonella sp.; Selenomonas ruminantium; Serratia entomophila; Shigella sp.; Sinorhizobium meliloti; Staphylococcus sp.; Streptococcus sp.; Streptomyces sp.; Synechococcus sp.; Synechocystis sp. PCC 6803; Thermotoga maritima; Treponema sp.; Ureaplasma urealyticum; Vibrio cholerae; Vibrio parahaemolyticus; Xylella fastidiosa; Yersinia sp.; Zymomonas mobilis.

Der Begriff "kodogener Genabschnitt" bezieht sich auf eine Nukleinsäure. Nukleinsäuren werden aus Monomeren (Nukleotiden) gebildet und enthalten Zucker, Phosphat sowie entweder ein Purin oder Pyrimidin bzw. Derivate davon. Hierzu gehören DNA und RNA-Sequenzen, die einzel- oder doppelsträngig sein und gegebenenfalls synthetische, nicht natürliche oder veränderte, in DNA oder RNA einbaubare Nukleotidbasen aufweisen können.

Insbesondere bezieht sich der Ausdruck "kodogener Genabschnitt "auf die kodierende Sequenz, d.h. den Teil eines Gens, der ein Protein, Polypeptid oder ein Teil davon kodiert.

Der Begriff "Gen" bezieht sich auf eine DNA-Sequenz, die sowohl den kodogenen Genabschnitt, also insbesondere die kodierende Sequenz, als auch regulatorische Elemente umfasst.

Das den kodogenen Genabschnitt umfassende Strukturgen kann eine fortlaufende kodierende Sequenz aufweisen (offenes Leseraster, kurz ORF) oder es kann ein oder mehrere Introns beinhalten, die über geeignete Spleißverbindungen mit den Exons verbunden sind.

Die erfindungsgemäßen kodogenen Genabschnitte sind normalerweise in den Zellen des Spenderorganismus zu finden. Es handelt sich damit in Bezug auf den Spenderorganismus um autologe Sequenzen. Hingegen sind diese kodogenen Genabschnitte normalerweise nicht in den Pflanzen zu finden, auf denen die erfindungsgemäßen Populationen basieren (Empfängerpflanze). Es handelt sich somit in Bezug auf die Empfängerpflanze um heterologe Sequenzen.

Kritisch für die vorliegende Erfindung ist die Integration der kodogenen Genabschnitte im Genom der Pflanzen. Dabei kann ein bestimmter kodogener Genabschnitte als fortlaufende kodierende Sequenz (ORF) integriert sein oder ein oder mehrere Introns beinhalten. Ist letzteres der Fall, werden derartige Sequenzen im Zuge der Expression durch die Pflanze in der Regel gespleißt, wobei das Spleißmuster dem des Spenderorganismus entsprechen kann, aber nicht muss.

Grundsätzlich können die kodogenen Genabschnitte in das extranukleare Genom, z.B. dem Plastidengenom, einer Pflanze integriert sein. Erfindungsgemäß bevorzugt ist allerdings die Integration in das nukleare Genom.

Erfindungsgemäß bevorzugt ist es, die Sequenzen, welche die heterologen kodogene Genabschnitte umfassen, stabil in das Genom der Pflanzen zu integrieren. Dies ist verbunden mit einem oder mehreren der folgenden Aspekte:
- die Anzahl an Kopien eines bestimmten kodogenen Genabschnitts pro Zelle ist während des Lebenszyklus einer Pflanze im Wesentlichen konstant;
- die Anzahl an Kopien eines bestimmten kodogenen Genabschnitts pro Zelle ist bestimmbar,
- der kodogene Genabschnitt ist als Merkmal der Pflanze vererbbar, bei nuklearer Integration nach den Mendelschen Regeln.

In der Regel beträgt die Anzahl an integrierten Kopien eines bestimmten kodogenen Genabschnitts pro Zelle weniger als 20 und in den meisten Fällen weniger als 10. Erfindungsgemäß bevorzugt sind Pflanzen mit Zellen, die etwa 1 bis 5 Kopien und insbesondere 1 Kopie eines bestimmten kodogenen Genabschnitts umfassen. Die Anzahl an Kopien pro Zelle können in an sich bekannter Weise mittels "Southern-Blot"-Analyse (Extraktion der genomischen DNA, restriktionsenzymatischer Verdau, elektrophoretische Auftrennung, Membrantransfer, Hybridisierung mit markierter DNA-spezifischer Sonde oder quantitativer PCR bestimmt werden.

Erfindungsgemäß umfasst der Lebenszyklus einer Pflanze vorteilhafterweise sämtliche Entwicklungsstadien von der Keimung bis hin zur Samenreife. Beispielsweise umfasst der Lebenszyklus von *Arabidopsis thaliana* die epigäische Keimung, die Entwicklung des Keimlings zur Rosette, die Ausbildung des Hauptsprosses aus der Rosette, dessen Verzweigung, die Entwicklung von Blühten an den Trieben, den Blühvorgang und die Samenreife. *Arabidopsis thaliana* kann bei günstigen Bedingungen innerhalb von etwa 6 Wochen von Samen zu Samen vermehrt werden.

Gemäß einer besonderen Ausführungsform der vorliegenden Erfindung sind die heterologen kodogenen Genabschnitte im Genom der transgenen Pflanzen von T-DNA-Sequenzen, insbesondere von Agrobakterium-Ti-Plasmidsequenzen, einseitig oder vorzugsweise beidseitig flankiert. Dies ist ebenfalls Ausdruck der erfindungsgemäßen stabilen Integration der kodogenen Genabschnitte in das Genom der Pflanzen.

Erfindungsgemäße Populationen transgener Pflanzen umfassen wenigstens 50 % aller kodogenen Genabschnitte eines bestimmten Spenderorganismus. Populationen transgener Pflanzen mit wenigstens 70 % und insbesondere mit wenigstens 90 % aller kodogenen Genabschnitte eines bestimmten Spenderorganismus sind bevorzugt.

Darüber hinaus lassen sich die kodogenen Genabschnitte eines Spenderorganismus in der Regel durch die Funktion ihrer Expressionsprodukte kennzeichnen. Hierzu gehören insbesondere Funktionen in den Bereichen Metabolismus, Energie, Transkription, Proteinsynthese, Proteinprozessierung, zellulärer Transport und Transportmechanismen, zelluläre Kommunikation und Signaltransduktion, Zellrettung, Zellverteidigung und Zellvirulenz, Regulation der zellulären Umgebung und Interaktion der Zelle mit ihrer Umgebung, Zellschicksal, transponierbare Elemente, virale Proteine und Plasmidproteine, zelluläre Organisationskontrolle, subzelluläre Lokalisation, Regulation der Proteinaktivität, Proteine mit Bindungsfunktion oder Cofaktor-Erfordemis und Transporterleichterung. Kodogene Genabschnitte gleicher Funktion werden zu sogenannten funktionellen Genfamilien zusammengefasst.

Metabolische Funktionen betreffen vor allem den Aminosäuremetabolismus, den Stickstoff- und Schwefelmetabolismus, den Nukleotidmetabolismus, den Phosphatmetabolismus, den Kohlenstoff und Kohlenhydratmetabolismus, den Fett-, Fettsäure- und Isoprenoidmetabolismus, den Metabolismus von Vitaminen, Cofaktoren und prosthetischen Gruppen sowie den sekundären Metabolismus.

Energetische Funktionen betreffen vor allem die Glykolyse und Glykoneogenese, den Pentose-Phosphat-Stoffwechselweg, den Citratzyklus, den Elektronentransport und die membranassoziierte Energiespeicherung, die Atmung, die Photosynthese, die Fermentation, den Metabolismus von Energiereserven, den Glyoxylatzyklus und die Fettsäureoxidation. Funktionen im Bereich der Transkription betreffen vor allem die Transkription von rRNA-, tRNA und mRNA, den RNA-Transport und die Transkript-Prozessierung.

Funktionen im Bereich der Proteinsynthese betreffen vor allem die Ribosombiogenese, die Translation, die Translationskontrolle und Aminoacyl-tRNA-Synthetasen.

Funktionen im Bereich der Proteinprozessierung betreffen vor allem die Faltung und Stabilisierung, das Targeting, Sorting und die Translokation sowie die Modifikation von Proteinen, den Zusammenbau von Proteinkomplexen und den proteolytischen Abbau von Proteinen.

Funktionen im Bereich des zellulären Transports und von Transportmechanismen betreffen vor allem den nuklearen, mitochondrialen, vesikulären, peroxysomalen, vakuolären und extrazellulären Transport, die Exocytose und Sekretion, die Endocytose, den zellulären Import und den Zytoskelett-abhängigen Transport.

Funktionen im Bereich der zellulären Kommunikation und Signaltransduktion betreffen vor allem die intrazelluläre Signalgebung und -rezeption, und die Transmembran-Signaltransduktion.

Funktionen im Bereich der Zellrettung, Zellverteidigung und Zellvirulenz betreffen vor allem die Stressantwort, die Detoxifizierung und den Abbau fremder (exogener) Stoffe.

Funktionen im Bereich der Regulation der zellulären Umgebung und der Interaktion der Zelle mit ihrer Umgebung betreffen vor allem die lonenhomeostase und die zelluläre Wahrnehmung sowie Antwort.

Funktionen im Bereich Zellschicksal betreffen vor allem den Zellzyklus und das Zellwachstum, die Zellmorphogenese, die Zelldifferenzierung, den Zelltod und die Zellalterung.

Funktionen im Bereich der zellulären Organisationskontrolle betreffen vor allem die Zellwand, die Plasmamembran, das Zytoplasma, das Zytoskelett, das endoplasmatische Retikulum, den Golgi, die Plastiden, die intrazellulären Transportvesikel, den Zellkern, die Mitochondrien, die Peroxysomen, die Endosomen, sowie die Vakuolen oder Lysosomen. Funktionen im Bereich der subzellulären Lokalisation betreffen insbesondere die Zellwand, die Plasmamembran, das Zytoplasma, das Zytoskelett, die Zentrosomen, das endoplasmatische Retikulum, den Golgi, intrazelluläre Transportvesikel, den Zellkern, Mitochondrien, Peroxysomen, Endosomen, Vakuolen oder Lysomen, extrazelluläre oder sekretierte Proteine, und prokariotische Zellmembrane.

Funktionen im Bereich der Regulation der Proteinaktivität betreffen vor allem die jeweiligen zu regulierenden Targets.

Funktionen im Bereich von Proteinen mit Bindungsfunktion oder Cofaktor-Erfordemis betreffen vor allem die Proteinbindung und die Lipidbindung.

Funktionen im Bereich der Transporterleichterung betreffen vor allem Transporter vom Kanal/Poren-Typ, lonentransporter, Kohlenstoff- und Kohlenhydrattransporter, Aminosäure-Transporter, Peptidtransporter, Lipidtransporter, Nukleotidtransporter, Allantoin- und Allantoattransporter, Wirkstofftransporter und Transportmechanismen.

Erfindungsgemäß von besonderer Bedeutung sind Funktionen in den Bereichen Metabolismus und Energie, insbesondere Enzyme des Primär- und Sekundärstoffwechsels, z.B. P450-Enzyme, im Bereich Transkription, insbesondere Transkriptionsfaktoren, und im Bereich zellulärer Transport und Transportmechanismen, insbesondere Kanäle und Transporter.

So sind insbesondere Populationen transgener Pflanzen bevorzugt, die wenigstens eine Subpopulation aufweisen, welche dadurch gekennzeichnet ist, dass die Subpopulation wenigstens 55 % und insbesondere wenigstens 80 % aller kodogenen Genabschnitte eines Spenderorganismus umfassen, die einer funktionellen Genfamilie zuzuordnen sind.

Die kodogenen Genabschnitte eines bestimmten Spenderorganismus sind in der Regel allgemein zugänglich. Insbesondere sind hier allgemeine Gen-Datenbanken zu nennen, wie die EMBL-Datenbank (Stoesser G. et al., Nucleic Acids Res 2001, Vol. 29, 17-21), die GenBank-Datenbank (Benson D.A. et al., Nucleic Acids Res 2000, Vol. 28,15-18), oder die PIR-Datenbank (Barker W. C. et al., Nucleic Acids Res. 1999, Vol. 27, 39-43).

Weiterhin können Organismen-spezifische Gen-Datenbanken verwendet werden, so z.B. für Hefe die SGD-Datenbank (Cherry J. M. et al., Nucleic Acids Res. 1998, Vol. 26, 73-80) oder die MIPS-Dateribank (Mewes H.W. et al., Nucleic Acids Res. 1999, Vol. 27, 44-48), für E. *coli* die GenProtEC-Datenbank (http://web.bham.ac.uk/bcm4ght6/res.html), für Arabidopsis die TAIR-Datenbank (Huala, E. et al., Nucleic Acids Res. 2001 Vol. 29(1). 102-5) oder die MIPS-Datenbank.

Anhand dieser Datenbank-Informationen können in an sich bekannter Weise Nukleinsäuren, welche die kodogenen Genabschnitte umfassen, aus geeigneten Quellen gewonnen und für die anschließende Integration in die Pflanzengenome bereitgestellt werden.

Zu den Spenderorganismen gehören sowohl prokaryotische als auch eukaryotische Organismen.

Die erfindungsgemäßen Spenderorganismen sind Mikroorganismen, deren Genome sequenziert sind, z.B. Acetobacter (subgen. Acetobacter) aceti; Acidithiobacillus ferrooxidans; Acinetobacter sp.; Actinobacillus sp; Aeromonas salmonicida; Agrobacterium tumefaciens; Aquifex aeolicus; Arcanobacterium pyogenes; Aster yellows phytoplasma; Bacillus sp.; Bifidobacterium sp.; Borrelia burgdorferi; Brevibacterium linens; Brucella melitensis; Buchnera sp.; Butyrivibrio fibrisolvens; Campylobacter jejuni; Caulobacter crescentus; Chlamydia sp.; Chlamydophila sp.; Chlorobium limicola; Citrobacter rodentium; Clostridium sp.; Comamonas testosteroni; Corynebacterium sp.; Coxiella burnetii; Deinococcus radiodurans; Dichelobacter nodosus; Edwardsiella ictaluri; Enterobacter sp.; Erysipelothrix rhusiopathiae; Escherichia coli; Flavobacterium sp.; Francisella tularensis; Frankia sp. CpI1; Fusobacterium nucleatum; Geobacillus stearothermophilus; Gluconobacter oxydans; Haemophilus sp.; Helicobacter pylori; Klebsiella pneumoniae; Lactobacillus sp.; Lactococcus lactis; Listeria sp.; Mannheimia haemolytica; Mesorhizobium loti; Methylophaga thalassica; Microcystis aeruginosa; Microscilla sp. PRE1; Moraxella sp. TA144; Mycobacterium sp.; Mycoplasma sp.; Neisseria sp.; Nitrosomonas sp.; Nostoc sp. PCC 7120; Novosphingobium aromaticivorans; Oenococcus oeni; Pantoea citrea; Pasteurella multocida; Pediococcus pentosaceus; Phormidium foveolarum; Phytoplasma sp.; Plectonema boryanum; Prevotella ruminicola; Propionibacterium sp.; Proteus vulgaris; Pseudomonas sp.; Ralstonia sp.; Rhizobium sp.; Rhodococcus equi; Rhodothermus marinus; Rickettsia sp.; Riemerella anatipestifer, Ruminococcus flavefaciens; Salmonella sp.; Selenomonas ruminantium; Serratia entomophila; Shigella sp.; Sinorhizobium meliloti; Staphylococcus sp.; Streptococcus sp.; Streptomyces sp.; Synechococcus sp.; Synechocystis sp. PCC 6803; Thermotoga maritima; Treponema sp.; Ureaplasma urealyticum; Vibrio cholerae; Vibrio parahaemolyticus; Xylella fastidiosa; Yersinia sp.; Zymomonas mobilis.

Gemäß einer besonderen Ausführungsform der vorliegenden Erfindung ist der Spenderorganismus eine Hefe, vorzugsweise der Gattung *Saccharomyces,* insbesondere *Saccharomyces cerevisiae.*

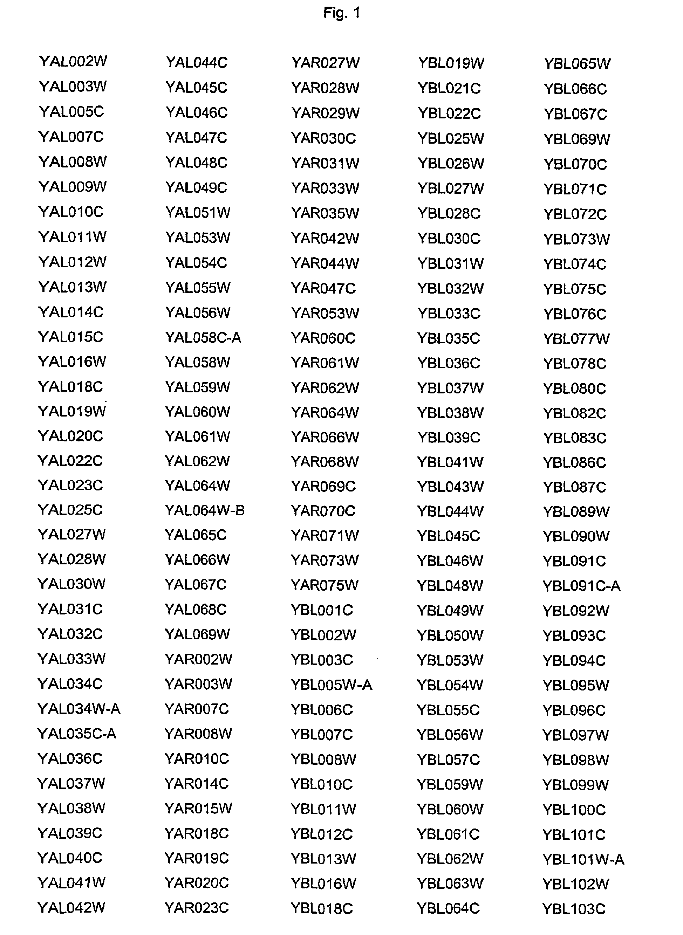

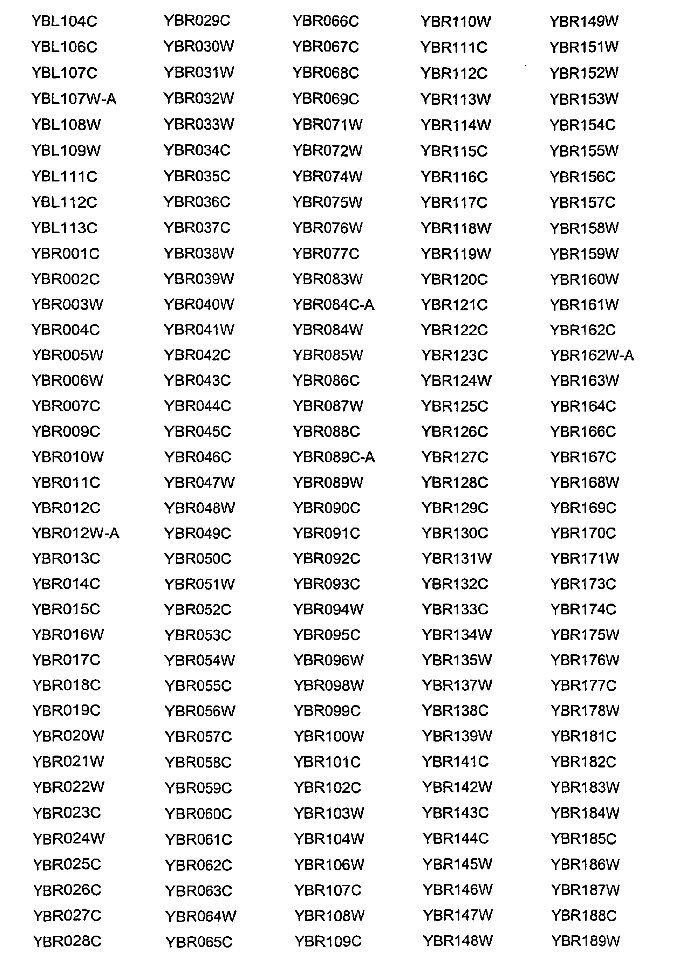

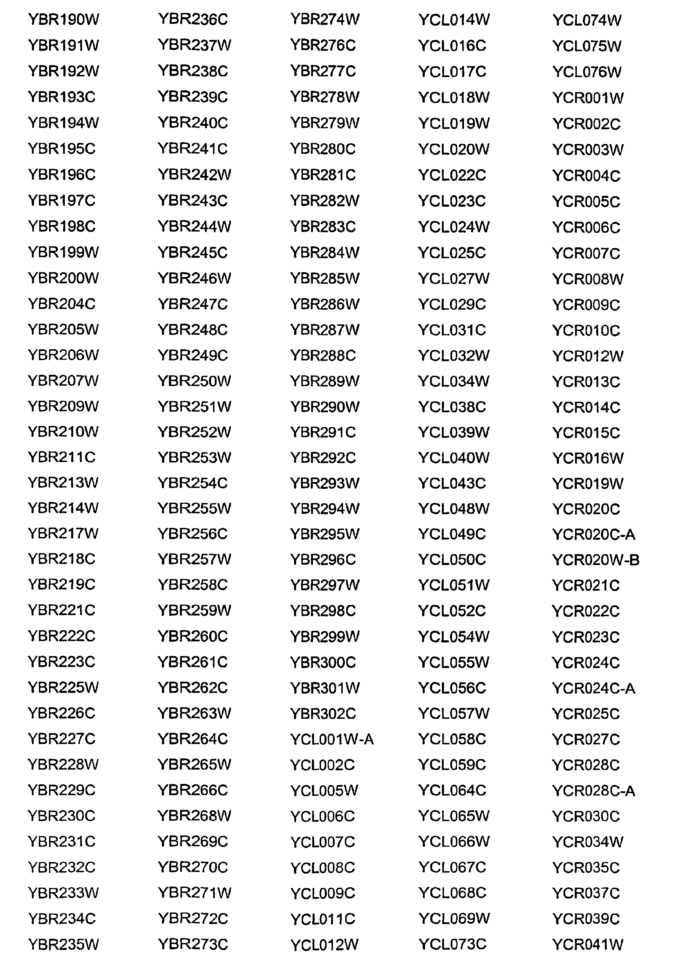

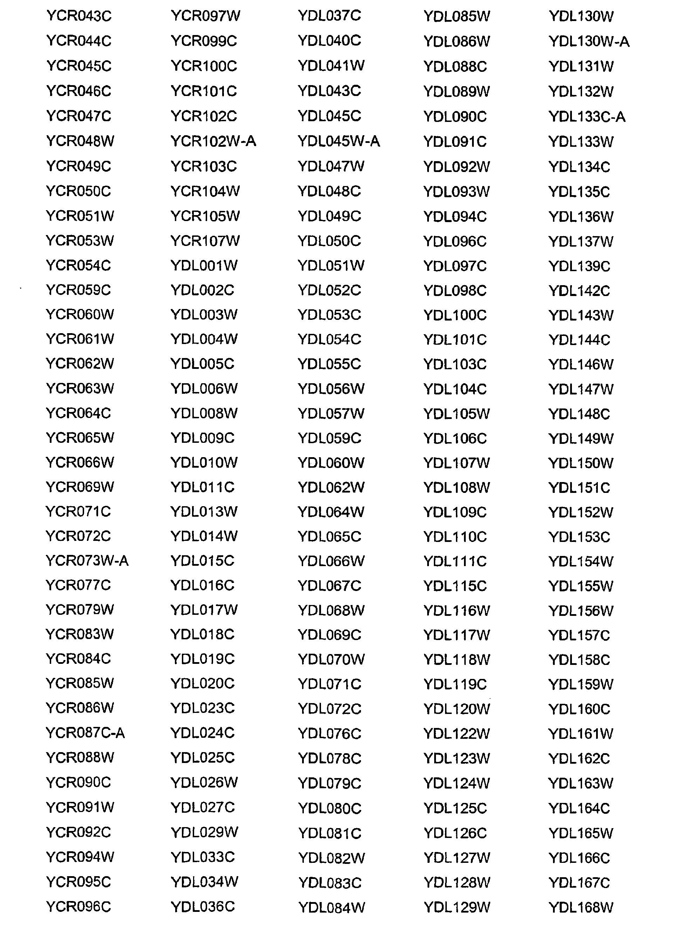

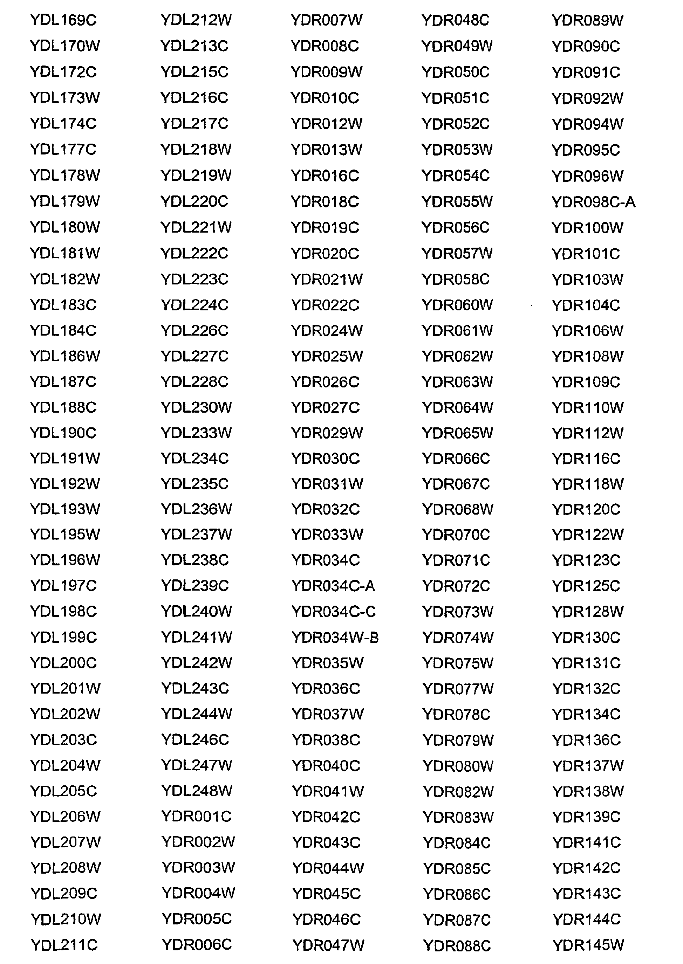

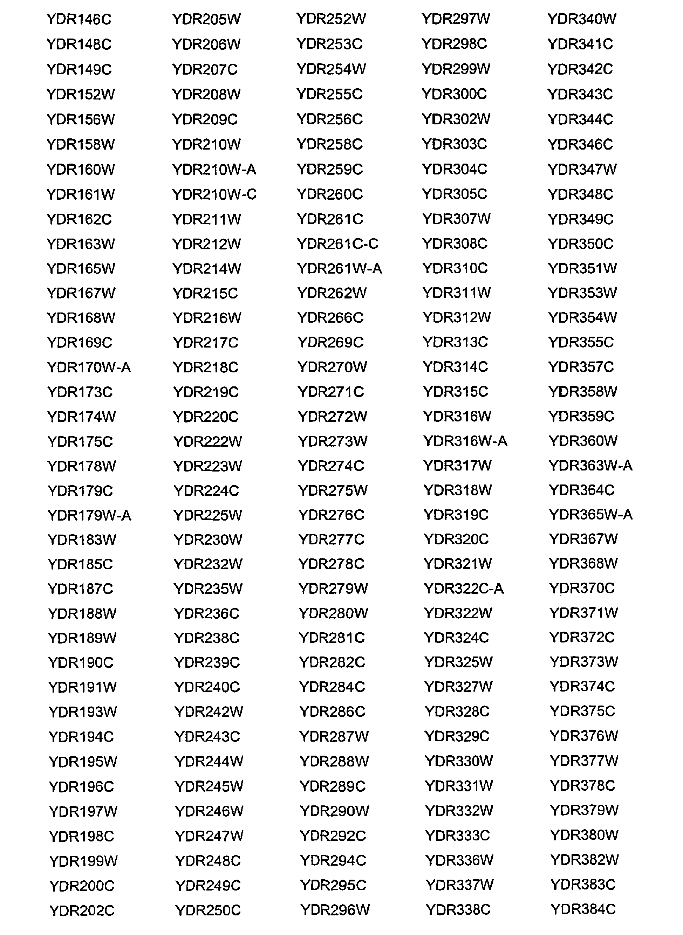

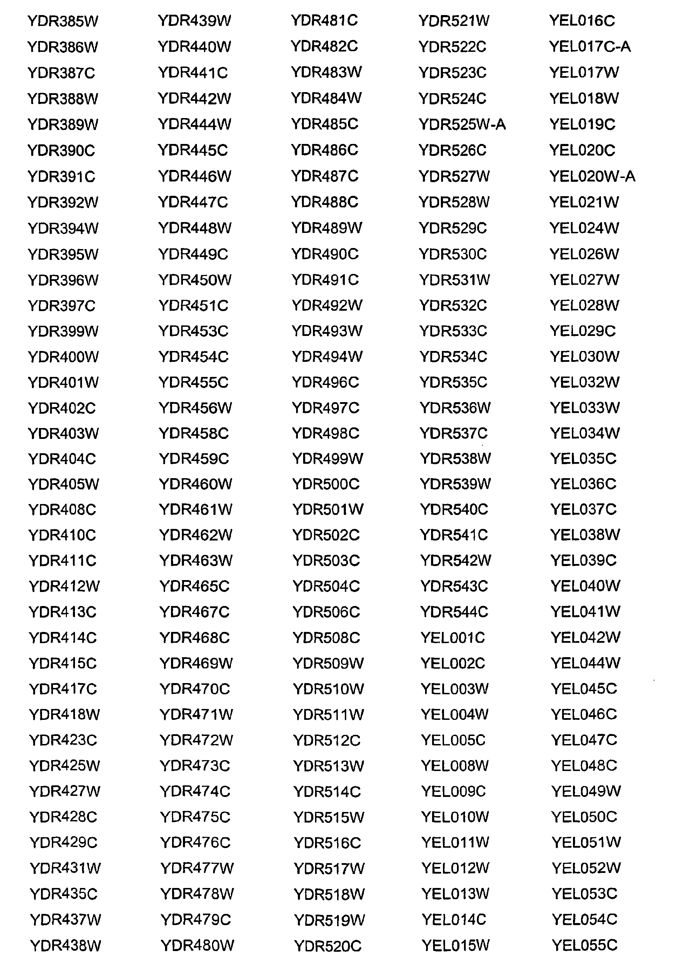

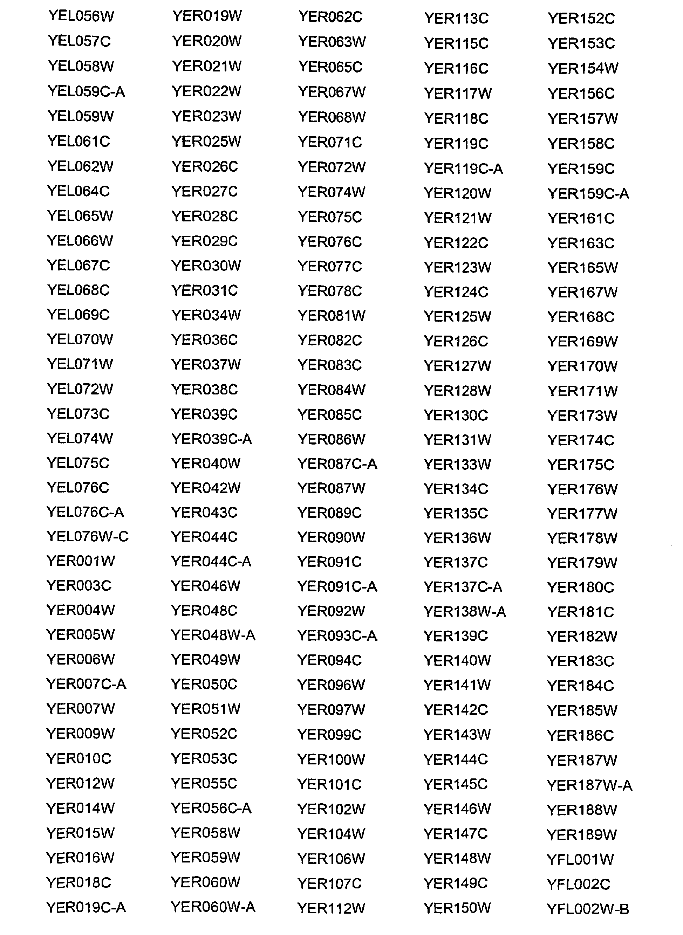

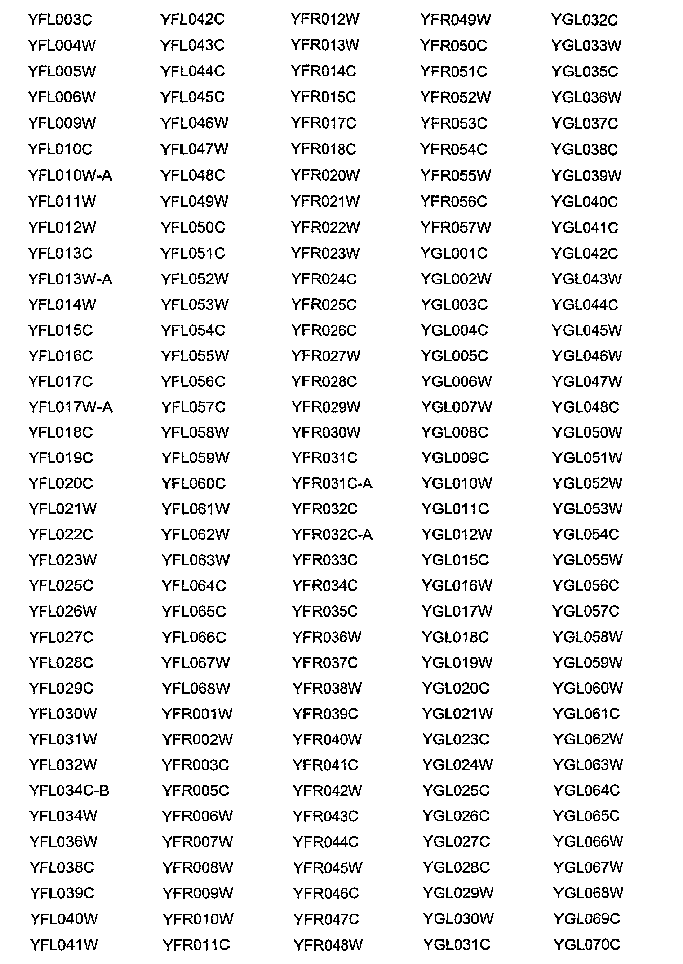

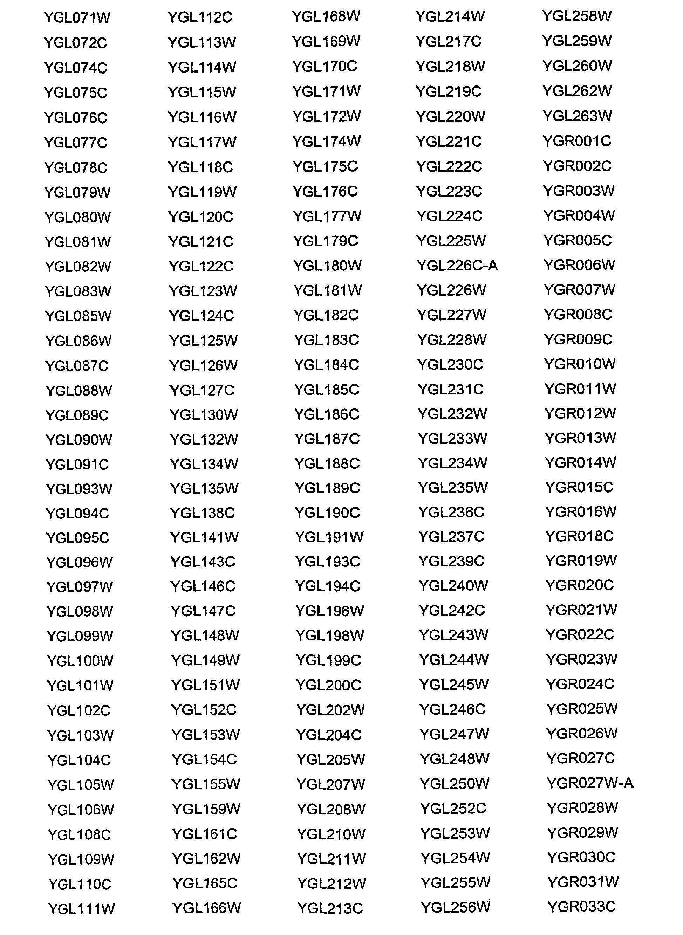

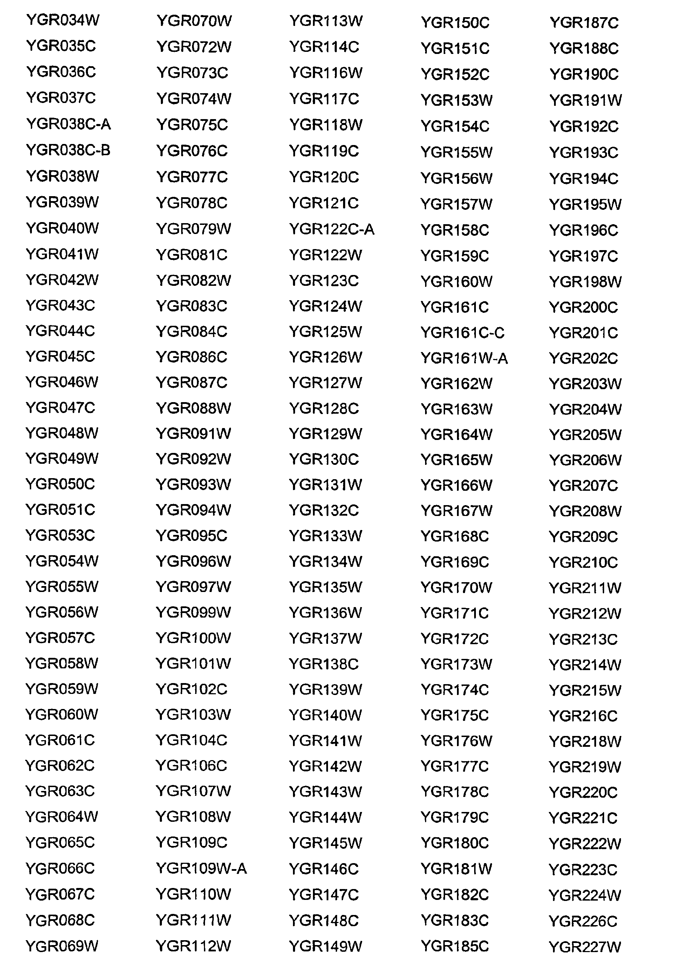

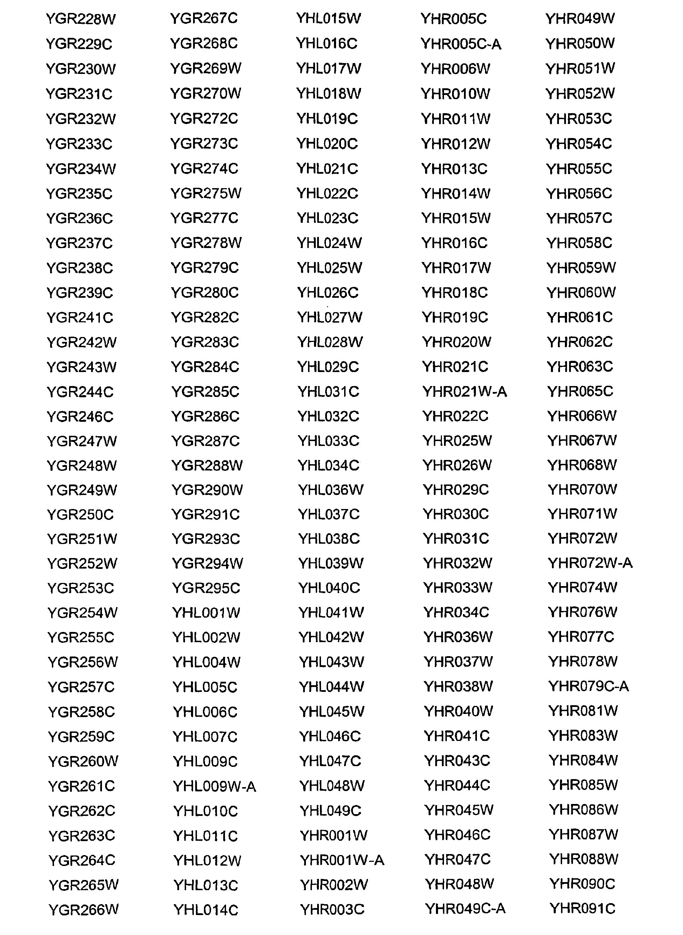

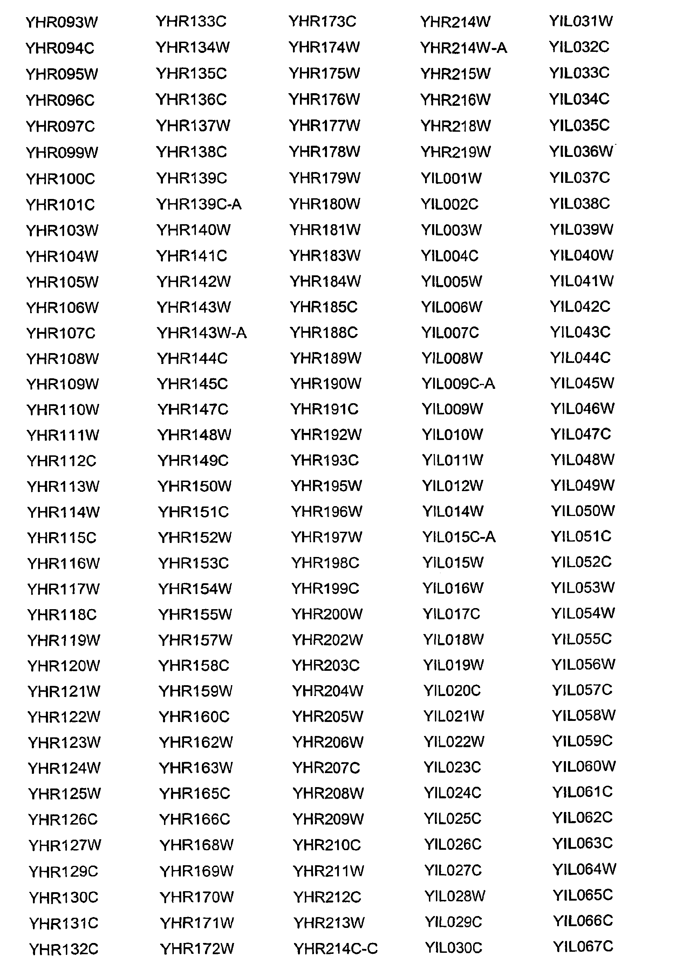

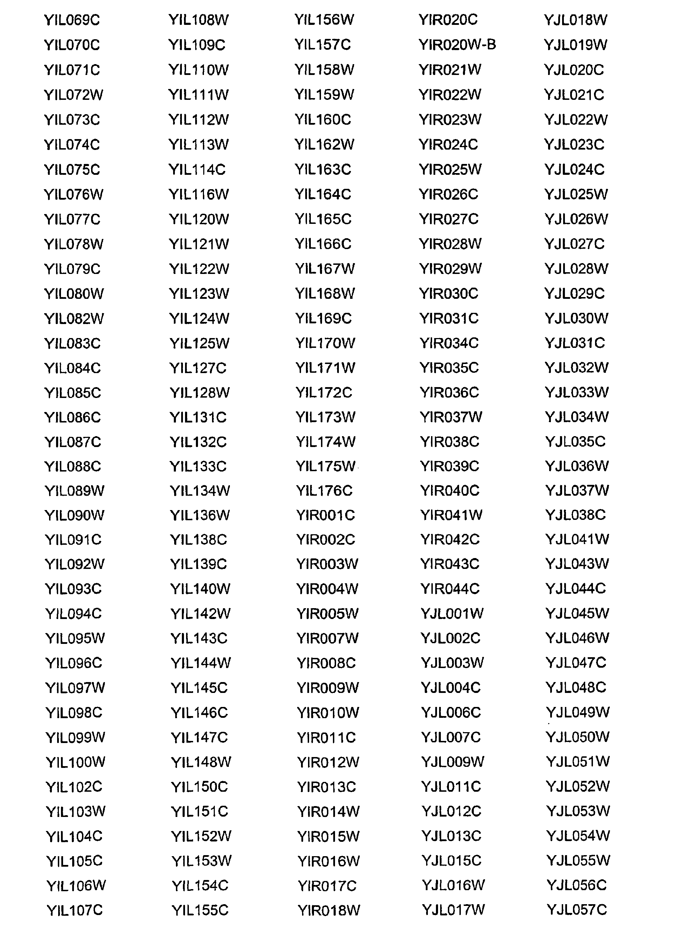

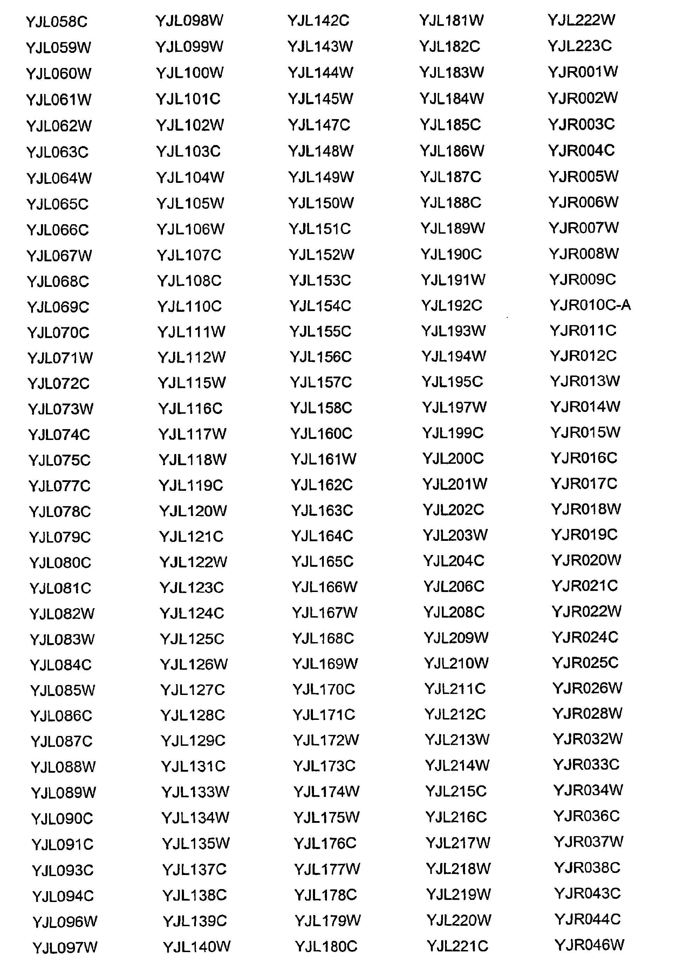

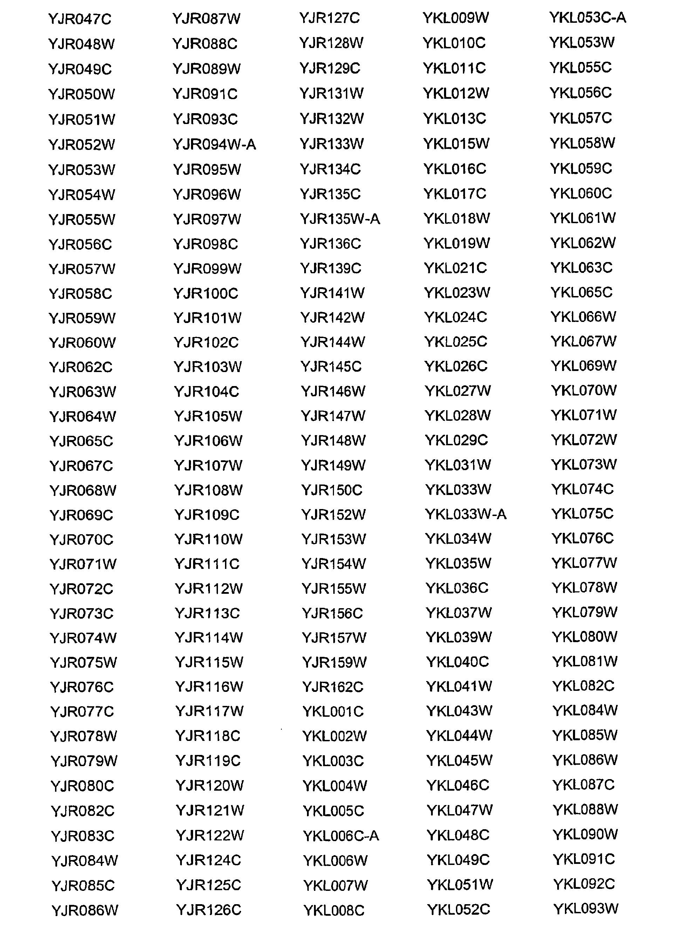

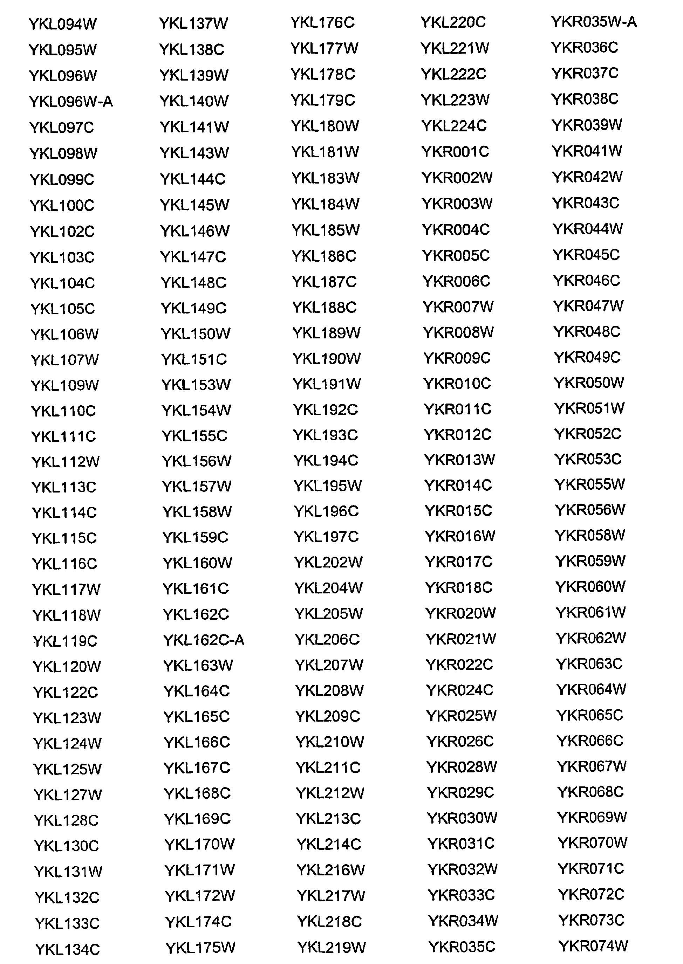

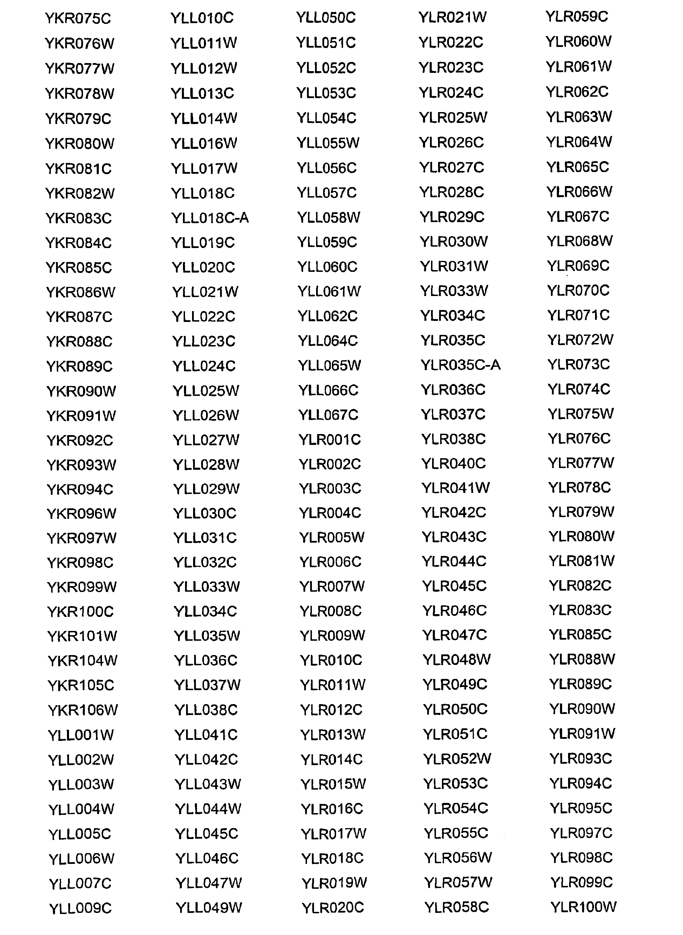

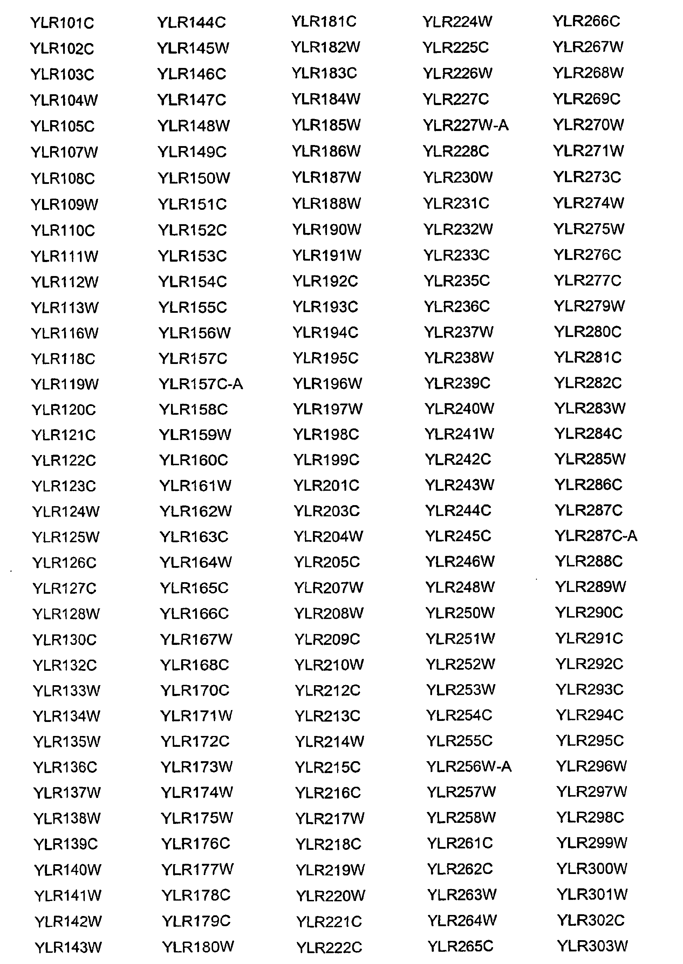

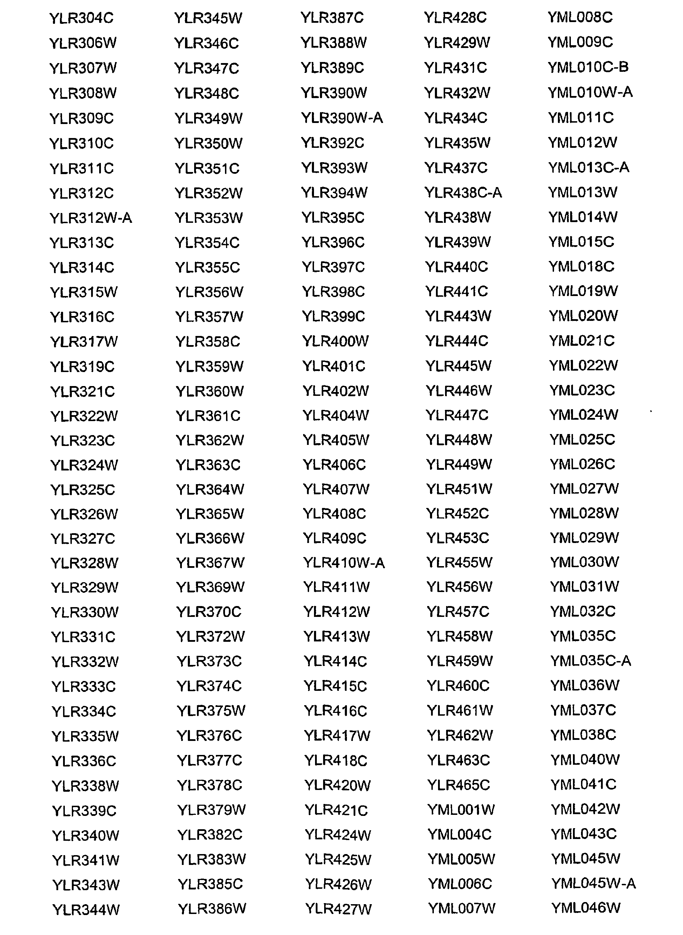

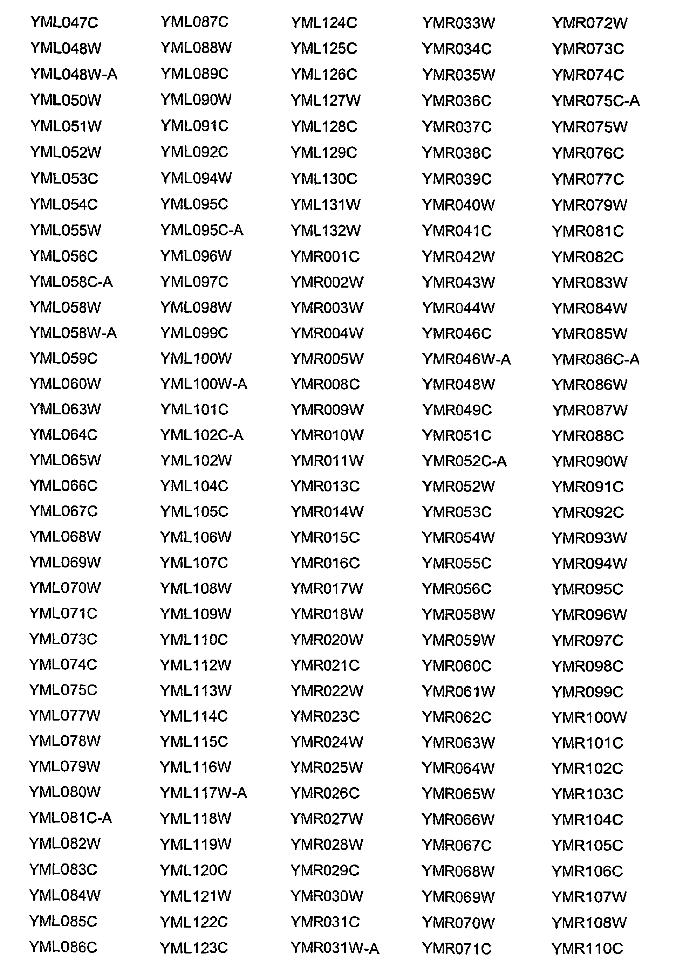

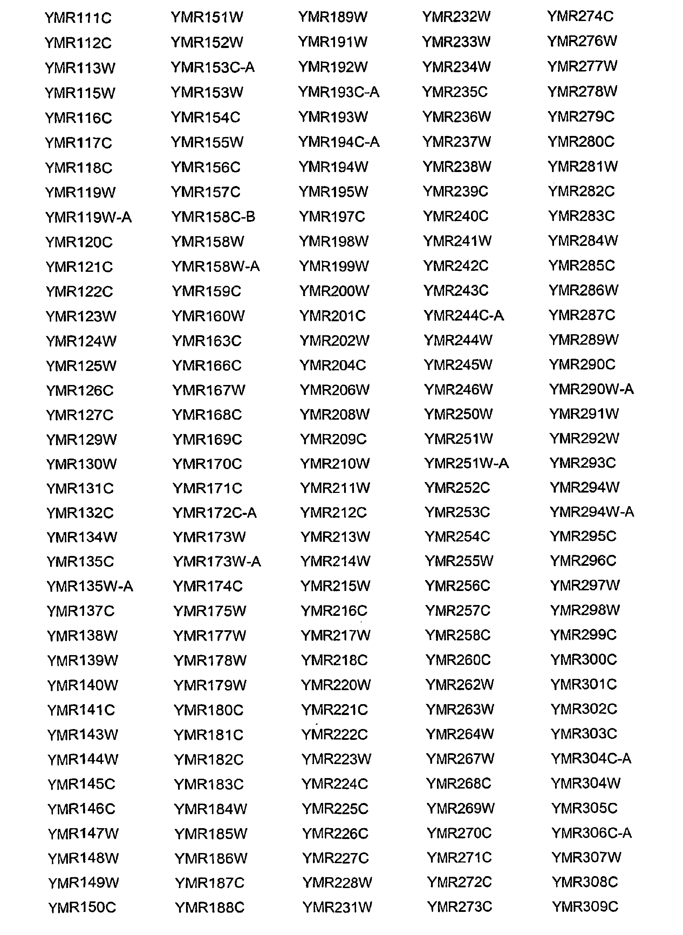

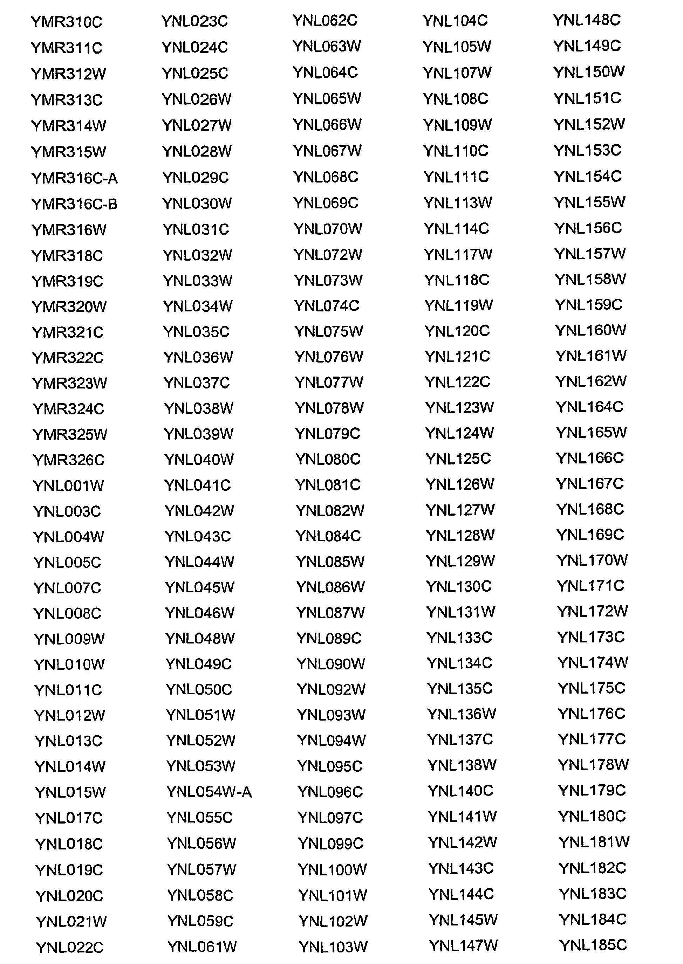

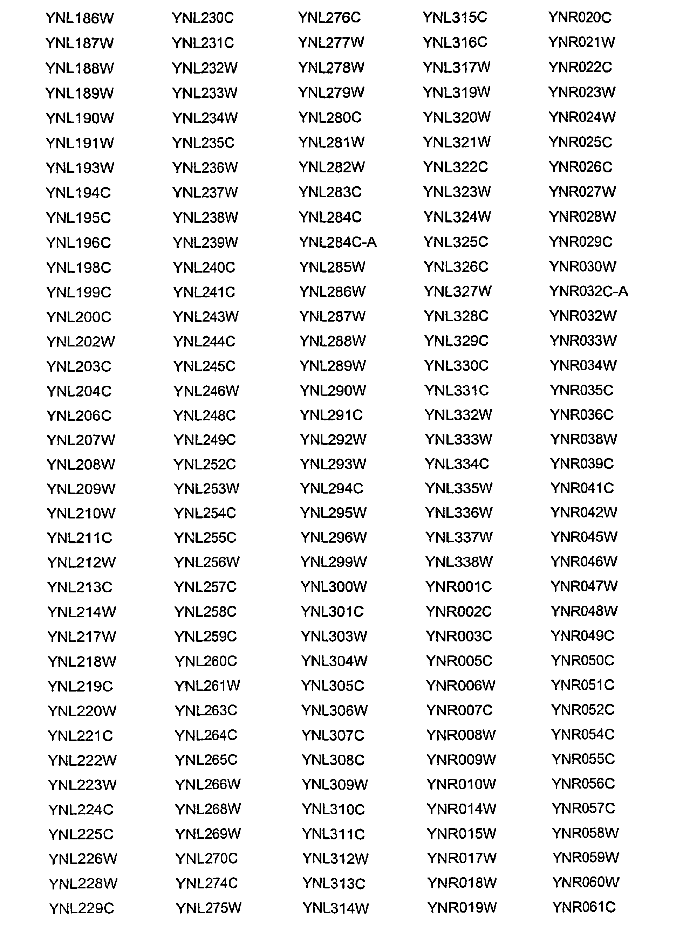

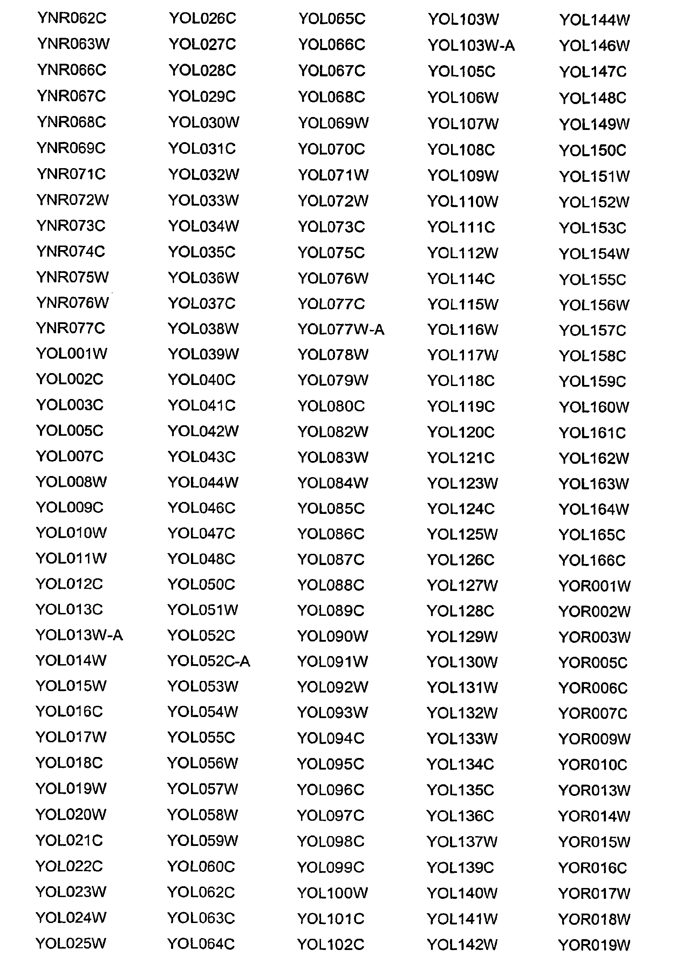

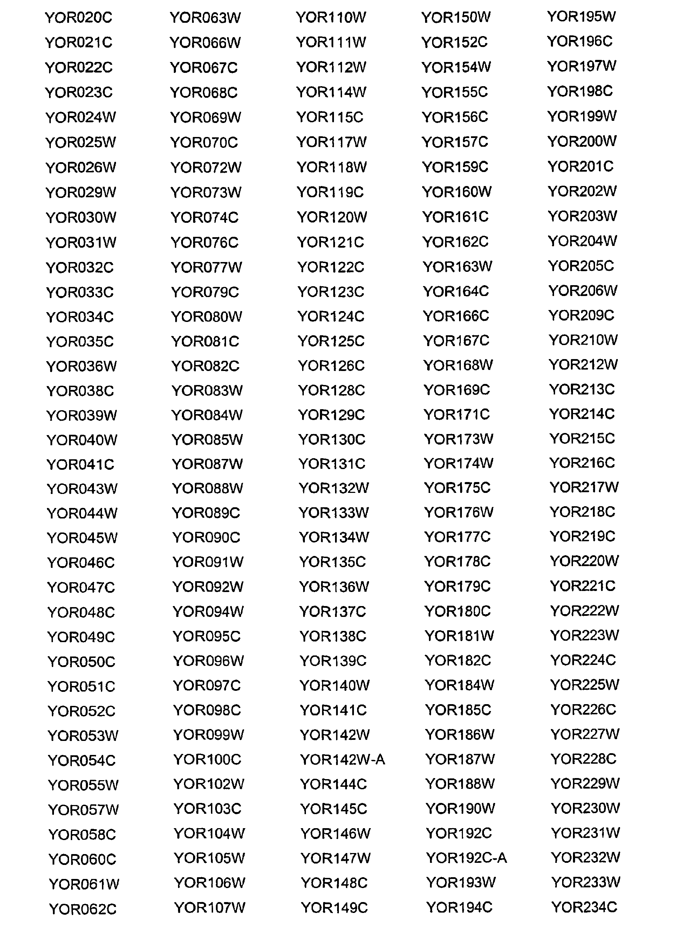

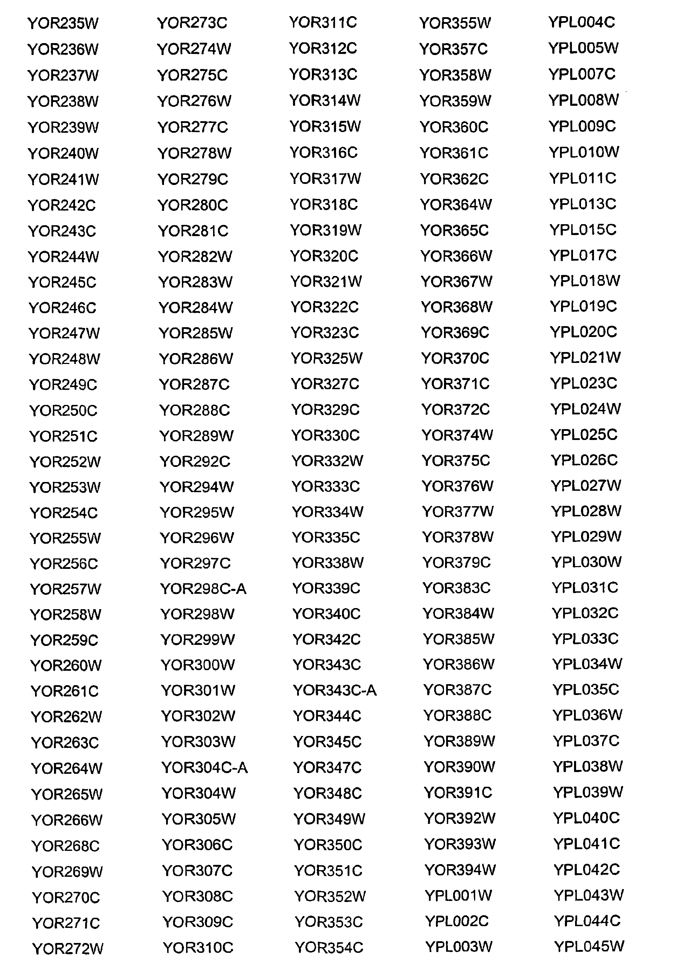

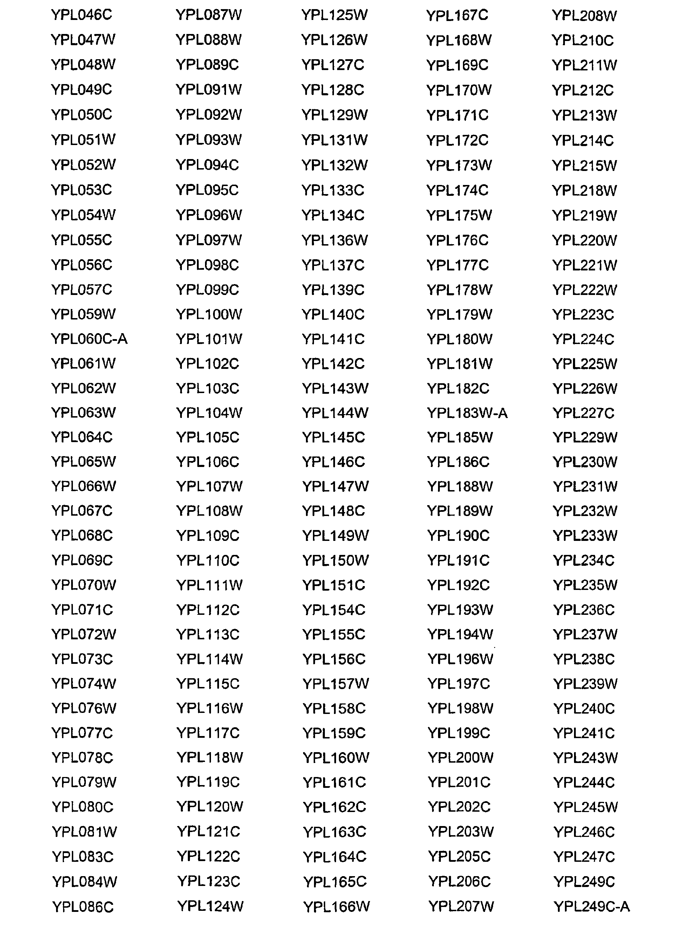

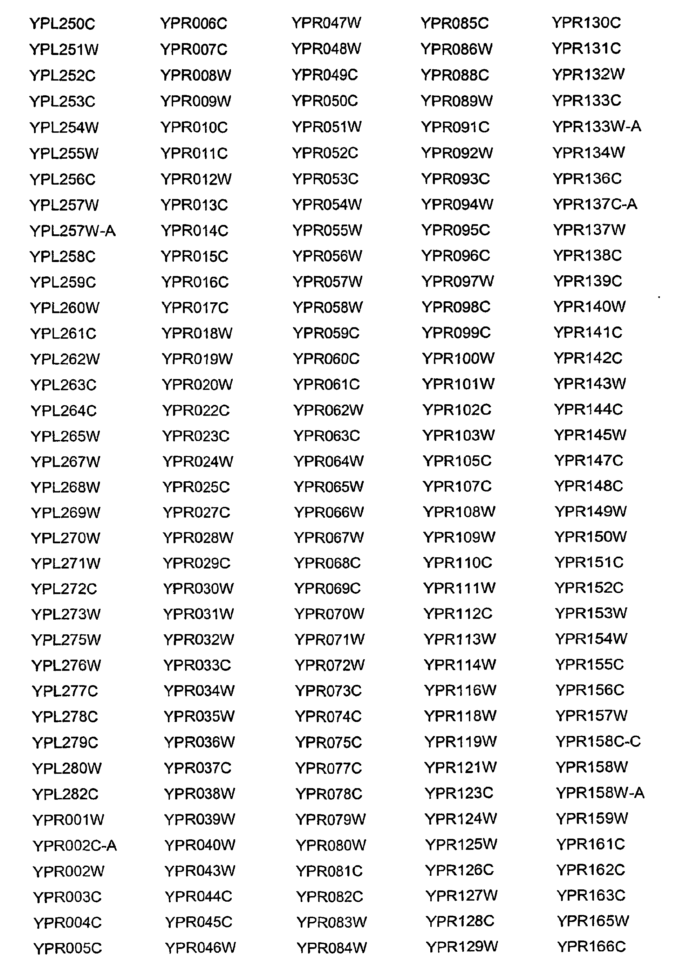

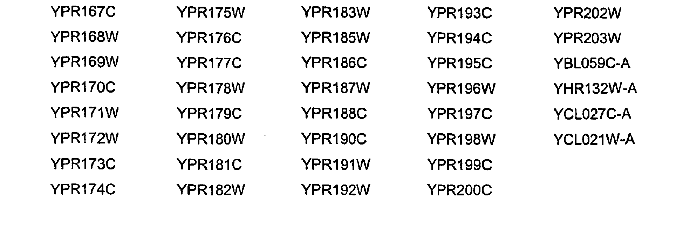

Demnach betrifft die vorliegende Erfindung gemäß einer ganz besonderen Ausführungsform Populationen transgener Pflanzen, die wenigstens etwa 3000, vorzugsweise wenigstens etwa 4500 und vorteilhafterweise wenigstens etwa 5500 kodogene Genabschnitte aus *Saccharomyces cerevisiae* umfassen, wobei von einer ungefähren Gesamtanzahl an kodogenen Genabschnitten von 6300 ausgegangen wird (Mewes et al., Nature 387 (Suppl) Jul 65, 1997). Insbesondere sind die kodogenen Genabschnitte ausgewählt unter den in Figur 1 bezeichneten kodogenen Genabschnitten.

Gemäß einer weiteren besonderen Ausführung ist der Spenderorganismus ein Bakterium der Gattung *Escherichia* vorzugsweise *E. coli.*

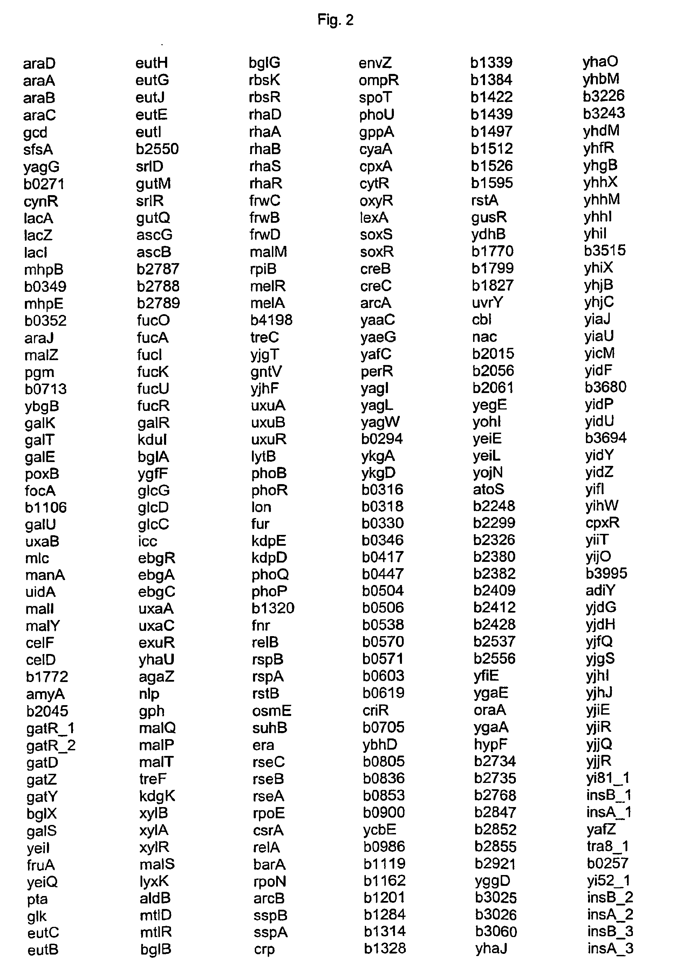

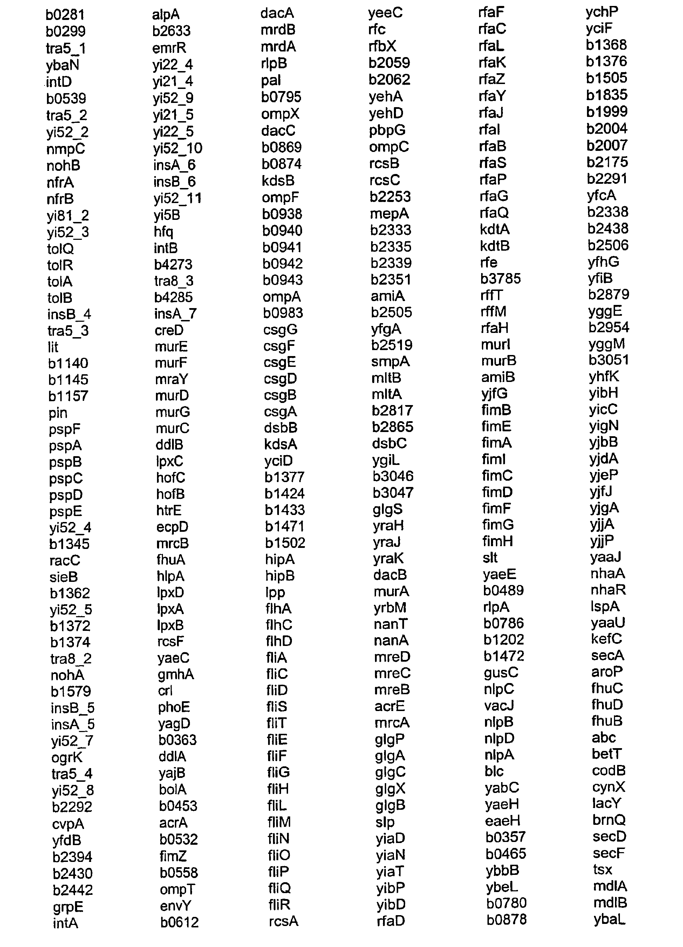

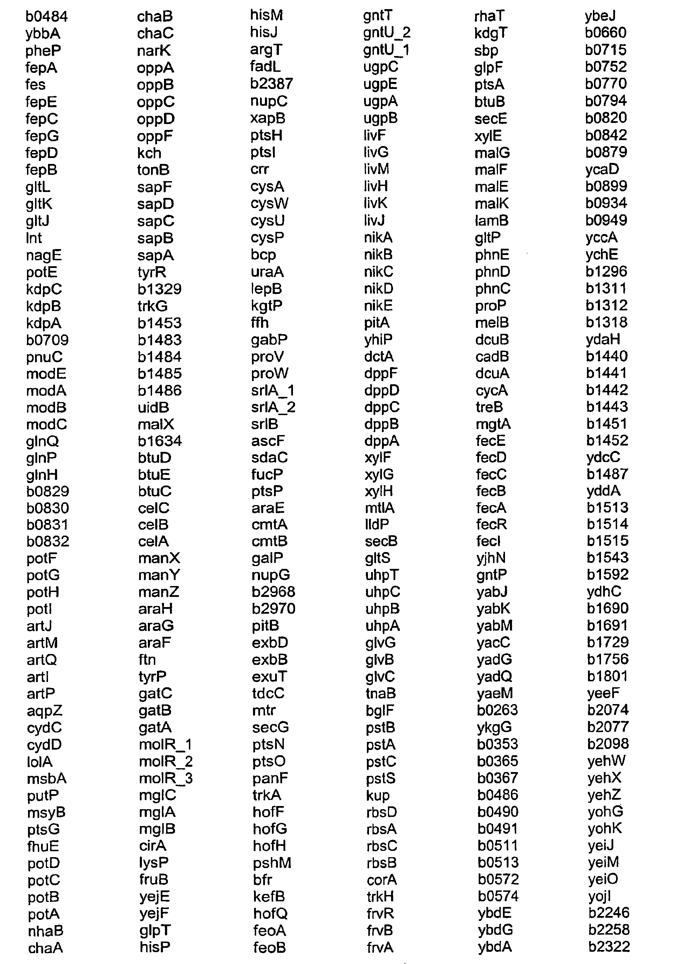

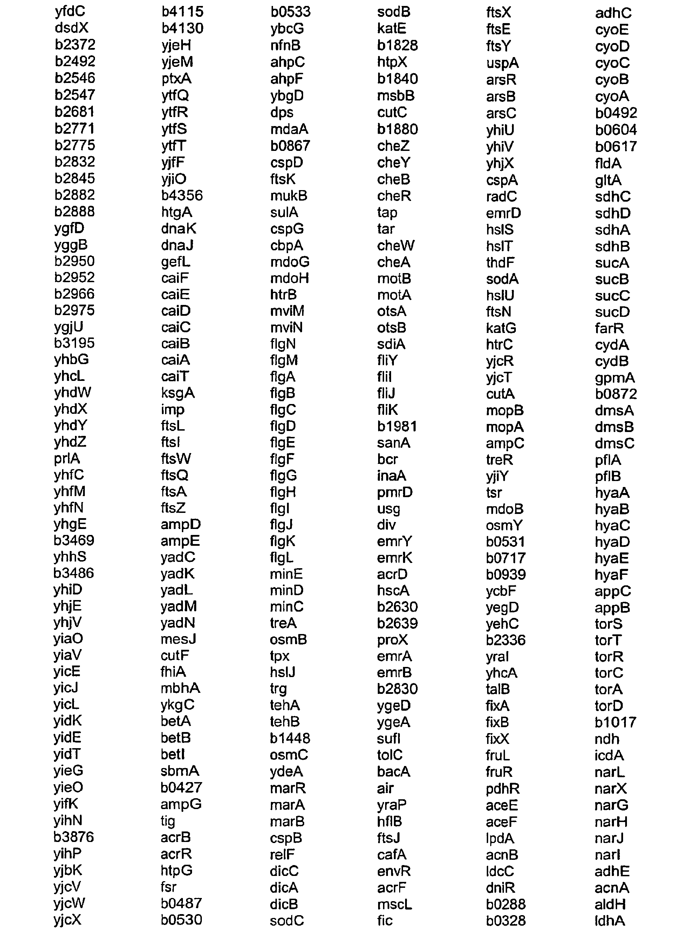

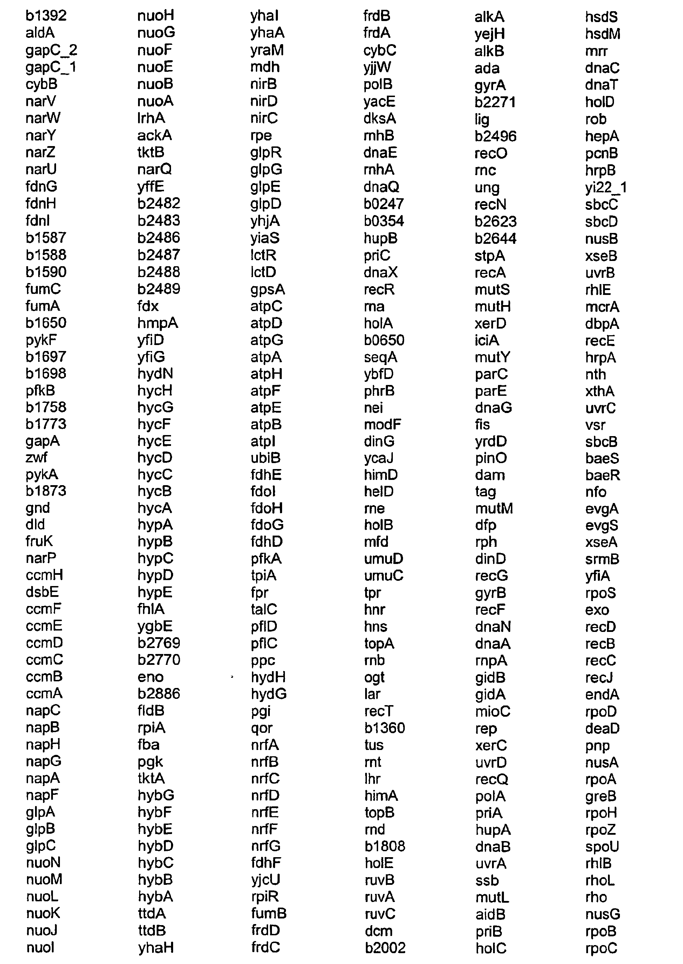

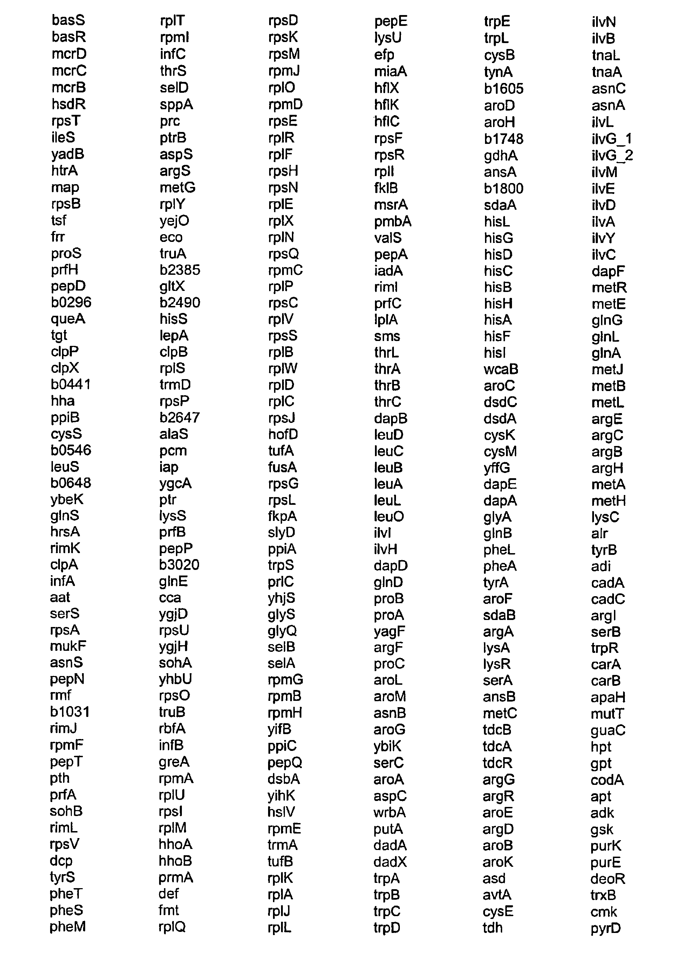

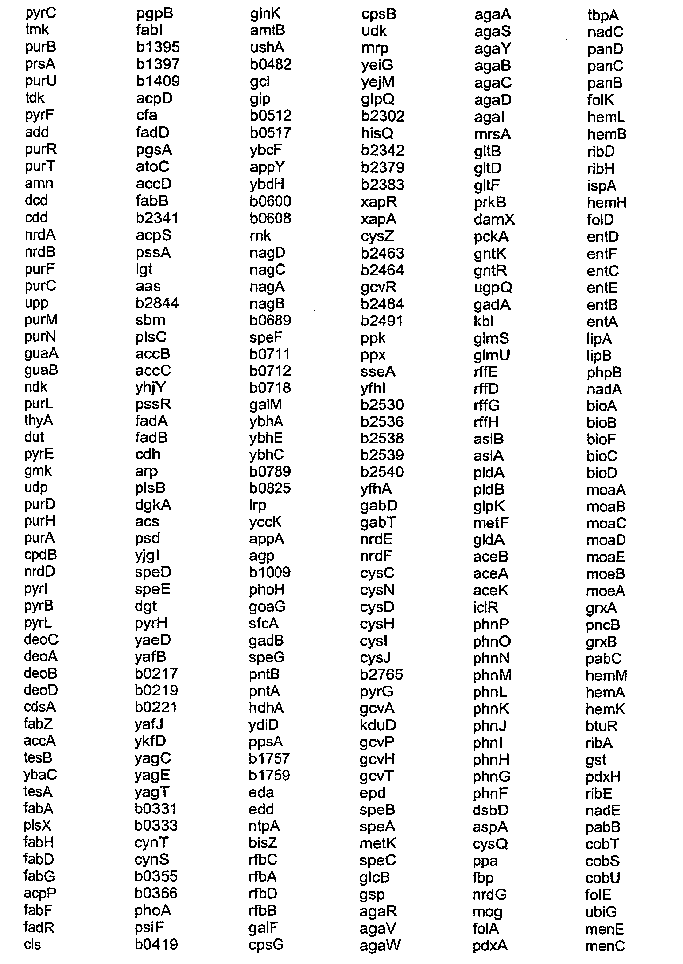

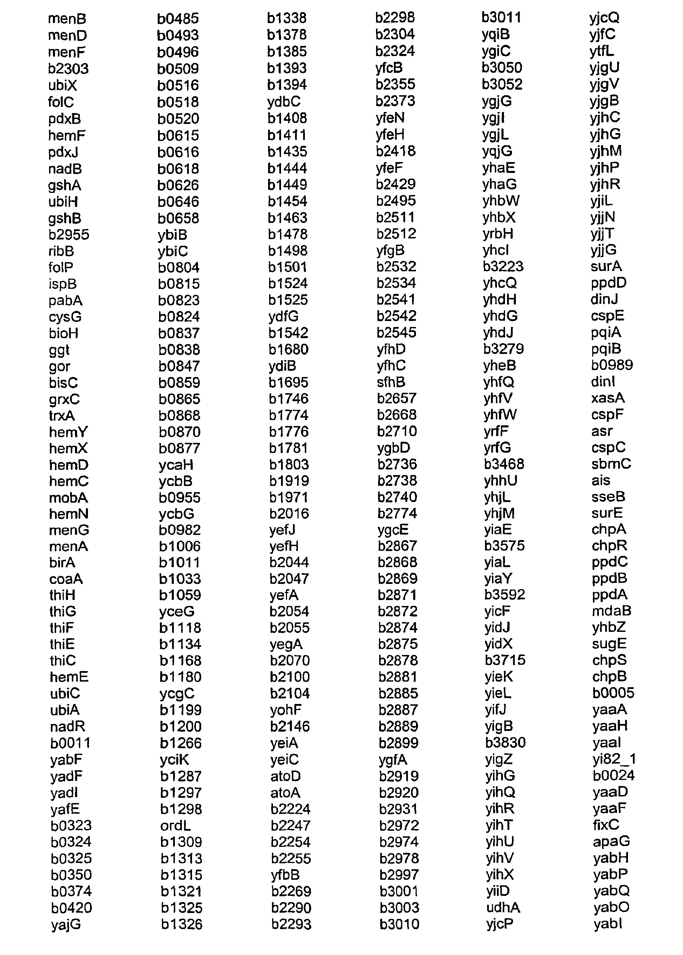

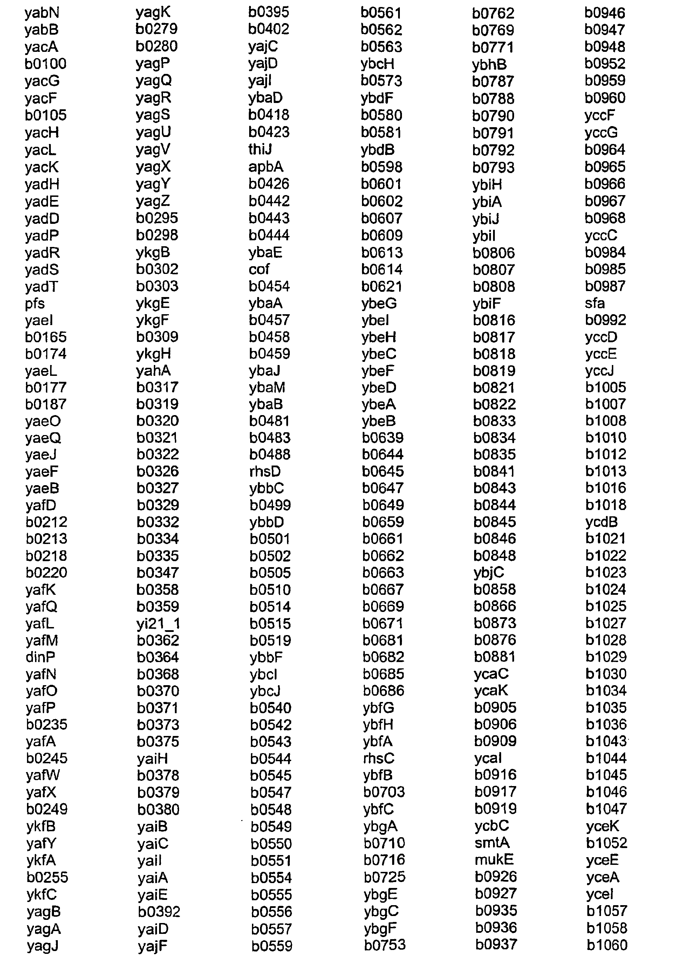

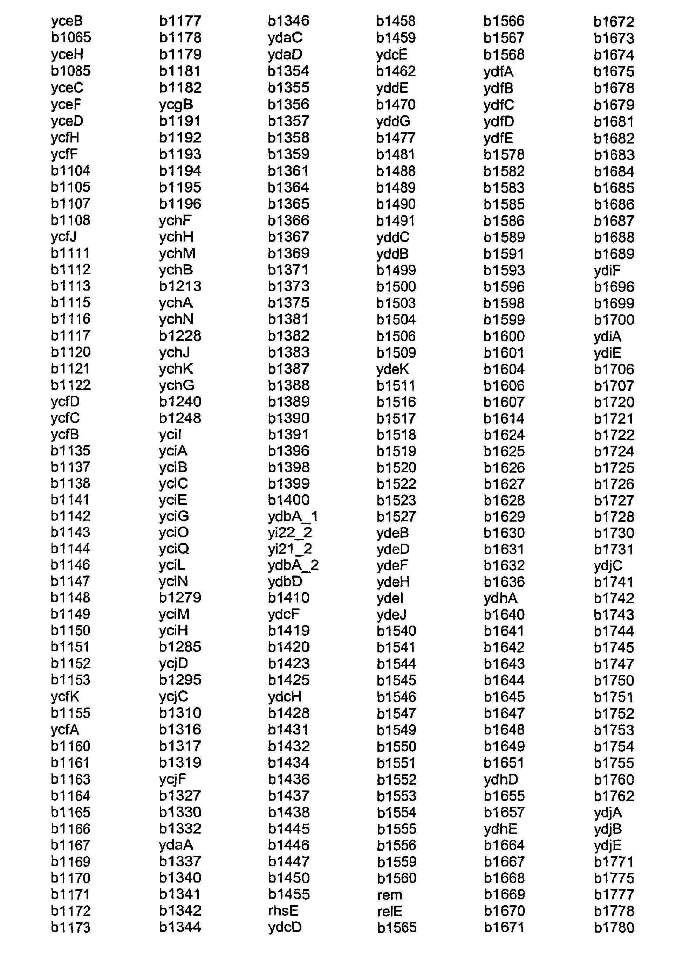

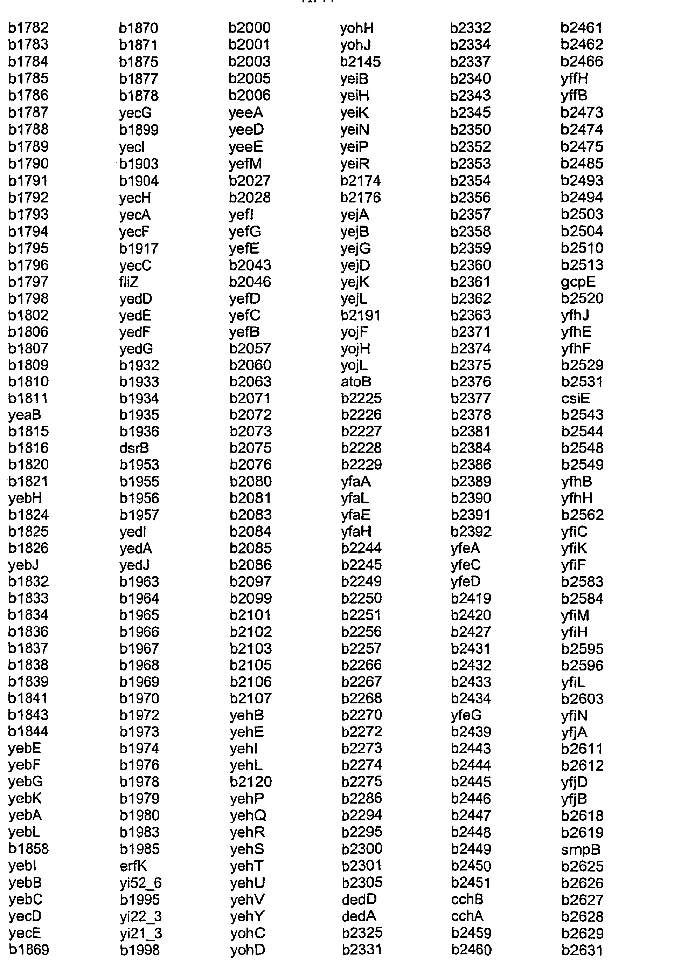

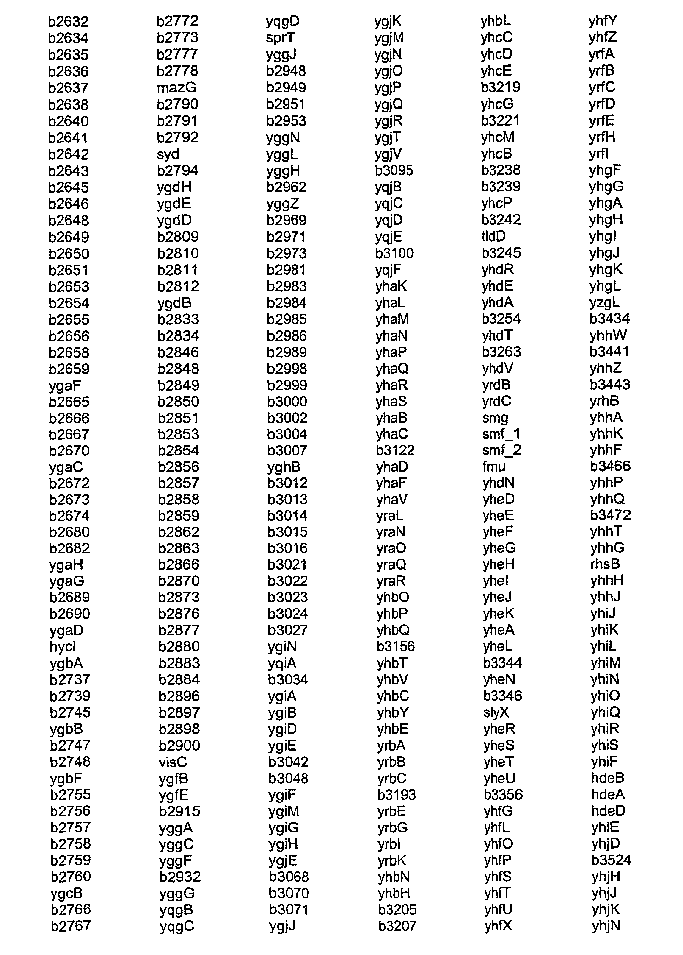

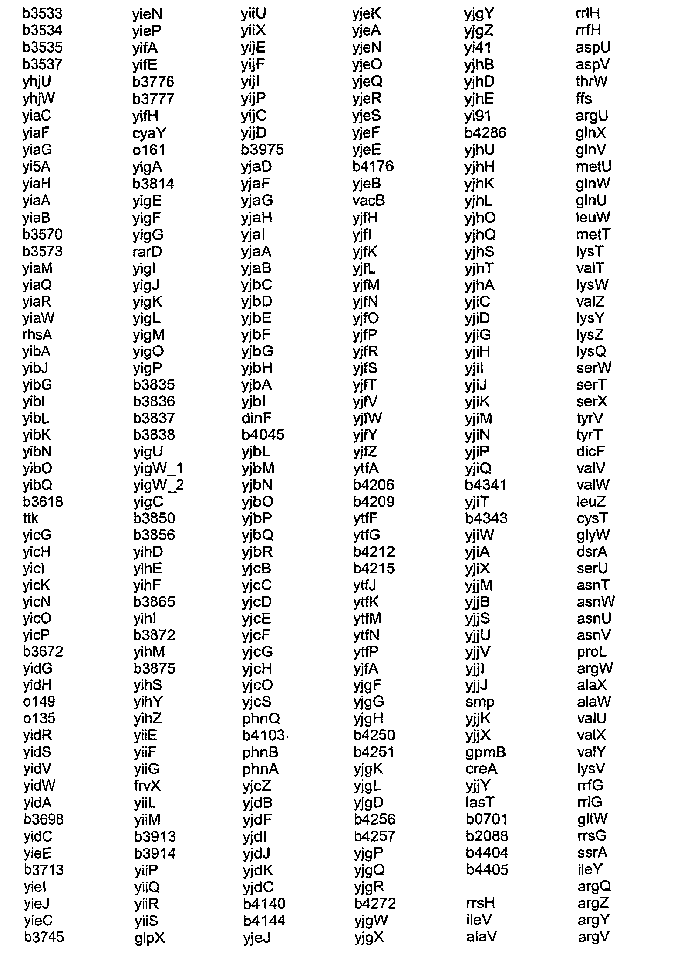

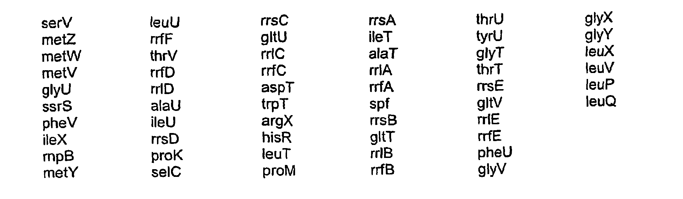

Demnach betrifft die vorliegende Erfindung gemäß einer weiteren ganz besonderen Ausführungsform Populationen transgener Pflanzen, die wenigstens etwa 2000, vorzugsweise wenigstens etwa 3000 und vorteilhafterweise wenigstens etwa 3500 kodogene Genabschnitte aus *E. coli* umfassen, wobei von einer ungefähren Gesamtanzahl an kodogenen Genabschnitten von 4300 ausgegangen wird. Insbesondere sind die kodogenen Genabschnitte ausgewählt unter den in Figur 2 bezeichneten kodogenen Genabschnitten.

Erfindungsgemäße Populationen transgener Pflanzen setzen sich demnach aus mehreren Pflanzenindividuen zusammen. Diese Pflanzenindividuen sind zumindest auf molekularer Ebene unterscheidbar. So gibt es für jeden kodogenen Genabschnitt aus der Gesamtheit der von der erfindungsgemäßen Population transgener Pflanzen umfassten kodogenen Genabschnitte eines bestimmten Spenderorganismus wenigstens ein Individuum der Population, das diesen kodogenen Genabschnitt aufweist. Demnach beinhaltet eine erfindungsgemäße Population transgener Pflanzen, die eine bestimmte Anzahl verschiedener kodogener Genabschnitte eines Spenderorganismus umfasst, zumindest die gleiche Anzahl an Pflanzenindividuen, von denen jedes einzelne dadurch gekennzeichnet ist, dass es einen bestimmten besagter kodogener Genabschnitte aufweist, die restlichen besagter kodogener Genabschnitte jedoch nicht.

Darüber hinaus können erfindungsgemäße Populationen zumindest ein weiteres Pflanzenindividuum umfassen, in dessen Genom ein bestimmter kodogener Genabschnitt eines Spenderorganismus in Kombination mit einem weiteren kodogenen Genabschnitt oder mehreren weiteren kodogenen Genabschnitten des Spenderorganismus integriert ist.

In der Regel sind die kodogenen Genabschnitte im Genom der Pflanzen mit regulatorischen Sequenzen funktional verknüpft. Innerhalb einer Population transgener Pflanzen ist es bevorzugt, für sämtliche heterologe kodogenen Genabschnitte analoge regulatorischen Sequenzen zu verwenden.

Eine funktionale Verknüpfung eines bestimmten kodogenen Genabschnitts mit einer oder mehreren regulatorischen Sequenzen beinhaltet in der Regel eine chemische Fusion von zwei oder mehreren DNA-Fragmenten in geeigneter Orientierung, z.B. in sense- oder antisense-Orientierung, so dass durch die fusionierten Sequenzen ein geeigneter Leserahmen erhalten oder geschaffen wird und eine zweckmäßige Regulation der Expression der DNA-Sequenzen in der Pflanzenzelle gewährleistet ist.

Der Begriff "Expression" meint die Transkription und/oder Translation eines kodogenen Genabschnitts. In der Regel ist das resultierende Produkt ein Protein. Zur Expression gehört allerdings auch die Transkription einer DNA, die in Bezug auf regulatorische Elemente in antisense-Orientierung insertiert ist, wodurch eine antisense-mRNA erzeugt wird. Zu den Produkten gehören auch Ribozyme. Die Expression kann systemisch oder lokal, z.B. auf bestimmte Zelltypen, Gewebe oder Organe beschränkt, erfolgen.

Grundsätzlich kann es sich bei den regulatorischen Sequenzen um Sequenzen der erfindungsgemäßen Empfängerpflanzen, Sequenzen des Spenderorganismus, Sequenzen eines weiteren Organismus oder um synthetische Sequenzen handeln, solange die Funktion dieser Sequenzen in den erfindungsgemäßen Pflanzen gewährleistet ist.

Regulatorische Sequenzen sind in der Regel stromaufwärts (5'), innerhalb und/oder stromabwärts (3') in Bezug auf einen bestimmten kodogenen Genabschnitts angeordnet. Sie kontrollieren insbesondere die Transkription und/oder Translation sowie die Transkriptstabilität des kodogenen Genabschnitts, gegebenenfalls in Zusammenspiel mit weiteren zelleigenen funktionellen Systemen wie dem Proteinbiosynthese-Apparat der Zelle.

Zu regulatorischen Sequenzen gehören vor allem stromaufwärts (5') angeordnete Sequenzen, welche insbesondere die Regulation der Transkriptionsinitiation betreffen, wie Promotoren, und stromabwärts (3') angeordnete Sequenzen, welche vor allem die Regulation der Transkriptionstermination betreffen, wie Polyadenylierungssignale. Promotoren kontrollieren vornehmlich die Expression des kodogenen Genabschnitts, indem sie den Anknüpfungspunkt für RNA-Polymerasen und/oder weitere für eine geeignete Transkriptionsinitiation benötigte Faktoren bieten.

Grundsätzlich sind alle Promotoren einsetzbar, welche die Transkription von Genen in Pflanzen stimulieren können. Geeignete in Pflanzen funktionsfähige Promotoren sind allgemein bekannt. Es kann sich um konstitutive oder induzierbare Promotoren handeln. Geeignete Promotoren können auch entwicklungs- und/oder gewebespezifisch sein, wie Blatt-, Wurzel-, Samen- und Frucht-spezifische Promotoren.

Insbesondere haben sich Promotoren aus pflanzenpathogenen Viren und Bakterien als nützlich erwiesen. Beispiele hierfür sind der 35S-Promotoren aus CaMV, der 34S-Promotor aus FMV oder ein Promotor aus dem Cassava Vein Mosaic Virus (CsVMV), sowie beispielsweise verschiedene Promotoren aus *Agrobacterium tumefaciens,* wie der Octopine-Synthase-Promotor (ocs), der Nopaline-Synthase-Promotor (nos), oder der Mannopine-Synthase-Promotor. Weiterhin stehen zahlreiche pflanzliche Promotoren für die Expression von Genen in bestimmten Geweben, wie Wurzeln, Blüten oder Schließzellen und vor allem Samen, wie beispielsweise der arc5-Promotor aus Bohne, der LeB4-Promotor und der USP-Promotor aus *Vicia faba* sowie der DcG3-Promotor aus Karotte zur Verfügung. Vor allem auch für die Expression in Samen stehen verschiedenen Promotoren zur Verfügung. Weitere samenspezifische Promotoren sind der Sucorse-Binding-Protein-Promotor (WO 00/26388), der Phaseolin-Promtor und der Napin-Promotor. Für die starke Expression heterologer Sequenzen in möglichst vielen Geweben, insbesondere auch Blättern, werden neben verschiedenen der oben genannten viralen und bakteriellen Promotoren, bevorzugt pflanzliche Promotoren von Actin- oder Ubiquitin-Genen, wie beispielsweise der Actin 1-Promotor aus Reis verwendet. Einen weiteres Beispiel für konstitutive pflanzliche Promotoren stellen die V-ATPase-Promtoren aus Zuckerrübe dar (WO 01/14572). Beispielhaft für synthetische konstitutive Promotoren sind der Super-Promotor (WO 95/14098) und von G-Boxen abgeleitete Promotoren (WO 94/12015) zu nennen. Weiterhin können unter Umständen auch chemisch induzierbare Promotoren genutzt werden, vergleiche EP-A 388186, EP-A 335528, WO 97/06268. Auch stehen für die Expression von Genen in Pflanzen blattspezifische Promotoren wie in DE-A 19644478 beschrieben, oder lichtregulierte Promtoren, wie beispielsweise der petE-Promotor aus Erbse zur Verfügung.

Von den Polyadenylierungssignalen ist insbesondere die Poly-A-Additionssequenz aus dem ocs-Gen oder nos-Gen von *Agrobacterium tumefaciens* zu nennen.

Zu weiteren gegebenenfalls zweckmäßigen regulatorischen Sequenzen gehören auch Sequenzen, die den Transport und/oder die Lokalisierung der Expressionsprodukte steuern (Targeting). Hier sind insbesondere die an sich bekannten Signalpeptid oder Transitpeptid kodierenden Sequenzen zu nennen. Beispielsweise gelingt es mit Hilfe von Plastid-Transitpeptid kodierenden Sequenzen, das Expressionsprodukt in die Plastide einer Pflanzenzelle zu leiten.

Als Empfängerpflanzen werden insbesondere Pflanzen bevorzugt, die in zweckmäßiger Weise transformiert werden können. Hierzu gehören mono- und dikotelydone Pflanzen. Insbesondere sind landwirtschaftliche Nutzpflanzen wie Getreide und Gräser, z.B.Triticum spp., Zea mais, Hordeum vulgare, Hafer, Secale cereale, Oryza sativa, Pennisetum glaucum, Sorghum bicolor, Triticale, Agrostis spp., Cenchrus ciliaris, Dactylis glomerata, Festuca arundinacea, Lolium spp., Medicago spp. und Saccharum spp., Hülsenfrüchte und Ölfrüchte, z.B. Brassica juncea, Brassica napus, Glycine max, Arachis hypogaea, Gossypium hirsutum, Cicer arietinum, Helianthus annuus, Lens culinaris, Linum usitatissimum, Sinapis alba, Trifolium repens und Vicia narbonensis, Gemüse und Früchte, z.B. Bananen, Weintrauben, Lycopersicon esculentum, Spargel, Kohl, Wassermelonen, Kiwis, Solanum tuberosum, Beta vulgaris, Cassava und Chicory, Bäume, z.B. Coffea species, Citrus spp., Eucalyptus spp., Picea spp., Pinus spp. und Populus spp., medizinische Pflanzen und Bäume sowie Blumen zu nennen.

Gemäß einer besonderen Ausführungsform betrifft die vorliegende Erfindung transgene Pflanzen der Gattung *Arabidopsis,* z.B. *Arabidopsis thaliana* und der Gattung *Oryza,* z.B. *Oryza sativa.*

Erfindungsgemäß besonders bevorzugte Populationen transgener Pflanzen basieren auf einer Population von Empfängerpflanzen, die im Wesentlichen homogen ist. Demnach ist das wesentliche Unterscheidungsmerkmal einzelner Individuen dieser Populationen die Integration eines oder mehrerer kodogener Genabschnitte eines Spenderorganismus. Allerdings werden aus einer in planta Transformation, die mit einem oder mehreren kodogenen Genabschnitten durchgeführt wurde, in der Regel mehrere transgene Pflanzen erhalten, die dann zusammen mit den jeweiligen direkte Nachkommen Linien eines oder mehrerer kodogener Genabschnitte bilden (Liniensammlung). Dabei kann es zwischen zwei Linien, d.h. individuellen transgenen Pflanzen und deren Nachkommen, in deren Genom der gleiche oder die gleichen kodogenen Genabschnitte eines Spenderorganismus integriert sind, zu einer gewissen Variabilität kommen, die im Wesentlichen auf Unterschiede im Transformationsprozess zurückzuführen ist. Beispielsweise können unterschiedliche Integrationsorte und/oder unterschiedliche Kopienzahlen zwischen den Linien zu einer quantitativ unterschiedlichen Expressionen des oder der kodogenen Genabschnitte führen.

Gemäß einer besonderen Ausführungsform umfassen erfindungsgemäße Populationen wenigstens 5, wenigstens 10 oder wenigstens 20 Linien pro kodogenem Genabschnitt.

Insbesondere ist es bevorzugt, für eine bestimmte Population transgener Pflanzen regulatorische und weitere durch den Transformationsprozess bedingte Sequenzen für unterschiedliche kodogene Genabschnitte einheitlich zu wählen, so dass Unterschiede innerhalb der Population im Wesentlichen auf die unterschiedlichen kodogenen Genabschnitte zurückzuführen sind.

Demnach sind erfindungsgemäße transgene Pflanzen gekennzeichnet durch wenigstens eine transgene Zelle, die einen oder mehrere kodogene Genabschnitte eines Spenderorganismus umfasst und diesen vorteilhafterweise zu exprimieren vermag. Vorzugsweise sind im Wesentliche sämtliche Zellen einer erfindungsgemäßen transgenen Pflanze transgen.

Der erfindungsgemäß verwendete Begriff "transgene Pflanze" bezieht sich auch auf die Nachkommenschaft einer transgenen Pflanze, z.B. die T₁-, T₂-, T₃- und nachfolgende Pflanzengenerationen oder die BC₁-, BC₂-, BC₃- und nachfolgende Pflanzengenerationen. So können die erfindungsgemäßen transgenen Pflanzen aufgezogen und mit sich selbst oder anderen Individuen gekreuzt werden, um weitere erfindungsgemäße transgene Pflanzen zu erhalten. Auch können transgene Pflanzen durch vegetative Propagation transgener Pflanzenzellen erhalten werden.

Gegenstand der vorliegenden Erfindung ist auch von einer erfindungsgemäßen Population transgener Pflanzen ableitbares transgenes pflanzliches Material, das wenigstens 50 % sämtlicher kodogener Genabschnitte des Spenderorganismus umfasst. Hierzu gehören Pflanzenzellen und bestimmte Gewebe, Organe und Teile von Pflanzen in all ihren Erscheinungsformen, wie Samen, Blätter, Antheren, Fasern. Wurzeln, Wurzelhaare, Stängel, Embryos, Kalli, Kotelydonen, Petiolen, Erntematerial, pflanzliches Gewebe, reproduktives Gewebe und Zellkulturen, das von der eigentlichen transgenen Pflanze abgeleitet ist und/oder dazu verwendet werden kann, die transgene Pflanze hervorzubringen. Auch derartiges Material umfasst wenigstens 50 % sämtlicher kodogener Genabschnitte des entsprechenden Spenderorganismus.

Ein weiterer Gegenstand der vorliegenden Erfindung ist ein Verfahren zur Erzeugung einer Population transgener Pflanzen, wobei man
a) einen kodogenen Genabschnitt eines Spenderorganismus bereitstellt;
b) diesen kodogenen Genabschnitt im Genom einer Pflanze integriert; und
c) die Schritte a) und b) für wenigstens 50 % sämtlicher kodogener Genabschnitte des Spenderorganismus an einer entsprechenden Anzahl von Pflanzen durchführt, so dass jede dieser Pflanzen einen, nicht aber einen anderen der kodogenen Genabschnitte aufweist,
wobei der Spenderorganismus ausgewählt ist unter Acetobacter (subgen. Acetobacter) aceti; Acidithiobacillus ferrooxidans; Acinetobacter sp.; Actinobacillus sp; Aeromonas salmonicida; Agrobacterium tumefaciens; Aquifex aeolicus; Arcanobacterium pyogenes; Aster yellows phytoplasma; Bacillus sp.; Bifidobacterium sp.; Borrelia burgdorferi; Brevibacterium linens; Brucella melitensis; Buchnera sp.; Butyrivibrio fibrisolvens; Campylobacter jejuni; Caulobacter crescentus; Chlamydia sp.; Chlamydophila sp.; Chlorobium limicola; Citrobacter rodentium; Clostridium sp.; Comamonas testosteroni; Corynebacterium sp.; Coxiella burnetii; Deinococcus radiodurans; Dichelobacter nodosus; Edwardsiella ictaluri; Enterobacter sp.; Erysipelothrix rhusiopathiae; Escherichia coli; Flavobacterium sp.; Francisella tularensis; Frankia sp. Cpl1; Fusobacterium nucleatum; Geobacillus stearothermophilus; Gluconobacter oxydans; Haemophilus sp.; Helicobacter pylori; Klebsiella pneumoniae; Lactobacillus sp.; Lactococcus lactis; Listeria sp.; Mannheimia haemolytica; Mesorhizobium loti; Methylophaga thalassica; Microcystis aeruginosa; Microscilla sp. PRE1: Moraxella sp. TA144; Mycobacterium sp.; Mycoplasma sp.; Neisseria sp.; Nitrosomonas sp.; Nostoc sp. PCC 7120; Novosphingobium aromaticivorans; Oenococcus oeni; Pantoea citrea; Pasteurella multocida; Pediococcus pentosaceus; Phormidium foveolarum; Phytoplasma sp.; Plectonema boryanum; Prevotella ruminicola; Propionibacterium sp.; Proteus vulgaris; Pseudomonas sp.; Ralstonia sp.; Rhizobium sp.; Rhodococcus equi; Rhodothermus marinus; Rickettsia sp.; Riemerella anatipestifer, Ruminococcus flavefaciens; Saccharomyces cerevisiae; Salmonella sp.; Selenomonas ruminantium; Serratia entomophila; Shigella sp.; Sinorhizobium meliloti; Staphylococcus sp.; Streptococcus sp.; Streptomyces sp.; Synechococcus sp.; Synechocystis sp. PCC 6803; Thermotoga maritima; Treponema sp.; Ureaplasma urealyticum; Vibrio cholerae; Vibrio parahaemolyticus; Xylella fastidiosa; Yersinia sp.; Zymomonas mobilis.

Die zweckmäßige Bereitstellung eines kodogenen Genabschnitts kann in an sich bekannter Weise, z.B. durch übliche Klonierung, erfolgen. Insbesondere kann man genomische DNA oder mRNA mittels cDNA des Spenderorganismus amplifizieren. Die zuerst genannte Vorgehensweise bietet sich bei Spenderorganismen an, deren Genom keine Introns aufweist. Die zuletzt genannte Vorgehensweise ist dann zweckmäßig, wenn das Genom des Spenderorganismus Introns aufweist und diese Introns nicht zusammen mit dem kodogenen Genabschnitt im Genom der Pflanze integriert werden sollen. Man erhält so eine Nukleinsäure-Sequenz, die den gewünschten kodogenen Genabschnitt beinhaltet.

Zur weiteren Klonierung wird die so gewonnene, den kodogenen Genabschnitt beinhaltende Sequenz in der Regel in einen geeigneten Klonierungsvektor insertiert. Geeignet sind vor allem Vektoren, die in geeigneten Zwischenwirten wie bakteriellen Wirtszellen, z.B. *Bacillus, Streptomyces, Salmonella* und vor allem *Escherichia coli,* repliziert werden können. Dem Fachmann sind eine Vielzahl geeigneter Vektoren bekannt und auch Manipulationen, wie Restriktion, Insertion, Ligation, Deletion, etc. gehören zum fachmännischen Können, so dass es in der Regel gelingt, die gewünschte Sequenz, die den kodogenen Genabschnitt gegebenenfalls zusammen mit regulatorischen und/oder weiteren zur Transformation und Expression benötigten Elementen beinhaltet, bereitzustellen.

Gemäß einer bevorzugten Ausführungsform kann man zur Bereitstellung eines kodogenen Genabschnitts diesen Ligations-unabhängig klonieren. Dies hat den Vorteil, dass die kodogenen Genabschnitte weder durch den vorherigen Einsatz von Restriktionsendonukleasen zerschnitten noch über blund-ends bzw. sticky-ends ungerichtet kloniert werden und der Vektor selbst kein Religationspotenzial besitzt, wodurch eine hohe Klonierungseffizienz erzielt wird.

Insbesondere kann man dazu
a1) den kodogenen Genabschnitt unter zufälligem Einbau eines Anteils an Thio-dNTPs amplifizieren;
a2) auf das Amplifikat eine 3'-5'-Exonuklease einwirken lassen, so dass Fragmente mit einzelsträngigen Enden gebildet werden; und
a3) die in Schritt a2) resultierenden Fragmente mit einem Vektor, dessen einzelsträngige Enden zumindest teilweise komplementär sind zu den einzelsträngigen Enden der Fragmente, unter Anlagerungsbedingungen inkubieren.

Die Amplifikation selbst kann in an sich bekannter Weise erfolgen.

Vorzugsweise geht man in Anlehnung an das Protokoll der Pfu-DNA-Polymerase oder eines Pfu/*Taq*-DNA-Polymerasegemisches vor.

Die Primer werden in Anlehnung an die zu amplifizierende Sequenz gewählt. Zweckmäßigerweise sollten die Primer so gewählt werden, dass das Amplifikat die gesamte kodogene Sequenz vom Start- bis zum Stopp-Kodon umfasst. Bevorzugt werden Chimäre eingesetzt, deren 5'-Ende jeweils von einer universellen Adaptersequenz und deren 3'-Ende jeweils von einer spezifischen Sequenz des kodogenen Genabschnitts gebildet werden. Sind die universellen Adaptersequenzen für forward- und reverse-Primer unterschiedlich, ist eine gerichtete Klonierung in den Expressionsvektor möglich.

Im Anschluss an die Amplifikation werden die Amplifikate zweckmäßigerweise analysiert. Beispielsweise kann die Analyse nach gelelektrophoretischer Auftrennung hinsichtlich Qualität und Quantität erfolgen.

Werden für die Amplifikation dNTPs im Gemisch mit Thio-dNTPs eingesetzt, so hat sich ein Verhältnis von 125:1 als zweckmäßig erwiesen, d.h. theoretisch wird an jeder 125. Position des Amplifikats anstelle eines dNTPs ein Thio-dNTP inkorporiert.

Der Einbau von Thio-dNTPs bietet die Möglichkeit, die Amplifikate mit Hilfe von Exonukleasen zu modifizieren. Insbesondere kann man die amplifizierten, in der Regel doppelsträngigen Enden aufweisenden Amplifikate mit dem Enzym Exonuklease **III** inkubieren. Das Enzym besitzt eine 3'-5' Exonukleaseaktivität, so dass die doppelsträngigen DNA-Enden der Amplifikate vom 3'-Ende her abgebaut werden und zwar in Abhängigkeit von Amplifikatmenge, Temperatur, Zeit und Enzymmenge. Zurück bleibt der Gegenstrang mit dem resistenten 5'-Ende. Da dieses Enzym Thio-dNTPs nicht abbauen kann, stoppt der Abbau am ersten eingebauten Thionukleotid. Infolge der Inkorporation der Thio-dNTPs werden die Amplifikate, unabhängig von ihrer Größe und DNA-Konzentration, somit nur begrenzt abgebaut.

Im Anschluss können die modifizierten Amplifikate nach einem Standardprotokoll gereinigt werden (z.B. Qiagen). Ein Aliquot der gereinigten Amplifikate steht dann für die nachfolgende Klonierung zur Verfügung, die obigen Ausführungen zufolge insbesondere Ligations-unabhängig erfolgen kann.

Geeignete Klonierungsvektoren sind dem Fachmann allgemein bekannt. Hierzu gehören insbesondere Vektoren, die in bakteriellen Systemen replizierbar sind, also vor allem Vektoren, die eine effiziente Klonierung in *E*. *coli* gewährleisten, und die stabile Transformation von Pflanzen ermöglichen. Zu nennen sind insbesondere verschiedene für die T-DNA-vermittelte Transformation geeignete, binäre und co-integrierte Vektorsysteme. Derartige Vektorsysteme sind in der Regel dadurch gekennzeichnet, dass sie zumindest die für die Agrobakterium-vermittelte Transformation benötigten *vir*-Gene sowie die T-DNA begrenzenden Sequenzen (T-DNA-Border) beinhalten. Vorzugsweise umfassen diese Vektorsysteme auch weitere cis-regulatorische Regionen wie Promotoren und Terminatoren und/oder Selektionsmarker, mit denen entsprechend transformierte Organismen identifiziert werden können. Während bei co-integrierten Vetorsystemen *vir*-Gene und T-DNA-Sequenzen auf demselben Vektor angeordnet sind, basieren binäre Systeme auf wenigstens zwei Vektoren, von denen einer *vir*-Gene, aber keine T-DNA und ein zweiter T-DNA, jedoch kein *vir*-Gen trägt. Dadurch sind letztere Vektoren relativ klein, leicht zu manipulieren und sowohl in *E-coli* als auch in Agrobakterium zu replizieren. Zu diesen binären Vektoren gehören Vektoren der Serien pBIB-HYG, *pPZP*, pBecks, pGreen. Erfindungsgemäß bevorzugt verwendet werden Bin19, pB1101, pBinAR, pGPTV und pCAMBIA. Eine Übersicht über binäre Vektoren und ihre Verwendung gibt Hellens et al, Trends in Plant Science (2000) 5, 446-451.

Für die Vektorpräparation können die Vektoren zunächst mit Restriktionsendonuklease(n) linearisiert und dann in geeigneter Weise enzymatisch modifiziert werden. Ein weiteres hierfür geeignetes Enzym mit 3'-5' Exonukleaseaktivität ist die T4 DNA-Polymerase, die in Abhängigkeit von Vektormenge, Temperatur, Zeit und Enzymmenge die doppelsträngigen DNA-Enden des linearisierten Vektors zu Einzelstränge abbaut. Im Anschluss wird der Vektor gereinigt und jeweils ein Aliquot für die Ligations-unabhängige Klonierung eingesetzt.

Bei der Ligations-unabhängigen Klonierung werden die enzymatisch modifizierten und erforderlichenfalls gereinigten Amplifikate mit ähnlich präparierten Vektorfragmenten ohne Einsatz von Ligase kloniert. Anstelle dieses üblicherweise verwendeten Enzyms wird das Annealing-Potential der einzelsträngigen Enden der modifizierten Amplifikate und der einzelsträngigen Enden des Vektors, deren Adaptersequenzen kompatibel sind, genutzt. Auf diese Weise erhält man Vektoren, die den oder die gewünschten kodogenen Genabschnitte umfassen (Plasmidkonstrukte).

Gegenstand der vorliegenden Erfindung sind daher auch Kollektionen von Plasmidkonstrukten, die eine Gruppe von Plasmidkonstrukten umfassen, wobei in jedes Konstrukt der Gruppe ein kodogener Genabschnitt eines Spenderorganismus, nicht aber ein anderer kodogener Genabschnitt des Spenderorganismus, integriert ist, die Kollektion erhältlich ist, indem man:
a) einen kodogenen Genabschnitt des Spenderorganismus bereitstellt;
b) diesen kodogenen Genabschnitt in ein Plasmidkonstrukt integriert; und
c) die Schritte a) und b) für wenigstens 50 % sämtlicher kodogener Genabschnitte des Spenderorganismus an einer entsprechenden Anzahl von Plasmidkonstrukten durchführt, so dass jedes dieser Konstrukte einen, nicht aber einen anderen der kodogenen Genabschnitte aufweist,
und der Spenderorganismus ausgewählt ist unter Acetobacter (subgen. Acetobacter) aceti; Acidithiobacillus ferrooxidans; Acinetobacter sp.; Actinobacillus sp; Aeromonas salmonicida; Agrobacterium tumefaciens; Aquifex aeolicus; Arcanobacterium pyogenes; Aster yellows phytoplasma; Bacillus sp.; Bifidobacterium sp.; Borrelia burgdorferi; Brevibacterium linens; Brucella melitensis; Buchnera sp.; Butyrivibrio fibrisolvens; Campylobacter jejuni; Caulobacter crescentus; Chlamydia sp.; Chlamydophila sp.; Chlorobium limicola; Citrobacter rodentium; Clostridium sp.; Comamonas testosteroni; Corynebacterium sp.; Coxiella burnetii; Deinococcus radiodurans; Dichelobacter nodosus; Edwardsiella ictaluri; Enterobacter sp.; Erysipelothrix rhusiopathiae; Escherichia coli, Flavobacterium sp.; Francisella tularensis; Frankia sp. Cpl1; Fusobacterium nucleatum; Geobacillus stearothermophilus; Gluconobacter oxydans; Haemophilus sp.; Helicobacter pylori; Klebsiella pneumoniae; Lactobacillus sp.; Lactococcus lactis; Listeria sp.; Mannheimia haemolytica; Mesorhizobium loti; Methylophaga thalassica; Microcystis aeruginosa; Microscilla sp. PRE1; Moraxella sp. TA144; Mycobacterium sp.; Mycoplasma sp.; Neisseria sp.; Nitrosomonas sp.; Nostoc sp. PCC 7120; Novosphingobium aromaticivorans; Oenococcus oeni; Pantoea citrea; Pasteurella multocida; Pediococcus pentosaceus; Phormidium foveolarum; Phytoplasma sp.; Plectonema boryanum; Prevotella ruminicola; Propionibacterium sp.; Proteus vulgaris; Pseudomonas sp.; Ralstonia sp.; Rhizobium sp.; Rhodococcus equi; Rhodothermus marinus; Rickettsia sp.; Riemerella anatipestifer; Ruminococcus flavefaciens; Saccharomyces cerevisiae; Salmonella sp.; Selenomonas ruminantium; Serratia entomophila; Shigella sp.; Sinorhizobium meliloti; Staphylococcus sp.; Streptococcus sp.; Streptomyces sp.; Synechococcus sp.; Synechocystis sp. PCC 6803; Thermotoga maritima; Treponema sp.; Ureaplasma urealyticum; Vibrio cholerae; Vibrio parahaemolyticus; Xylella fastidiosa; Yersinia sp.; Zymomonas mobilis. Dabei kann ein bestimmtes Plasmidkonstrukt einen oder auch mehrere kodogene Genabschnitte aufweisen. Vorzugsweise sind die kodogenen Genabschnitte in diesen Plasmidkonstrukten mit regulatorischen Sequenzen funktional verknüpft. Zu den regulatorischen Sequenzen gehören insbesondere pflanzliche

Sequenzen wie die oben beschriebenen Promotoren und Terminatoren. Die Plasmidkonstrukte lassen sich vorteilhafterweise in Mikroorganismen, insbesondere *Escherichia coli* und *Agrobacterium turnefaciens,* unter selektiven Bedingungen stabil propagieren und ermöglichen einen Transfer von heterologer DNA in Pflanzen.

Gemäß einer besonderen Ausführungsform basieren die erfindungsgemäßen Plasmidkonstrukte auf binären Vektoren (Übersicht zu binären Vektoren in Hellens et al., 2000). Diese beinhalten in der Regel prokaryotische regulatorische Sequenzen, wie Replikationsursprung und Selektionsmarker, zur Vermehrung in Mikroorganismen wie *Escherichia coli* und *Agrobacterium turnefaciens,* und Agrobakterium-T-DNA-Sequenzen zwecks Transfer von DNA in Pflanzengenome. Von der gesamtem T-DNA-Sequenz aus Agrobakterium wird zumindest die rechte, etwa 25 Basenpaare umfassende Border-Sequenz benötigt. Gewöhnlich beinhalten die erfindungsgemäßen Vektorkonstrukte T-DNA-Sequenzen sowohl aus dem rechten wie auch aus dem linken Begrenzungsbereich, welche zweckmäßige Erkennungsstellen für ortsspezifisch agierende Enzyme, die wiederum von einem Teil der vir-Gene kodiert werden, beinhalten.

Gegenstand der vorliegenden Erfindung sind überdies auch Kollektionen von Wirtsorganismen, welche die erfindungsgemäßen Kollektionen von Plasmidkonstrukten beinhalten. In diesem Sinnen sind die Wirtsorganismen mit den erfindungsgemäßen Plasmidkonstrukten transformiert. Geeignete Wirtsorganismen sind dem Fachmann bekannt. Hierzu zählen vor allem bakterielle Wirte, von denen einige bereits oben im Rahmen von Spender-Mikroorganismen genannt sind, z.B. Bakterien der Gattungen *Bacillus Streptomyces, Salmonella,* und dergleichen. Erfindungsgemäß bevorzugt sind Wirtsorganismen der Gattung *Escherichia,* insbesondere *Escherichia coli, und Agrobakterium,* insbesondere *Agrobacterium turnefaciens.*

Im Übrigen gelten die Ausführungen zu den Pflanzenpopulation analog für die erfindungsgemäßen Kollektionen von Plasmidkonstrukten.

Die resultierenden Plasmidkonstrukte können anschließend zwecks Überprüfung in einen geeigneten Zwischenwirt, z.B. ein Bakterium, übertragen werden. Als zweckmäßig hat sich hier die Transformation in *E*. *coli* erwiesen, die in an sich bekannter Weise, z.B. mittels Hitzeschock oder Elektroporation, durchgeführt werden kann.

So können die transformierten *E. coli*-Kolonien hinsichtlich der Klonierungseffizienz untersucht werden. Dies kann mit Hilfe einer PCR erfolgen. Dabei kann sowohl die Identität als auch die Integrität des Plasmidkonstrukts anhand einer definierten Kolonienzahl überprüft werden, indem man ein Aliquot der Kolonien besagter PCR unterwirft. Hierfür werden in der Regel universelle, von Vektorsequenzen abgeleitete Primer eingesetzt, wobei der forward-Primer stromaufwärts vom Start-ATG und der reverse-Primer stromabwärts vom Stop-Kodon des kodogenen Genabschnitts angeordnet ist. Die Amplifikate werden elektrophoretisch aufgetrennt und hinsichtlich Quantität und Qualität bewertet. Wird ein Fragment in der entsprechenden Größe detektiert, erfolgt eine positive Bewertung.

Die gegebenenfalls überprüften Plasmidkonstrukte werden anschließend für die Transformation der Pflanzen verwendet. Hierfür kann es zunächst erforderlich sein, die Konstrukte aus dem Zwischenwirt zu gewinnen. Beispielsweise lassen sich die Konstrukte als Plasmide aus bakteriellen Wirten in Anlehnung an eine herkömmliche Plasmidisolierung gewinnen.

Es sind zahlreiche Verfahren zur Transformation von Pflanzen bekannt. Da erfindungsgemäß eine stabile Integration heterologer DNA in das Genom von Pflanzen von Vorteil ist, hat sich insbesondere die T-DNA-vermittelte Transformation als zweckmäßig erwiesen.

Hierzu ist es zunächst erforderlich, geeignete Vehikel, insbesondere Agrobakterien, mit dem kodogenen Genabschnitt bzw. dem entsprechenden Plasmidkonstrukt zu transformieren. Dies kann in an sich bekannter Weise erfolgen. Beispielsweise kann man das obigen Ausführungen entsprechend erzeugte Plasmidkonstrukt mittels Elektroporation oder Hitzeschock in kompetente Agrobakterien transformieren.

Prinzipiell ist hierbei zwischen der Bildung co-integrierter Vektoren einerseits und der Transformation mit binären Vektoren zu unterscheiden. Bei der ersten Alternative weisen die den kodogenen Genschnitt umfassenden Vektorkonstrukte keine T-DNA-Sequenzen auf, sondern die Bildung der co-integrierten Vektoren erfolgt in den Agrobakterien durch homologe Rekombination des Vektorkonstruktes mit T-DNA. Die T-DNA liegt in den Agrobakterien in Form von Ti- oder Ri-Plasmiden, in denen die Onkogene zweckmäßigerweise durch exogene DNA ersetzt wurden, vor. Verwendet man binäre Vektoren, so können diese durch bakterielle Konjugation oder direkten Transfer auf Agrobakterien übertragen werden. Diese Agrobakterien enthalten zweckmäßigerweise bereits den Vektor, der die *vir*-Gene trägt (häufig als Helfer-Ti(Ri)-Plasmid bezeichnet).

Zusammen mit dem Plasmidkonstrukt und der T-DNA können zweckmäßigerweise auch ein oder mehrere Marker verwendet werden, anhand derer die Selektion transformierter Agrobakterien und transformierter Pflanzenzellen möglich ist. Für diesen Zweck wurde eine Vielzahl von Markern entwickelt. Hierzu gehören beispielsweise solche, die eine Resistenz gegen Chloramphenicol, Kanamycin, dem Aminoglykosid G418, Hygromycin und ähnliches verleihen.

Gewünschtenfalls können die Plasmidkonstrukte vor ihrer Transformation in Agrobakterien nochmals im Hinblick auf Identität und/oder Integrität überprüft werden. Dies kann beispielsweise in Anlehnung an obige PCR zur Überprüfung der Klonierungseffizienz erfolgen.

In der Regel ist es erwünscht, dass die Plasmidkonstrukte an einer oder beiden Seiten des kodogenen Genabschnitts durch T-DNA flankiert sind. Dies ist insbesondere dann von Nutzen, wenn Bakterien der Arten *Agrobacterium turnefaciens* oder *Agrobacterium rhizogenes* für die Transformation verwendet werden.

Erfindungsgemäß bevorzugt ist die Transformation mit Hilfe von *Agrobacterium turnefaciens.*

Die transformierten Agrobakterien können in an sich bekannter Weise kultiviert werden und stehen damit für eine zweckmäßige Transformation der Pflanzen bereit.

Die zu transformierenden Pflanzen oder Pflanzenteile werden in herkömmlicher Weise angezogen bzw. bereitgestellt. Anschließend lässt man die transformierten Agrobakterien so lange auf die Pflanzen bzw. Pflanzenteile einwirken, bis eine hinreichende Transformationsrate erreicht ist.

Das Einwirken der Agrobakterien auf die Pflanzen bzw. Pflanzenteile kann in unterschiedlicher Art und Weise erfolgen.

Beispielsweise kann man eine Kultur morphogener pflanzlicher Zellen oder Gewebe verwenden. Im Anschluss an den T-DNA-Transfer werden die Bakterien in der Regel durch Antibiotika eliminiert und die Regeneration pflanzlichen Gewebes induziert. Insbesondere verwendet man hierzu geeignete pflanzliche Hormone, um nach anfänglicher Kallus-Bildung die Sproßentwicklung zu fördern.

Erfindungsgemäß bevorzugt erfolgt die Transformation *in planta.* Hierzu kann man beispielsweise die Agrobakterien auf pflanzliche Samen einwirken lassen oder Pflanzenmeristem mit Agrobakterien inokulieren.

Erfindungsgemäß hat es sich insbesondere als zweckmäßig erwiesen, eine Suspension transformierter Agrobakterien auf die gesamte Pflanze oder zumindest die Blütenanlagen einwirken zu lassen. Diese wird anschließend weiter angezogen, bis Samen der behandelten Pflanze gewonnen werden (Clough und Bent, Plant J. (1998) 16, 735-743).

Zur Auswahl transformierter Pflanzen wird das aus der Transformation gewonnene pflanzliche Material in der Regel selektiven Bedingungen unterworfen, so dass transformierte von nichttransformierten Pflanzen unterschieden werden können. Beispielsweise können die in der vorstehend beschriebenen Art und Weise gewonnenen Samen erneut ausgebracht und nach Anzucht einer geeigneten Sprühselektion unterworfen werden. Eine weitere Möglichkeit besteht darin, die Samen, erforderlichenfalls nach Sterilisation, auf Agarplatten unter Verwendung eines geeigneten Selektionsagens so anzuziehen, dass lediglich die transformierten Samen zu Pflanzen anwachsen können.

Gegenstand der vorliegenden Erfindung ist auch die Verwendung einer erfindungsgemäßen Population transgener Pflanzen und/oder eines davon abgeleiteten biologischen Materials für funktionelle Untersuchungen.

Gemäß einer Ausführungsform beziehen sich die funktionellen Untersuchungen auf den Metabolismus der transgenen Pflanzen. So können insbesondere biochemische Parameter untersucht werden, wie die Akkumulation bestimmter Substrate oder Produkte enzymatischer Reaktionen oder die Expression endogener Gene der Empfängerpflanze, beispielsweise anhand des RNA- oder Proteinprofils der Pflanze.

Gemäß einer weiteren Ausführungsform beziehen sich die funktionellen Untersuchungen auf phänotypische Merkmale der transgenen Pflanzen. Zu interessierenden phänotypischen Merkmalen gehören beispielsweise das Wachstum, die Farbe, die Morphologie, das Blühverhalten der Pflanzen und weitere den Phänotyp der Pflanze kennzeichnende Merkmale. Derartige Untersuchungen können durchgeführt werden, indem man einzelne Zellen der transgenen Pflanze bewertet. In der Regel werden solche Untersuchungen aber auf multizellulärer Ebene durch Bewertung organisierter Strukturen, d.h. insbesondere der gesamten Pflanze bzw. Pflanzenteile wie Blätter, Wurzeln, und ähnlichem vorgenommen. Es kann sich daher um makroskopische oder mikroskopische Untersuchungen handeln.

Gemäß einer besonderen Ausführungsform untersucht man wenigstens ein Merkmal, das ausgewählt ist unter Keimungsverhalten, Zahl der Kotelydonen, Fläche der Kotelydonen, Zahl der Rosettenblätter, Fläche der Rosettenblätter, Zahl der Spaltöffnung auf den Rosettenblättern, Form der Rosettenblätter, Trockengewicht der Rosettenblätter, Pflanzenwachstumsrate, Zeitpunkt der Sprossbildung, Verzweigung des Sprosses, Sprosslänge, Zahl der Seitensprossen, Zahl der Blüten, Blütengröße, Blühdauer, Form des Blütenstandes, Pollengröße, Farbe und Pollenzahl, Samengröße, Samenfarbe, Samenform, relatives (bezogen auf Samenvolumen) und absolutes Samengewicht, Samengesamtzahl, Samenzahl pro Schote, Schotenlänge, Schoten Zahl pro Pflanze, Dauer der Samenreifung, Wurzellänge, Wurzelgesamtgewicht, Wurzelverzweigung, Zahl und Länge der Wurzelhaare, Zeitpunkt des Einsetzen der Seneszenz, Dauer der Seneszenz und Pflanzenpigmentierung (Chlorophyllgehalt).

Einem besonderen Aspekt der vorliegenden Erfindung zufolge werden die funktionellen Untersuchungen an Pflanzen durchgeführt, die besonderen Umweltbedingungen ausgesetzt sind. Hierzu gehören abiotische Stressoren, wie Kältestress, Froststress, Trockenstress, Salzstress, Ozonstress, CO₂-Stress, Lichtstress, oxidativer Stress, Hitzestress, anoxischer Stress, Schwermetalle, ionisierende/UV-Strahlung, Nährstoffmangel (z.B. N, P, K, Mikroelemente und ähnliches), biotische Stressoren, wie pflanzliche Krankheitserreger, z.B. Pilze, Plasmodien, Bakterien und Viren, Pflanzenpathogene, wie Nematoden, Protozoen, Schnecken und Insekten, ferner höhere parasitierende Pflanzen, und biologische Interaktionen, wie Symbiosen.

Von besonderer Bedeutung ist die Funktionsbestimmung der kodogenen Genabschnitte aus dem Spenderorganismus in der Empfängerpflanze. Diese Anwendung gilt sowohl für kodogene Genabschnitte, deren Funktion im Spenderorganismus bekannt ist, als auch für kodogene Genabschnitte, deren Funktion im Spenderorganismus nicht oder nur unzureichend aufgeklärt ist. Dabei kann die Funktion bereits durch die transkribierte RNA, beispielsweise im Sinne einer antisense- oder co-Suppression, oder einer regulatorischen RNA ausgeübt werden. In den meisten Fällen bezieht sich die Funktion allerdings auf das translatierte Protein.

Die Funktionsbestimmung kann beispielsweise anhand der oben beschriebenen metabolischen oder phänotypischen Veränderungen erfolgen.

Die erfindungsgemäße Verwendung zu funktionellen Untersuchungen beinhaltet in der Regel ein Verfahren, wobei man
a) die erfindungsgemäße Population transgener Pflanzen bereitstellt;
b) die gewünschte funktionelle Untersuchung durchführt; und
c) das Untersuchungsergebnis mit einem analog an wenigstens einer Referenzpflanze erhaltenen Untersuchungsergebnis vergleicht.

Die Referenzpflanze ist dadurch gekennzeichnet, dass in ihr Genom keiner der von der erfindungemäßen Population umfassten kodogenen Genabschnitte integriert ist oder zumindest nicht exprimiert wird. In diesem Sinne ist die Referenzpflanze als Wildtyp zu bezeichnen, von dem sich die transgenen Pflanzen der erfindungsgemäßen Population durch die Integration und insbesondere die Expression wenigstens eines kodogenen Genabschnitts unterscheiden.

Ergibt sich aus dem Vergleich eine Abweichung, so lässt sich auf eine funktionelle Veränderung der Empfängerpflanze schließen, die mit der Expression eines oder mehrerer bestimmter kodogener Genabschnitte eines Spenderorganismus korreliert.

Die vorliegende Erfindung wird im folgenden anhand von Beispielen näher beschrieben.

### Beispiel 1: Erzeugung der Population transgener Pflanzen

Figur 1 zeigt eine Auflistung 5393 kodogener Genabschnitte aus *Saccharomyces cerevisiae.*
Figur 2 zeigt eine Auflistung kodogener Genabschnitte aus *E*. *coli.*

Die Erzeugung einer erfindungsgemäßen Population transgener Pflanzen wird am Beispiel von *Arabidopsis thaliana* beschrieben, in deren Genom die kodogenen Genabschnitte aus *Saccharomyces cerevisiae* integriert werden. Spezifische Angaben werden dabei exemplarisch für das YKL174C-Gen und das YKR009C-Gen gemacht. Die Integration der übrigen kodogenen Genabschnitte erfolgt in der Regel analog oder erforderlichenfalls in entsprechend modifizierter Form.

### 1. Erzeugung transformierter Agrobakterien

Zur Erzeugung der erfindungsgemäßen Population transgener Pflanzen werden zunächst die zu integrierenden kodogenen Genabschnitte auf Agrobakterien übertragen. Die dazu verwendete, acht Schritte umfassende Prozesskette erlaubt eine effiziente Amplifikation und gerichtete Klonierung der Amplifikate in konstitutive Expressionsvektoren mit Pflanzenselektionsmarkem sowie deren Transformation in Agrobakterien im Hochdurchsatzverfahren.

Die Prozesskette gliedert sich in die folgenden acht Prozess-Schritte: Amplifikation (1 ), Amplifikat-Modifikation (2), Vektorpräparation (3), Ligations-unabhängige Klonierung (4), *E.coli*-Transformation (5), PCR zur Überprüfung der Klonierungseffizienz (6), Plasmidpräparation (7) und Agrobakterien-Transformation (8). Soweit nichts anderes angegeben ist, werden Standardmethoden nach Sambrook et al., Molecular Cloning: A laboratory manual, Cold Spring Harbor 1989, Cold Spring Harbor Laboratory Press, verwendet.

### 1.1. Amplifikation

Die Amplifikation erfolgt in einer 96-well Thermoplatte entsprechend dem Protokoll der Pfu Turbo oder Herculase DNA-Polymerase (Fa. Stratagene). Die Zusammensetzung ist wie folgt: 1x PCR-Puffer [20 mM Tris-HCl (pH 8,8), 2 mM MgSO₄, 10 mM KCI, 10mM (NH₄)SO₄, 0,1 % Triton X-100, 0,1 mg/ml BSA], 0,2 mM α-Thio-dNTP und dNTP (1:125), 100 ng genomische DNA von *Saccharomyces cerevisiae* (Stamm S288C; Fa. Research Genetics, Inc., jetzt Invitrogen), 50 pmol forward-Primer, 50 pmol reverse-Primer, 2,5 u Pfu oder Herculase DNA Polymerase. Die Amplifikationszyklen sind wie folgt: 1 Zyklus für 3' bei 94 °C, gefolgt von 25-30 Zyklen jeweils mit 30" 94 °C, 30" 55 °C und 5-6' 72 °C, gefolgt von 1 Zyklus für 7-10' bei 72 °C, dann 4 °C ∞. Die Amplifikationsprodukte werden elektrophoretisch aufgetrennt und hinsichtlich Quantität und Qualität bewertet. Wird ein Fragment in der entsprechenden Größe detektiert, erfolgt eine positive Bewertung.

Folgende Primer-Sequenzen werden gewählt für das
YKL174C-Gen:
1) forward-Primer (SEQ ID NO:1)
   5'-GGAATTCCAGCTGACCACCATGCCAGAGTATACGCTACTGGC
2) reverse-Primer (SEQ ID NO:2)
   5'-ATCCCCGGGAATTGCCATGTCATATATCATATCTACGATCATGG
YKR009C-Gen:
1) forward-Primer (SEQ ID NO:3)
   5'-GGAATTCCAGCTGACCACCATGCCTGGAAATTTATCCTTCAAAG
2) reverse Primer (SEQ ID NO:4)
   5'-ATCCCCGGGAATTGCCATGTTATAGTTTAGATTTTGCCTGCGATA

### 1.2. Amplifikat-Modifikation

Die Exonuklease III-Behandlung erfolgt in der selben 96-well Thermoplatte wie zuvor die Amplifikation, durch Zugabe von 10 u Exonuklease III (Fa. MBI-Fermentas). Es wird im Cycler 10' bei 20 °C inkubiert und durch Zugabe von Hochsalzpuffer aus dem Qiaquick Purification Kit (Fa. Qiagen) gestoppt. Die Reinigung erfolgt nach dem Qiaquick-Standardprotokoll (Fa. Qiagen).

### 1.3. Vektorpräparation

Die Restriktion mit *N*col und deren Inhibierung wird mit 30 µg Vektor-DNA nach dem *N*col-Protokoll durchgeführt (Fa. MBI-Fermentas). Es wird ein binärer Vektor verwendet, der zwischen den T-DNA-Bordersequenzen eine Selektionskassette (Promoter, Selektionsmarker, Terminator) sowie eine Expressionskassette mit Promotor, Klonierungskassette und Terminatorsequenz, enthält. Außer in der Klonierungskassette besitzt der binäre Vektor keine NcoI-Schnittstelle. Die Klonierungskassette besteht aus der folgenden Sequenz: 5'-GGAATTCCAGCTGACCACCATGGCAATTCCCGGGGATC-3'. Die Nukleasereaktion wird nach Zugabe von 1 u T4-DNA-Polymerase für 2' bei 37°C inkubiert und durch Zugabe von Hochsalzpuffer gestoppt. Die Reinigung der linearisierten und modifizierten Vektorfragmente erfolgt über Nucleobond-Säulen nach Standardprotokoll (Machery-Nagel).

### 1.4. Ligations-unabhängige Klonierung

Es werden ca. 30 ng präparierter Vektor (gemäß 1.3.) und eine definierte Menge modifiziertes Amplifikat (ca. 80 ng, gemäß 1.2) in einer 96-well Thermoplatte gemischt.

Das Annealing erfolgt im Cycler wie folgt: 1 Zyklus für 15' bei 65°C, abkühlen auf 37°C (0,1 °C/1"), 1 Zyklus für 10' bei 37°C, abkühlen auf 4°C (0,1 °C/1"), dann 4 °C ∞.

### 1.5. Escherichia coli-Transformation

Die Transformation erfolgt in der selben 96-well Thermoplatte wie zuvor die Ligations-unabhängige Klonierung, durch Zugabe von kompetenten *E. coli*-Zellen (Stamm DH5α) und Inkubation im Cycler für 20' bei 1°C, gefolgt von einem Hitzeschock für 90" bei 42 °C und Abkühlung auf 4°C. Dann erfolgt die Zugabe von Vollmedium (SOC) und deren Transfer in eine 96-well Deepwell Platte mit Inkubation für 45' bei 37°C. Im Anschluss wird der gesamte Ansatz auf Agarplatten mit Kanamycin ausplattiert und über Nacht bei 37°C inkubiert.

### 1.6. PCR zur Überprüfung der Klonierungseffizienz

Die Amplifikation erfolgt in einer 96-well Thermoplatte entsprechend dem Protokoll der *Taq* DNA-Polymerase (Fa. Gibco-BRL). Die Zusammensetzung ist wie folgt: 1x PCR-Puffer [20 mM Tris-HCL (pH 8,4), 1,5 mM MgCl₂, 50 mM KCI, 0,2 mM dNTP, 5 pmol forward-Primer, 5 pmol reverse-Primer, 0,625 u Taq DNA-Polymerase. Eine definierte Anzahl von Plasmidkonstrukten wird von jeder Agarplatte gepickt und ein Aliquot jeweils in ein mit dem PCR-Mastermix befülltes Well einer 96-well Thermoplatte transferiert. Die Amplifikationszyklen sind wie folgt: 1 Zyklus für 5' bei 94 °C, gefolgt von 35 Zyklen jeweils mit 15" 94 °C, 15" 66 °C und 5' 72 °C, gefolgt von 1 Zyklus für 10' bei 72 °C. dann 4 °C ∞.

Es werden in der Regel vier verschiedene Kolonien pro kodogenem Plasmidkonstrukt analysiert. Dazu werden die entsprechenden Kolonien mit einer Pipettenspitze aufgenommen und in die Lösung für die PCR transferiert.

Es werden Kontrollprimer benutzt, die stromaufwärts und stromabwärts von der Klonierungskassette binden und somit die Amplifikation der Insertion ermöglichen.

Die Amplifikationsprodukte werden elektrophoretisch aufgetrennt und hinsichtlich ihrer Qualität bewertet. Wird ein PCR-Fragment in der entsprechenden Größe detektiert, erfolgt eine positive Bewertung. Aus jeder 4er-Gruppe wir jeweils die Kolonie des ersten positiven PCR-Fragments in die nachfolgende Plasmidpräparation überführt.

### 1.7. Plasmidpräparation

Ein Aliquot positiver Kolonien wird in ein mit Vollmedium (LB) und Kanamycin befülltes Well einer Deepwell-Platte transferiert und über Nacht bei 37 °C inkubiert.

Die Plasmidpräparation erfolgt nach den Vorgaben des Qiaprep-Standardprotokolls (Fa. Qiagen).

### 1.8. Agrobakterien-Transformation

1 ng der isolierten Plasmid-DNA wird mittels Elektroporation in kompetente Zellen von *Agrobacterium tumefaciens,* Stamm GV 3101 pMP90 (Koncz and Schell, Mol. Gen. Gent. 204, 383-396, 1986) in einer 96-well Thermoplatte transformiert. Dann erfolgt die Zugabe von Vollmedium (YEP) und deren Transfer in eine 96-well Deepwell-Platte mit Inkubation für 3 h bei 28 °C. Im Anschluss wird der gesamte Ansatz auf YEP-Agarplatten mit Kanamycin ausplattiert und 48 h bei 28 °C inkubiert.

### 2. Pflanzentransformation und Selektion

Die in den gemäß 1. erzeugten Agrobakterien vorliegenden Plasmidkonstrukte stehen dann für die Pflanzentransformation zur Verfügung.

### 2.1. Anzucht der Bakterien

Es wird mit Hilfe einer Pipettenspitze eine Kolonie von der Agarplatte gepickt und in 3 ml TB Medium flüssig, das auch Kanamycin, Rifampicin und Gentamycin enthält, aufgenommen. Die Vorkultur wächst 48 h bei 28 °C und 120 rpm.

Zur Hauptkultur werden 400 ml LB Medium, das ebenfalls Kanamycin und Gentamycin enthält, verwendet. Die Vorkultur wird in die Hauptkultur überführt. Diese wächst 18 h bei 28 °C und 120 rpm. Nach dem Zentrifugieren bei 4000 rpm wird das Pellet in Infiltrationsmedium (MS-Medium, 10 % Saccharose) resuspendiert.

### 2.2. Anzucht der Pflanzen

Schalen (Piki Saat 80, grün mit Siebboden, 30 x 20 x 4,5 cm, Firma Wiesauplast, Kunststofftechnik, Deutschland) werden mit einem GS 90 Substrat (Einheitserde, Werkverband E.V., Deutschland) bis zur Hälfte gefüllt. Die Schalen werden über Nacht mit 0,05 % Previcur Lösung ( Previcur N, Aventis CropScience) gewässert. *Arabidopsis thaliana* C24 Samen werden auf die Schale gestreut, etwa 1000 Samen je Schale. Die Schalen werden mit einer Haube abgedeckt und in die Stratifizierung gestellt (8 h, 110 µE, 22 °C; 16 h, dunkel, 6 °C). Nach 5 Tagen werden die Schalen in das Kurztag-Phytotron gestellt ( 8h 130 µE, 22 °C; 16 h, dunkel 20 °C). Hier bleiben sie etwa 10 Tage, bis die ersten Laubblätter gebildet sind.

Die Keimlinge werden in Töpfe, die das gleiche Substrat enthalten, transferiert (Teku-Töpfe, 10 cm Ø, Serie LC, Hersteller Pöppelmann GmbH&Co, Deutschland). Es werden neun Pflanzen in einen Topf pikiert. Die Töpfe werden dann wieder zum weiteren Wachstum in das Kurztag-Phytotron gestellt.

Nach 10 Tagen kommen die Pflanzen dann in die Gewächshauskabine (Zusatzbeleuchtung, 16 h, 340 µE, 22 °C; 8 h, dunkel, 20 °C). Hier wachsen sie noch 17 Tage weiter.

### 2.3. Transformation

6 Wochen alte, gerade blühende Arabidopsis-Pflanzen werden 10 sec in die oben beschriebene Agrobakterien-Suspension getaucht. Dieses wurde zuvor mit 10µl Silwett L77 (Crompton S.A., Osi Specialties, Schweiz) versetzt. Die entsprechende Methode ist beschrieben in Clough und Bent, 1998.

Anschließend werden die Pflanzen 18 h in eine feuchte Kammer gelegt. Danach stellt man die Töpfe zum weiteren Wachstum wieder ins Gewächshaus. Hier verbleiben die Pflanzen noch 10 Wochen, bis die Samen geerntet werden können.

### 2.4. Selektion

Je nach verwendetem Resistenzmarker für die Selektion der transformierten Pflanzen werden die geernteten Samen im Gewächshaus ausgebracht und der Sprühselektion unterworfen oder aber nach Sterilisation auf Agarplatten mit dem respektiven Selektionsagens angezogen. Nach ca. 10-14 Tagen unterscheiden sich die transformierten resistenten Pflanzen deutlich von den abgestorbenen Wildtypkeimlingen und können in 6-cm-Töpfe pikiert werden. Zur Selektion im Gewächshaus werden Keimlinge 3 Tage nach der Stratifizierung mit einer wässrigen Lösung aus 4 mg/l Pursuit gesprüht. Diese Selektion wird nach 3 Tagen und 5 Tagen wiederholt. Nach weiteren 2 Tagen können dann deutlich die transformierten, resistenten Keimlinge (Pflänzchen im Vierblattstadium) unterschieden werden von den nicht transformierten Pflänzchen. Die nicht transgenen Keimlinge sind ausgebleicht oder abgestorben. Die transformierten resistenten Pflanzen werden in 6-cm-Töpfe pikiert, dort angezogen und deren Samen gewonnen. Die Samen der transgenen A. *thaliana* Pflanzen werden im Gefrierschrank (bei -20 °C) aufbewahrt.

Auf diese Art und Weise wird eine Population transgener Pflanzen bzw. davon abgeleitetes biologisches Material der Spezies *Arabidopsis thaliana* erzeugt, in deren Genome jeweils ein kodogener Genabschnitt aus *Saccharomyces cerevisiae* integriert ist.

Diese Population wird funktionell untersucht. Als Beispiel dient die phänotypische Untersuchung unter Normal- und Stressbedingungen.

### 3. Beschreibung der morphologischen Analysen.

### 3.1. Bereitstellung des Pflanzenmaterials

Für die Aussaaten werden Plastiktöpfe mit einem Durchmesser von 60 mm verwendet. Als Bodengemisch wird ein Gemisch aus GS-90 Substrat und Quarzsand (4:1 V/V) in der Topfmaschine zusammengemischt und in die Töpfe gefüllt. Danach werden 35 Töpfe in eine Schale zusammengestellt und mit Previcur behandelt. Für die Behandlung werden 25 ml von Previcur in 10 I Leitungswasser aufgenommen. Diese Menge reichte aus, um ca. 200 Töpfe zu behandeln. Die Töpfe werden in die Previcur-Lösung gestellt und von oben zusätzlich mit Leitungswasser ohne Previcur begossen. Die Aussaat findet am gleichen Tag statt.

Für die Aussaat werden die im Gefrierschrank (bei -20 °C) aufbewahrten Samen aus den Reaktionsgefäßen mit Hilfe eines Zahnstochers entnommen und in die Töpfe mit dem Erdgemisch überführt. Insgesamt werden ca. 5-12 Samen in dem Topf mittig verteilt.

Nach der Aussaat werden die Schalen mit den Töpfen mit einer dazu passenden Plastikhaube bedeckt und in die Wachstumskammer bei 16 h Licht (20 °C) und 3-4 Tage Dunkelheit (4 °C) gestellt. Die Feuchtigkeit beträgt ca. 80-90 %. Nach der Stratifizierung werden die Testpflanzen 21 Tage bei einem 16 h-Tag- und 8 h-Nachtrhythmus bei 20 °C und einer Luftfeuchtigkeit von 60 % kultiviert. Als Lichtquelle dienen Vialox-Röhren von Osram, die ein Licht der Farbe SON-T PLUS mit einer Intensität von 220 µE/m²/s erzeugen.

Nach der Stratifizierung, werden die Keimlinge im Alter von 10 Tagen vereinzelt. Die am optimalsten in der Mitte des Topfes gewachsenen Pflanzen werden als Zielpflanze betrachtet. Mit Hilfe von Metallpinzetten werden alle übrigen Pflanzen vorsichtig entfernt und verworfen.

Während des Wachstums werden die Pflanzen zweimal täglich mit Leitungswasser von oben (auf die Erde) gegossen.

### 3.2. Morphologische Analyse

Die Testpflanzen werden im Alter von 21 Tagen nach einem Entwicklungsschlüssel begutachtet. Alle morphologischen Abweichungen von der Referenzpflanze (nicht gentechnisch veränderter Wildtyp) werden dabei registriert. Der Entwicklungsschlüssel gruppiert alle morphologischen Abweichungen der Arabidopsis-Pflanzen in sechs Hauptgruppen. Dabei wird zwischen Pigment-, Kohlkopf-, Zwerg-, Blatt-, Rosetten- und Blühmutanten unterschieden. In der jeweiligen Hauptgruppe werden zwischen zwei und fünf unterschiedliche Phänotype registriert. So wird zum Beispiel in der Hauptgruppe Pigmentmutanten zwischen den Albino/Gelb-Mutanten, Fusca-Mutanten und den dunkelgrünen Mutanten unterschieden. Zusätzlich zu den Hauptmerkmalen werden in der jeweiligen Hauptgruppe andere Merkmale wie die Form der Blätter, der Behaarungsgrad der Blätter, die Größe der Pflanze und die Zeit des Blühens festgehalten.

Mit Hilfe des Entwicklungsschlüssels werden innerhalb der Population mehrere Linien, bei denen eine deutliche morphologische Abweichung zu dem Phänotyp des Wildtyps beobachtet wird, detektiert.

So zeigen Linien (2510), die das YKL174C-Gen (similarity to choline transport protein HNM1P) exprimieren, eine Frühblühung. Dabei blühen die transgenen Pflanzen bereits im Alter von 25 Tagen. Dagegen blühen die nicht transgenen Wildtyp-Pflanzen unter den gleichen Wachstumsbedingungen erst im Alter von 30 Tagen oder mehr. Zusätzlich zu dem Phänomen der Frühblüte können mit Hilfe des Schlüssels andere morphologische Merkmale wie dunkelgrüne Pigmentierung und Kleinwuchs festgestellt werden.

Linien, die das YKR009C-Gen (Hydratase-Dehydrogenase-Epimerase, peroxisomal) exprimieren, zeigen am 21. Tag nach der Keimung einen kleinen Wuchs. Dabei beträgt der Rosettendurchmesser der transgenen Pflanzen ca. 15 mm, wohingegen der Rosettendurchmesser eines gleichaltrigen Wildtyps ca. 40 mm beträgt. Auch die Pigmentierung ist deutlich dunkler, was auf einen erhöhten Chlorophyllgehalt zurückgeführt werden kann.

Weitere Linien, die bestimmte andere kodogene Genabschnitte exprimieren zeigen ebenfalls phänotypische Veränderungen, während bei Expression der restlichen kodogenen Genabschnitte in dem zugrundeliegenden Untersuchungsmodell phänotypische Veränderungen an den transgenen Pflanzen nicht festgestellt werden können.

### 3.3. Trockenstressanalyse

Nach der Untersuchung der morphologischen Merkmale werden die Testpflanzen dem Trockenstresstest ausgesetzt. Um für alle Testpflanzen die gleichen Trockenstressbedingungen zu gewährleisten, werden die Testpflanzen vor Beginn des Trockenstress ausgiebig gewässert. Diese Maßnahme soll den Testpflanzen eine möglichst hohe Homogenität in Bezug auf den Feuchtgehalt zu Beginn des Testes gewährleisten. Um Lichtschäden zu minimieren, wird während des Tests die Lichtintensität auf ca. 150 µEm⁻²s⁻¹ reduziert. Die relative Luftfeuchte wird von 60 % auf 20 % bei 20 °C reduziert. Dabei verläuft die Reduktion der relativen Luftfeuchte schrittweise um 10 % pro Tag. Um positionsspezifische Effekte auszuschließen, rotieren während der ganzen Zeit des Versuches die Anzuchtswannen mit den Testpflanzen in der Kammer.

Die Pflanzen, die eine sensitive Reaktion gegenüber dem Stressor zeigen (sensitiv im Vergleich zu dem Wildtyp), werden am 7., 8., und 9. Tag nach Stressbeginn registriert. Dabei sind die alten Blätter der trockensensitiven Pflanze gelblich bzw. hellgrün verfärbt und liegen gewelkt auf dem Boden. Die jüngeren Blätter sind dagegen eingerollt und ähneln farblich den gesunden Blättern. Die Größe trockensensitiver Pflanzen hingegen entspricht etwa der des Wildtyps. Am 9. Tag nach dem Beginn des Stresses sind die trockensensitiven Pflanzen ausgetrocknet und brüchig.

Pflanzen, die im Vergleich zu Wildtyp-Referenzpflanzen eine Trockenresistenz zeigen, werden täglich zwischen dem 11. und dem 14. Tag nach Stressbeginn beobachtet. Die Beobachtungen werden dokumentiert. Der Phänotyp der trockenresistenten Pflanzen unterscheidet sich dabei nicht von dem Phänotyp einer nicht gestressten Pflanze. Die Wildtyppflanzen hingegen sind nach dem 10. Tag ausgetrocknet.

So bewirkt die Expression bestimmter kodogener Genabschnitte aus *Saccharomyces* cerevisiae (insbesondere die für Glutaredoxin und Thioredoxin kodierenden ORFs YDR51w und YER174c) eine im Vergleich zum Wildtyp verbesserte Trockenresistenz bei *Arabidopsis thaliana,* während die Expression der restlichen kodogenen Genabschnitte keinen Einfluss auf die Trockenresistenz hat.

### 3.4. Froststressanalyse.

Für die Frostanalyse werden die Testpflanzen in der oben beschriebenen Weise angezogen. Im Alter von 21 Tagen werden sie dann dem Froststresstest ausgesetzt. Um die Lichtschäden während des Stresstests zu vermeiden, ist die Lichtintensität während des Tests auf ca. 50 µEm-2s-1 reduziert. Die relative Luftfeuchte wird während des Tests nicht verändert. Der Froststress wird bei folgenden Temperaturen durchgeführt: zunächst 10 Stunden bei -2 °C und anschließend 48 Stunden bei -6 °C. Der Übergang von 20 °C auf -2 °C, von -2 °C auf -6 °C und von -6 °C auf 20 °C dauert jeweils 4 Stunden. Diese Stressbedingungen reichen aus, um die Wildtyp-Pflanzen abzutöten. 3 Tage nach Beendigung des Froststresses werden die Testpflanzen bezüglich des Vorkommens von Resistenzreaktionen untersucht.

Bei den frostresistenten Linien wird ein weiteres Wachstum 3 Tage nach der Beendigung des Stresses beobachtet. Dabei können keine bzw. vereinzelte Frostverletzungen beobachtet werden. Diese sind meistens auf die ältesten Blätter begrenzt. Die Frostverletzungen zeichnen sich durch das Austrocknen und durch die Verfärbung der verletzten Stellen, insbesondere der Blätter aus. Dabei sind die verfärbte Blätter hellgrün bzw. gelb.

So bewirkt die Expression bestimmter kodogener Genabschnitte aus *Saccharomyces cerevisiae* eine im Vergleich zum Wildtyp verbesserte Frostresistenz bei *Arabidopsis thaliana,* während die Expression der restlichen kodogenen Genabschnitte keinen Einfluss auf die Frostresistenz hat.

### 4. Anzucht der Pflanzen für bioanalytische Untersuchungen.

Für die bioanalytische Untersuchung der Pflanzen wird eine schnelle und gleichmäßige Anzucht der Pflanzen benötigt.

Als Bodengemisch wird ein Gemisch aus GS-90 Substrat und Quarzsand (4:1 VN) in der Topfmaschine (Laible System GmbH, Singen, Deutschland) zusammengemischt und in die Töpfe gefüllt. Danach werden 35 Töpfe in eine Schale zusammengestellt und mit Previcur behandelt. Für die Behandlung werden 25 ml Previcur in 10 l Leitungswasser aufgenommen. Diese Menge reichte aus, um ca. 200 Töpfe zu behandeln. Die Töpfe werden in die Previcur-Lösung gestellt und von oben zusätzlich mit Leitungswasser ohne Previcur begossen. Die Aussaat findet am gleichen Tag statt.

Für die Aussaat werden die im Kühlschrank (bei -20 °C) aufbewahrten Samen aus den Eppendorfröhrchen mit Hilfe eines Zahnstochers entnommen und in die Töpfe mit dem Erdgemisch überführt. Insgesamt werden ca. 5-12 Samen in dem Topf mittig verteilt.

Nach der Aussaat werden die Schalen mit den Töpfen mit einer dazu passenden Plastikhaube bedeckt und in die Wachstumskammer bei 16 h Licht bei 20 °C und 8 h Dunkelheit bei 4 °C gestellt. Die Feuchtigkeit beträgt ca. 80-90 % bei einer Dauer der Behandlung von 72 h. Nach der Stratifizierung werden die Testpflanzen 21 Tage bei einem 16 h Licht und 8 h Dunkelrhythmus bei 20 °C, einer Luftfeuchtigkeit von 60 % und einer CO₂-Konzentration von 400 ppm kultiviert. Als Lichtquelle dienen Powerstar HQI-T 250 W/D Daylight Lampen von Osram, die ein dem Sonnenfarbspektrum ähnelndes Licht mit einer Lichtintensität von 220 µE/m²/s erzeugen.

Nach der Stratifizierung werden die Keimlinge im Alter von 10 Tagen vereinzelt. Die am besten in der Mitte des Topfes gewachsenen Pflanzen werden als Zielpflanze betrachtet. Mit Hilfe von Metallpinzetten werden alle übrigen Pflanzen rausgezogen und verworfen.

Während des Wachstums werden die Pflanzen zweimal täglich mit Leitungswasser von oben (auf die Pflanzen/Töpfe) begossen. Die Pflanzen werden nach ca. 22 Tagen bei einem Gewicht von ca. 300-400 mg für die Analyse geerntet.

### 5. Metabolische Analyse

Das Pflanzenmaterial wird nach dem schnellen Ernten in flüssigem Stickstoff schockgefroren, gefriergetrocknet und anschließend mittels beschleunigter Lösungsmittelextraktion (ASE) mit Methanol/Wasser und Methanol/Dichlormethan extrahiert. Nach einer flüssig-flüssig-Verteilung werden Aliquote der organischen und der wässrigen Phase zur Trockne eingeengt. Die Derivatisierung umfasst im Fall der organischen Phase eine Transmethylierung sowie eine anschließende Methoxyaminierung durch Reaktion mit Methoxyamin-Hydrochlorid und Trimethylsilylierung mit MSTFA (N-Methyl-N-(trimethylsilyl)-trifluoracetamid). Die methanolisch/wässrige Phase wird nur mittels Methoxyaminierung und Trimethylsilylierung derivatisiert. Die Endbestimmung der Analyten erfolgt mit GC/EI-MS. Für die LC-MS/MS werden die zur Trockne eingeengten Extrakte lediglich in Fließmittel aufgenommen und dann analysiert.

Auf diese Art und Weise kann das Pflanzenmaterial beispielsweise auf den Gehalt der Aminosäuren Methionin und Threonin untersucht werden. Dazu wird der Methionin- bzw. Threoningehalt flüssig- und gaschromatographisch (LC bzw. GC) für jede transgene Pflanze der oben beschriebenen Population (*Arabidopsis thaliana* mit codogenen Genabschnitten aus *Saccharomyces cerevisiae*) jeweils an 15 transgenenen Linien und weiteren zum Vergleich dienenden Pflanzenproben aus Wildtyppflanzen *(Arabidopsis thaliana)* bestimmt. Zur Auswertung der Chromatogramme werden die gemessenen Peakflächen oder Peakhöhen des zu bestimmenden Analyten (Methionin bzw. Threonin) durch die Peakfläche bzw. die Peakhöhe des jeweiligen internen Standards dividiert. Diese Werte werden auf das für die Pflanze bestimmte Frischgewicht normiert. Dann bezieht man den Wert für jede transgene Pflanze auf die jeweilige Wildtypkontrollgruppe, indem man ihn durch den Mittelwert aus den 5 für die betreffenden Wildtyppflanzen erhaltenen Werten dividiert (Faktor 1).

Zudem bezieht man den Wert für eine transgene Pflanze mit bestimmtem ORF auf den Mittelwert aus den Werten aller anderen 14 transgenen Pflanzen, in deren Genom der ORF nicht integriert ist, und den Werten der 5 Wildtypkontrollen (Faktor 2).

In der folgenden Tabelle sind diese Werte aus zwei unabhängig voneinander durchgeführten Experimenten für die Pflanzen aufgeführt, in deren Genom der ORF YEL046C aus *Saccharomyces cerevisiae* integriert ist.

| **Analyt** | **Faktor 1** | **Faktor 2** | **GC/LC** |
|---|---|---|---|
| Methionin | 3,46-3,58 | 3,31-3,4 | LC |
| Threonin | 0,45-0,15 | 0,61-0,15 | LC |
| Threonin | 0,17-0,16 | 0,18-0,16 | GC |
| Methionin | 3,31-3,67 | 3,5-3,53 | GC |

Demnach weisen diejenigen transgenen Pflanzen, in deren Genom der für die Threonin-Aldolase kodierende ORF YEL046C integriert ist, einen deutlich erhöhten Methionin-Gehalt und einen deutlich erniedrigten Threonin-Gehalt auf. Dies gilt sowohl für den Vergleich mit Wildtyppflanzen (Faktor 1) als auch für den Vergleich mit den übrigen transgenen Pflanzen der untersuchten Population, in deren Genom besagter ORF nicht integriert ist (Faktor 2).

## Patentansprüche

1. Population transgener Pflanzen, die eine Gruppe transgener Pflanzen umfasst, wobei in das Genom jeder transgenen Pflanze der Gruppe ein kodogener Genabschnitt eines Spenderorganismus, nicht aber ein anderer kodogener Genabschnitt des Spenderorganismus, integriert ist, die Population erhältlich ist, indem man:
a) einen kodogenen Genabschnitt des Spenderorganismus bereitstellt;
b) diesen kodogenen Genabschnitt im Genom einer Pflanze integriert; und
c) die Schritte a) und b) für wenigstens 50 % sämtlicher kodogener Genabschnitte des Spenderorganismus an einer entsprechenden Anzahl von Pflanzen durchführt, so dass jede dieser Pflanzen einen, nicht aber einen anderen der kodogenen Genabschnitte aufweist,
und der Spenderorganismus ausgewählt ist unter Acetobacter (subgen. Acetobacter) aceti; Acidithiobacillus ferrooxidans; Acinetobacter sp.; Actinobacillus sp; Aeromonas salmonicida; Agrobacterium tumefaciens; Aquifex aeolicus; Arcanobacterium pyogenes; Aster yellows phytoplasma; Bacillus sp.; Bifidobacterium sp.; Borrelia burgdorferi; Brevibacterium linens; Brucella melitensis; Buchnera sp.; Butyrivibrio fibrisolvens; Campylobacter jejuni; Caulobacter crescentus; Chlamydia sp.; Chlamydophila sp.; Chlorobium limicola, Citrobacter rodentium; Clostridium sp.; Comamonas testosteroni; Corynebacterium sp.; Coxiella burnetii; Deinococcus radiodurans; Dichelobacter nodosus; Edwardsiella ictaluri; Enterobacter sp.; Erysipelothrix rhusiopathiae; Escherichia coli; Flavobacterium sp.; Francisella tularensis; Frankia sp. Cpl1; Fusobacterium nucleatum; Geobacillus stearothermophilus; Gluconobacter oxydans; Haemophilus sp.; Helicobacter pylori; Klebsiella pneumoniae; Lactobacillus sp.; Lactococcus lactis; Listeria sp.; Mannheimia haemolytica; Mesorhizobium loti; Methylophaga thalassica; Microcystis aeruginosa; Microscilla sp. PRE1; Moraxella sp. TA144; Mycobacterium sp.; Mycoplasma sp.; Neisseria sp.; Nitrosomonas sp.; Nostoc sp. PCC 7120; Novosphingobium aromaticivorans; Oenococcus oeni; Pantoea citrea; Pasteurella multocida; Pediococcus pentosaceus; Phormidium foveolarum; Phytoplasma sp.; Plectonema boryanum; Prevotella ruminicola; Propionibacterium sp.; Proteus vulgaris; Pseudomonas sp.; Ralstonia sp.; Rhizobium sp.; Rhodococcus equi; Rhodothermus marinus; Rickettsia sp.; Riemerella anatipestifer; Ruminococcus flavefaciens; Saccharomyces cerevisiae; Salmonella sp.; Selenomonas ruminantium; Serratia entomophila; Shigella sp.; Sinorhizobium meliloti; Staphylococcus sp.; Streptococcus sp.; Streptomyces sp.; Synechococcus sp.; Synechocystis sp. PCC 6803; Thermotoga maritima; Treponema sp.; Ureaplasma urealyticum; Vibrio cholerae; Vibrio parahaemolyticus; Xylella fastidiosa; Yersinia sp.; Zymomonas mobilis.

2. Population nach Anspruch 1, **dadurch gekennzeichnet, dass** die kodogenen Genabschnitte in das nukleare Genom integriert sind.

3. Population nach Anspruch 1 oder 2, **dadurch gekennzeichnet, dass** 1 bis 5 Kopien pro transgener Pflanzenzelle integriert sind.

4. Population nach einem der Ansprüche 1 bis 3, **dadurch gekennzeichnet, dass** die kodogenen Genabschnitte von T-DNA-Sequenzen ein- oder beidseitig flankiert sind.

5. Population nach einem der vorhergehenden Ansprüche, **dadurch gekennzeichnet, dass** die kodogenen Genabschnitte mit regulatorischen Sequenzen funktional verknüpft sind.

6. Population nach Anspruch 5, **dadurch gekennzeichnet, dass** die regulatorischen Sequenzen Signal- und/oder Transitpeptid kodierende Sequenzen beinhalten.

7. Population nach einem der vorhergehenden Ansprüche, **dadurch gekennzeichnet, dass** die Population zumindest eine weitere Pflanze umfasst, in deren Genom ein kodogener Genabschnitt in Kombination mit einem weiteren kodogenen Genabschnitt oder mehreren weiteren kodogenen Genabschnitten des Spenderorganismus integriert ist.

8. Population nach einem der vorhergehenden Ansprüche, **dadurch gekennzeichnet, dass** die transgenen Pflanzen zur Gattung *Arabidopsis* gehören.

9. Population nach einem der Ansprüche 1 bis 7, **dadurch gekennzeichnet, dass** die transgenen Pflanzen zur Gattung *Oryza* gehören.

10. Verfahren zur Erzeugung einer Population transgener Pflanzen, wobei man
a) einen kodogenen Genabschnitt eines Spenderorganismus bereitstellt;
b) diesen kodogenen Genabschnitt im Genom einer Pflanze integriert; und
c) die Schritte a) und b) für wenigstens 50 % sämtlicher kodogener Genabschnitte des Spenderorganismus an einer entsprechenden Anzahl von Pflanzen durchführt, so dass jede dieser Pflanzen einen, nicht aber einen anderen der kodogenen Genabschnitte aufweist,
wobei der Spenderorganismus ausgewählt ist unter Acetobacter (subgen. Acetobacter) aceti; Acidithiobacillus ferrooxidans; Acinetobacter sp.; Actinobacillus sp; Aeromonas salmonicida; Agrobacterium tumefaciens; Aquifex aeolicus; Arcanobacterium pyogenes; Aster yellows phytoplasma; Bacillus sp.; Bifidobacterium sp.; Borrelia burgdorferi; Brevibacterium linens; Brucella melitensis; Buchnera sp.; Butyrivibrio fibrisolvens; Campylobacter jejuni; Caulobacter crescentus; Chlamydia sp.; Chlamydophila sp.; Chlorobium limicola; Citrobacter rodentium; Clostridium sp.; Comamonas testosteroni; Corynebacterium sp.; Coxiella burnetii: Deinococcus radiodurans; Dichelobacter nodosus; Edwardsiella ictaluri; Enterobacter sp.; Erysipelothrix rhusiopathiae; Escherichia coli; Flavobacterium sp.; Francisella tularensis; Frankia sp. CpI1; Fusobacterium nucleatum; Geobacillus stearothermophilus; Gluconobacter oxydans; Haemophilus sp.; Helicobacter pylori; Klebsiella pneumoniae; Lactobacillus sp.; Lactococcus lactis; Listeria sp.; Mannheimia haemolytica; Mesorhizobium loti; Methylophaga thalassica; Microcystis aeruginosa; Microscilla sp. PRE1; Moraxella sp. TA144; Mycobacterium sp.; Mycoplasma sp.; Neisseria sp.; Nitrosomonas sp.; Nostoc sp. PCC 7120; Novosphingobium aromaticivorans; Oenococcus oeni; Pantoea citrea; Pasteurella multocida; Pediococcus pentosaceus; Phormidium foveolarum; Phytoplasma sp.; Plectonema boryanum; Prevotella ruminicola; Propionibacterium sp.; Proteus vulgaris; Pseudomonas sp.; Ralstonia sp.; Rhizobium sp.; Rhodococcus equi; Rhodothermus marinus; Rickettsia sp.; Riemerella anatipestifer, Ruminococcus flavefaciens; Saccharomyces cerevisiae; Salmonella sp.; Selenomonas ruminantium; Serratia entomophila; Shigella sp.; Sinorhizobium meliloti; Staphylococcus sp.; Streptococcus sp.; Streptomyces sp.; Synechococcus sp.; Synechocystis sp. PCC 6803; Thermotoga maritima; Treponema sp.; Ureaplasma urealyticum; Vibrio cholerae; Vibrio parahaemolyticus; Xylella fastidiosa; Yersinia sp.; Zymomonas mobilis.

11. Verfahren nach Anspruch 10, **dadurch gekennzeichnet, dass** man zur Bereitstellung des kodogenen Genabschnitts Bakterien aus der Gattung *Agrobacterium* mit dem kodogenen Genabschnitt transformiert.

12. Verfahren nach Anspruch 11, **dadurch gekennzeichnet, dass** das Bakterium *Agrobacterium tumefaciens* ist.

13. Verfahren nach Anspruch 10, **dadurch gekennzeichnet, dass** man den kodogenen Genabschnitt in das Genom einer Pflanze integriert, indem man
b1) Agrobakterien, die mit dem kodogenen Genabschnitt transformiert sind, auf die Pflanze oder geeignetes biologisches Material davon einwirken lässt; und
b2) die transgene Pflanze oder geeignetes biologisches Material davon gewinnt.

14. Verfahren nach Anspruch 13, **dadurch gekennzeichnet, dass** man die Agrobakterien *in planta* einwirken lässt.

15. Verfahren nach Anspruch 13, **dadurch gekennzeichnet, dass** man zur Gewinnung der transgenen Pflanzen oder geeigneten biologischen Materials davon zunächst die Samen der mit Agrobakterien behandelten Pflanzen gewinnt, diese aussät und selektierenden Bedingungen unterwirft, und die transformierten Pflanzen oder geeignetes biologisches Material davon gewinnt.

16. Biologisches transgenes Material, das in an sich bekannter Weise aus einer Population transgener Pflanzen nach einem der Ansprüche 1 bis 9 erhältlich ist und wenigstens 50 % sämtlicher kodogener Genabschnitte des Spenderorganismus umfasst.

17. Kollektion von Plasmidkonstrukten, die eine Gruppe von Plasmidkonstrukten umfasst, wobei in jedes Konstrukt der Gruppe ein kodogener Genabschnitt eines Spenderorganismus, nicht aber ein anderer kodogener Genabschnitt des Spenderorganismus, integriert ist, die Kollektion erhältlich ist, indem man:
a) einen kodogenen Genabschnitt des Spenderorganismus bereitstellt;
b) diesen kodogenen Genabschnitt in ein Plasmidkonstrukt integriert; und
c) die Schritte a) und b) für wenigstens 50 % sämtlicher kodogener Genabschnitte des Spenderorganismus an einer entsprechenden Anzahl von Plasmidkonstrukten durchführt, so dass jedes dieser Konstrukte einen, nicht aber einen anderen der kodogenen Genabschnitte aufweist,
und der Spenderorganismus ausgewählt ist unter Acetobacter (subgen. Acetobacter) aceti; Acidithiobacillus ferrooxidans; Acinetobacter sp.; Actinobacillus sp; Aeromonas salmonicida; Agrobacterium tumefaciens: Aquifex aeolicus; Arcanobacterium pyogenes; Aster yellows phytoplasma; Bacillus sp.; Bifidobacterium sp.; Borrelia burgdorferi; Brevibacterium linens; Brucella melitensis; Buchnera sp.; Butyrivibrio fibrisolvens; Campylobacter jejuni; Caulobacter crescentus; Chlamydia sp.; Chlamydophila sp.; Chlorobium limicola; Citrobacter rodentium; Clostridium sp.; Comamonas testosteroni; Corynebacterium sp.; Coxiella burnetii; Deinococcus radiodurans; Dichelobacter nodosus; Edwardsiella ictaluri; Enterobacter sp.; Erysipelothrix rhusiopathiae: Escherichia coli; Flavobacterium sp.; Francisella tularensis; Frankia sp. Cpl1; Fusobacterium nucleatum; Geobacillus stearothermophilus; Gluconobacter oxydans; Haemophilus sp.; Helicobacter pylori; Klebsiella pneumoniae; Lactobacillus sp.; Lactococcus lactis; Listeria sp.; Mannheimia haemolytica; Mesorhizobium loti; Methylophaga thalassica; Microcystis aeruginosa; Microscilla sp. PRE1; Moraxella sp. TA144; Mycobacterium sp.; Mycoplasma sp.; Neisseria sp.; Nitrosomonas sp.; Nostoc sp. PCC 7120; Novosphingobium aromaticivorans; Oenococcus oeni; Pantoea citrea; Pasteurella multocida; Pediococcus pentosaceus; Phormidium foveolarum; Phytoplasma sp.; Plectonema boryanum; Prevotella ruminicola; Propionibacterium sp.; Proteus vulgaris; Pseudomonas sp.; Ralstonia sp.; Rhizobium sp.; Rhodococcus equi; Rhodothermus marinus; Rickettsia sp.; Riemerella anatipestifer; Ruminococcus flavefaciens; Saccharomyces cerevisiae; Salmonella sp.; Selenomonas ruminantium; Serratia entomophila; Shigella sp.; Sinorhizobium meliloti; Staphylococcus sp.; Streptococcus sp.; Streptomyces sp.; Synechococcus sp.; Synechocystis sp. PCC 6803; Thermotoga maritima; Treponema sp.; Ureaplasma urealyticum; Vibrio cholerae; Vibrio parahaemolyticus; Xylella fastidiosa; Yersinia sp.; Zymomonas mobilis.

18. Kollektion nach Anspruch 17, **dadurch gekennzeichnet, dass** die kodogenen Genabschnitte mit regulatorischen Sequenzen funktional verknüpft sind.

19. Kollektion nach Anspruch 18, **dadurch gekennzeichnet, dass** die regulatorischen Sequenzen pflanzlich sind.

20. Kollektion nach einem der Ansprüche 17 bis 19, **dadurch gekennzeichnet, dass** die Konstrukte auf binären Vektoren basieren.

21. Population eines Wirtsorganismus, welche die Kollektion von Plasmidkonstrukten nach einem der Ansprüche 17 bis 20 beinhaltet.

22. Verfahren zur funktionellen Untersuchung einer Population nach einem der Ansprüche 1 bis 9, wobei man
a) die Population bereitstellt;
b) gegebenenfalls die Pflanzen Stress-Bedingungen aussetzt; und
b) ein phänotypisches Merkmal oder den Metabolismus jeder Pflanzen der Population mit dem Phänotyp bzw. Metabolismus einer Referenzpflanze vergleicht,
wobei im Genom der Referenzpflanze keiner der von der Population umfassten kodogenen Genabschnitte des Spenderorganismus integriert ist.

23. Verfahren nach Anspruch 22, wobei das phänotypische Merkmal ausgewählt ist unter Wachstum, Farbe, Morphologie und Blühverhalten der Pflanzen.

24. Verfahren nach einem der Ansprüche 22 oder 23, wobei der Stress abiotisch oder biotisch ist.

25. Verfahren nach einem der Ansprüche 22 bis 24, wobei der Stress Kältestress oder Trockenstress ist.

26. Verwendung einer Population transgener Pflanzen nach einem der Ansprüche 1 bis 9 oder eines davon abgeleiteten biologischen Materials nach Anspruch 16, zur Funktionsbestimmung der kodogenen Genabschnitte.

## Claims

1. Population of transgenic plants which comprises a group of transgenic plants, where a codogenic gene segment of a donor organism, but not a different codogenic gene segment of the donor organism, is integrated into the genome of each transgenic plant of the group, the population being obtainable by:
a) providing a codogenic gene segment of the donor organism;
b) integrating this codogenic gene segment into the genome of a plant; and
c) carrying out steps a) and b) for at least 50% of all of the codogenic gene segments of the donor organism on a suitable number of plants, so that each of these plants has one, but not a different one, of the codogenic gene segments,
and wherein the donor organism is selected among Acetobacter (subgen. Acetobacter) aceti; Acidithiobacillus ferrooxidans; Acinetobacter sp.; Actinobacillus sp; Aeromonas salmonicida; Agrobacterium tumefaciens; Aquifex aeolicus; Arcanobacterium pyogenes; Aster yellows phytoplasma; Bacillus sp.; Bifidobacterium sp.; Borrelia burgdorferi; Brevibacterium linens; Brucella melitensis; Buchnera sp.; Butyrivibrio fibrisolvens; Campylobacter jejuni; Caulobacter crescentus; Chlamydia sp.; Chlamydophila sp.; Chlorobium limicola; Citrobacter rodentium; Clostridium sp.;
Comamonas testosteroni; Corynebacterium sp.; Coxiella burnetii; Deinococcus radiodurans; Dichelobacter nodosus; Edwardsiella ictaluri; Enterobacter sp.; Erysipelothrix rhusiopathiae; Escherichia coli; Flavobacterium sp.; Francisella tularensis; Frankia sp. CpI1; Fusobacterium nucleatum; Geobacillus stearothermophilus; Gluconobacter oxydans; Haemophilus sp.; Helicobacter pylori; Klebsiella pneumoniae; Lactobacillus sp.; Lactococcus lactis; Listeria sp.; Mannheimia haemolytica; Mesorhizobium loti; Methylophaga thalassica; Microcystis aeruginosa; Microscilla sp. PRE1; Moraxella sp. TA144; Mycobacterium sp.; Mycoplasma sp.; Neisseria sp.; Nitrosomonas sp.; Nostoc sp. PCC 7120; Novosphingobium aromaticivorans; Oenococcus oeni; Pantoea citrea; Pasteurella multocida; Pediococcus pentosaceus; Phormidium foveolarum; Phytoplasma sp.; Plectonema boryanum; Prevotella ruminicola; Propionibacterium sp.; Proteus vulgaris; Pseudomonas sp.; Ralstonia sp.; Rhizobium sp.; Rhodococcus equi; Rhodothermus marinus; Rickettsia sp.; Riemerella anatipestifer; Ruminococcus flavefaciens; Saccharomyces cerevisiae; Salmonella sp.; Selenomonas ruminantium; Serratia entomophila; Shigella sp.; Sinorhizobium meliloti; Staphylococcus sp.; Streptococcus sp.; Streptomyces sp.; Synechococcus sp.; Synechocystis sp. PCC 6803; Thermotoga maritima; Treponema sp.; Ureaplasma urealyticum; Vibrio cholerae; Vibrio parahaemolyticus; Xylella fastidiosa; Yersinia sp.; Zymomonas mobilis.

2. The population according to claim 1, wherein the codogenic gene segments are integrated into the nuclear genome.

3. The population as claimed in claim 1 or 2, wherein 1 to 5 copies are integrated per transgenic plant cell.

4. The population as claimed in any of claims 1 to 3, wherein the codogenic gene segments are unilaterally or bilaterally flanked by T-DNA sequences.

5. The population as claimed in any of the preceding claims, wherein the codogenic gene segments are linked operably to regulatory sequences.

6. The population as claimed in claim 5, wherein the regulatory sequences contain signal sequences and/or transit-peptide-encoding sequences.

7. The population as claimed in any of the preceding claims, wherein the population encompasses at least one further plant in whose genome a codogenic gene segment is integrated in combination with one or more further codogenic segment(s) of the donor organism.

8. The population as claimed in one of the preceding claims, wherein the transgenic plants belong to the genus *Arabidopsis.*

9. The population as claimed in any of claims 1 to 7, wherein the transgenic plants belong to the genus *Oryza.*

10. A method of generating a population of transgenic plants, in which
a) a codogenic gene segment of a donor organism is provided;
b) this codogenic gene segment is integrated into the genome of a plant; and
c) steps a) and b) are carried out for at least 50% of all of the codogenic gene segments of the donor organism on a suitable number of plants, so that each of these plants has one, but not a different one, of the codogenic gene segments,
where the donor organism is selected among Acetobacter (subgen. Acetobacter) aceti; Acidithiobacillus ferrooxidans; Acinetobacter sp.; Actinobacillus sp; Aeromonas salmonicida; Agrobacterium tumefaciens; Aquifex aeolicus; Arcanobacterium pyogenes; Aster yellows phytoplasma; Bacillus sp.; Bifidobacterium sp.; Borrelia burgdorferi; Brevibacterium linens; Brucella melitensis; Buchnera sp.; Butyrivibrio fibrisolvens; Campylobacter jejuni; Caulobacter crescentus; Chlamydia sp.; Chlamydophila sp.; Chlorobium limicola; Citrobacter rodentium; Clostridium sp.; Comamonas testosteroni; Corynebacterium sp.; Coxiella burnetii; Deinococcus radiodurans; Dichelobacter nodosus; Edwardsiella ictaluri; Enterobacter sp.; Erysipelothrix rhusiopathiae; Escherichia coli; Flavobacterium sp.; Francisella tularensis; Frankia sp. CpI1; Fusobacterium nucleatum; Geobacillus stearothermophilus; Gluconobacter oxydans; Haemophilus sp.; Helicobacter pylori; Klebsiella pneumoniae; Lactobacillus sp.; Lactococcus lactis; Listeria sp.; Mannheimia haemolytica; Mesorhizobium loti; Methylophaga thalassica; Microcystis aeruginosa; Microscilla sp. PRE1; Moraxella sp. TA144; Mycobacterium sp.; Mycoplasma sp.; Neisseria sp.; Nitrosomonas sp.; Nostoc sp. PCC 7120; Novosphingobium aromaticivorans; Oenococcus oeni; Pantoea citrea; Pasteurella multocida; Pediococcus pentosaceus; Phormidium foveolarum; Phytoplasma sp.; Plectonema boryanum; Prevotella ruminicola; Propionibacterium sp.; Proteus vulgaris; Pseudomonas sp.; Ralstonia sp.; Rhizobium sp.; Rhodococcus equi; Rhodothermus marinus; Rickettsia sp.; Riemerella anatipestifer; Ruminococcus flavefaciens; Saccharomyces cerevisiae; Salmonella sp.; Selenomonas ruminantium; Serratia entomophila; Shigella sp.; Sinorhizobium meliloti; Staphylococcus sp.; Streptococcus sp.; Streptomyces sp.; Synechococcus sp.; Synechocystis sp. PCC 6803; Thermotoga maritima; Treponema sp.; Ureaplasma urealyticum; Vibrio cholerae; Vibrio parahaemolyticus; Xylella fastidiosa; Yersinia sp.; Zymomonas mobilis.

11. The method as claimed in claim 10, wherein, to provide the codogenic gene segment, bacteria from the genus *Agrobacterium* are transformed with the codogenic gene segment.

12. The method as claimed in claim 11, wherein the bacterium is *Agrobacterium tumefaciens.*

13. The method as claimed in claim 10, wherein the codogenic gene segment is integrated into the genome of a plant by
b1) allowing agrobacteria which are transformed with the codogenic gene segment to act on the plant or suitable biological material thereof; and
b2) obtaining the transgenic plant or suitable biological material thereof.

14. The method as claimed in claim 13, wherein the agrobacteria are allowed to act *in planta.*

15. The method as claimed in claim 13, wherein, to obtain the transgenic plants or suitable biological material thereof, the seeds of the plants treated with agrobacteria are first obtained, planted and subjected to selective conditions, and obtaining the transformed plants or suitable biological material thereof.

16. A biological transgenic material which is obtainable in a manner known per se from a population of transgenic plants as claimed in any of claims 1 to 9 and which comprises at least 50% of all of the codogenic gene segments of the donor organism.

17. A collection of plasmid constructs which comprises a group of plasmid constructs, where a codogenic gene segment of a donor organism, but not a different codogenic gene segment of the donor organism, is integrated into each construct of the group, the collection being obtainable by
a) providing a codogenic gene segment of the donor organism;
b) integrating this codogenic gene segment into the genome of a plant; and
c) carrying out steps a) and b) for at least 50% of all of the codogenic gene segments of the donor organism on a suitable number of plasmid constructs, so that each of these plasmid constructs has one, but not a different one, of the codogenic gene segments,
and wherein the donor organism is selected among Acetobacter (subgen. Acetobacter) aceti; Acidithiobacillus ferrooxidans; Acinetobacter sp.; Actinobacillus sp; Aeromonas salmonicida; Agrobacterium tumefaciens; Aquifex aeolicus; Arcanobacterium pyogenes; Aster yellows phytoplasma; Bacillus sp.; Bifidobacterium sp.; Borrelia burgdorferi; Brevibacterium linens; Brucella melitensis; Buchnera sp.; Butyrivibrio fibrisolvens; Campylobacter jejuni; Caulobacter crescentus; Chlamydia sp.; Chlamydophila sp.; Chlorobium limicola; Citrobacter rodentium; Clostridium sp.; Comamonas testosteroni; Corynebacterium sp.;
Coxiella burnetii; Deinococcus radiodurans; Dichelobacter nodosus; Edwardsiella ictaluri; Enterobacter sp.; Erysipelothrix rhusiopathiae; Escherichia coli; Flavobacterium sp.; Francisella tularensis; Frankia sp. CpI1; Fusobacterium nucleatum; Geobacillus stearothermophilus; Gluconobacter oxydans; Haemophilus sp.; Helicobacter pylori; Klebsiella pneumoniae; Lactobacillus sp.; Lactococcus lactis; Listeria sp.; Mannheimia haemolytica; Mesorhizobium loti; Methylophaga thalassica; Microcystis aeruginosa; Microscilla sp. PRE1; Moraxella sp. TA144; Mycobacterium sp.; Mycoplasma sp.; Neisseria sp.; Nitrosomonas sp.; Nostoc sp. PCC 7120; Novosphingobium aromaticivorans; Oenococcus oeni; Pantoea citrea; Pasteurella multocida; Pediococcus pentosaceus; Phormidium foveolarum; Phytoplasma sp.; Plectonema boryanum; Prevotella ruminicola; Propionibacterium sp.; Proteus vulgaris; Pseudomonas sp.; Ralstonia sp.; Rhizobium sp.; Rhodococcus equi; Rhodothermus marinus; Rickettsia sp.; Riemerella anatipestifer; Ruminococcus flavefaciens; Saccharomyces cerevisiae; Salmonella sp.; Selenomonas ruminantium; Serratia entomophila; Shigella sp.; Sinorhizobium meliloti; Staphylococcus sp.; Streptococcus sp.; Streptomyces sp.; Synechococcus sp.; Synechocystis sp. PCC 6803; Thermotoga maritima; Treponema sp.; Ureaplasma urealyticum; Vibrio cholerae; Vibrio parahaemolyticus; Xylella fastidiosa; Yersinia sp.; Zymomonas mobilis.

18. The collection as claimed in claim 17, wherein the codogenic gene segments are linked operably to regulatory sequences.

19. The collection as claimed in claim 18, wherein the regulatory sequences are of plant origin.

20. The collection as claimed in any of claims 17 to 19, wherein the constructs are based on binary vectors.

21. A population of a host organism which contains the collection of plasmid constructs as claimed in any of claims 17 to 20.

22. A method for the functional study of a population according to any of claims 1 to 9, where
a) the population is provided;
b) if appropriate, the plants are exposed to stress conditions; and
b) a phenotypic trait or the metabolism of each plant of the population is compared with the phenotype or metabolism of a reference plant,
where none of the codogenic gene segments of the donor organism comprised by the population is integrated into the genome of the reference plant.

23. The method as claimed in claim 22, where the phenotypic trait is selected from among growth, color, morphology and flowering behavior of the plants.

24. The method according to any of claims 22 or 23, where the stress is abiotic or biotic.

25. The method as claimed in one of claims 22 to 24, where the stress is low-temperature stress or drought stress.

26. The use of The population of transgenic plants as claimed in any of claims 1 to 9 or of biological material derived therefrom as claimed in claim 16 for determining the function of the codogenic gene segments.

## Revendications

1. Population de plantes transgéniques, qui comprend un groupe de plantes transgéniques, où dans le génome de chaque plante transgénique du groupe est intégrée une section génique codogène d'un organisme donneur mais non une autre section génique codogène de l'organisme donneur, la population pouvant être obtenue en ce qu'on
a) met à disposition au moins une section génique codogène d'un organisme donneur;
b) intègre cette section génique codogène dans le génome d'une plante; et
c) réalise les étapes a) et b) pour au moins 50% de toutes les sections géniques codogènes de l'organisme donneur sur un nombre correspondant de plantes, de manière telle que chacune de ces plantes présente une, mais non une autre section génique codogène
et l'organisme donneur est choisi parmi Acetobacter (subgen. Acetobacter) aceti; Acidithiobacillus ferrooxidans; Acinetobacter sp.; Actinobacillus sp; Aeromonas salmonicida; Agrobacterium tumefaciens; Aquifex aeolicus; Arcanobacterium pyogenes; Aster yellows phytoplasma; Bacillus sp.; Bifidobacterium sp.; Borrelia burgdorferi; Brevibacterium linens; Brucella melitensis; Buchnera sp.; Butyrivibrio fibrisolvens; Campylobacter jejuni; Caulobacter crescentus; Chlamydia sp.; Chlamydophila sp.; Chlorobium limicola; Citrobacter rodentium; Clostridium sp.; Comamonas testosteroni; Corynebacterium sp.; Coxiella burnetii ; Deinococcus radiodurans; Dichelobacter nodosus; Edwardsiella
erobacter sp.; Erysipelothrix rhusiopathiae; Escherichia coli; Flavobacterium sp.; Francisella tularensis; Frankia sp. Cpl1; Fusobacterium nucleatum; Geobacillus stearothermophilus; Gluconobacter oxydans; Haemophilus sp.; Helicobacter pylori; Klebsiella pneumonsae; Lactobacillus sp.; Lactococcus lactis; Listeria sp.; Mannheimia haemolytica; Mesorhizobium loti; Methylophaga thalassica; Microcystis aeruginosa; Microscilla sp. PRE1; Moraxella sp. TA144; Mycobacterium sp.; Mycoplasma sp.; Neisseria sp.; Nitrosomonas sp.; Nostoc sp. PCC 7120; Novosphingobium aromaticivorans; Oenococcus oeni; Pantoea citrea; Pasteurella multocida; Pediococcus pentosaceus; Phormidium foveolarum; Phytoplasma sp.; Plectonema boryanum; Prevotella ruminicola; Propionibacterium sp.; Proteus vulgaris; Pseudomonas sp.; Ralstonia sp.; Rhizobium sp.; Rhodococcus equi; Rhodothermus marinus; Rickettsia sp.; Riemerella anatipestifer; Ruminococcus flavefaciens; Saccharomyces cerevisiae; Salmonella sp.; Selenomonas ruminantium; Serratia entomophila; Shigella sp.; Sinorhizobium meliloti; Staphylococcus sp.; Streptococcus sp.; Streptomyces sp.; Synechococcus sp.; Synechocystis sp. PCC 6803; Thermotoga maritima; Treponema sp.; Ureaplasma urealyticum; Vibrio cholerae; Vibrio parahaemolyticus; Xylella fastidiosa; Yersinia sp.; Zymomonas mobilis.

2. Population selon la revendication 1, **caractérisée en ce que** les sections géniques codogènes sont intégrées dans le génome du noyau.

3. Population selon la revendication 1 ou 2, **caractérisée en ce que** 1 à 5 copies sont intégrées par cellule de plante transgénique.

4. Population selon l'une quelconque des revendications 1 à 3, **caractérisée en ce que** les sections géniques codogènes sont flanquées d'un côté ou des deux côtés de séquences d'ADN-T.

5. Population selon l'une quelconque des revendications précédentes, **caractérisée en ce que** les sections géniques codogènes sont reliées fonctionnellement avec des sections régulatrices.

6. Population selon la revendication 5, **caractérisée en ce que** les sections régulatrices comportent des séquences codant pour des peptides signal et/ou de transit.

7. Population selon la revendication 7, **caractérisée en ce que** la population comprend au moins une autre plante, dans le génome de laquelle est intégrée une section génique codogène en combinaison avec une autre section génique codogène ou avec d'autres sections géniques codogènes de l'organisme donneur.

8. Population selon l'une quelconque des revendications précédentes, **caractérisée en ce que** les plantes transgéniques appartiennent au genre Arabidopsis.

9. Population selon l'une quelconque des revendications 1 à 7, **caractérisée en ce que** les plantes transgéniques appartiennent au genre Oryza.

10. Procédé pour générer une population de plantes transgéniques, dans lequel on
a) met à disposition au moins une section génique codogène d'un organisme donneur;
b) intègre cette section génique codogène dans le génome d'une plante; et
c) réalise les étapes a) et b) pour au moins 50% de toutes les sections géniques codogènes de l'organisme donneur sur un nombre correspondant de plantes, de manière telle que chacune de ces plantes, présente une, mais non une autre section génique codogène
et l'organisme donneur est choisi parmi Acetobacter (subgen. Acetobacter) aceti; Acidithiobacillus ferrooxidans; Acinetobacter sp.; Actinobacillus sp; Aeromonas salmonicida; Agrobacterium tumefaciens; Aquifex aeolicus; Arcanobacterium pyogenes; Aster yellows phytoplasma; Bacillus sp.; Bifidobacterium sp.; Borrelia burgdorferi; Brevibacterium linens; Brucella melitensis; Buchnera sp.; Butyrivibrio fibrisolvens; Campylobacter jejuni; Caulobacter crescentus; Chlamydia sp.; Chlamydophila sp.; Chlorobium limicola; Citrobacter rodentium; Clostridium sp.; Comamonas testosteroni; Corynebacterium sp.; Coxiella burnetii ; Deinococcus radiodurans; Dichelobacter nodosus; Edwardsiella terobacter sp.; Erysipelothrix rhusiopathiae; Escherichia coli; Flavobacterium sp.; Francisella tularensis; Frankia sp. Cpl1; Fusobacterium nucleatum; Geobacillus stearothermophilus; Gluconobacter oxydans; Haemophilus sp.; Helicobacter pylori; Klebsiella ctobacillus sp.; Lactococcus lactis; Listeria sp.; Mannheimia haemolytica; Mesorhizobium loti; Methylophaga thalassica; Microcystis aeruginosa; Microscilla sp. PRE1; Moraxella sp. TA144; Mycobacterium sp.; Mycoplasma sp.; Neisseria sp.; Nitrosomonas sp.; Nostoc sp. PCC 7120; Novosphingobium aromaticivorans; Oenococcus oeni; Pantoea citrea; Pasteurella multocida; Pediococcus pentosaceus; Phormidium foveolarum; Phytoplasma sp.; Plectonema boryanum; Prevotella ruminicola; Propionibacterium sp.; Proteus vulgaris; Pseudomonas sp.; Ralstonia sp.; Rhizobium sp.; Rhodococcus equi ; Rhodothermus marinus; Rickettsia sp.; Riemerella anatipestifer; Ruminococcus flavefaciens; Saccharomyces cerevisiae; Salmonella sp.; Selenomonas ruminantium; Serratia entomophila; Shigella sp.; Sinorhizobium meliloti; Staphylococcus sp.; Streptococcus sp.; Streptomyces sp.; Synechococcus sp.; Synechocystis sp. PCC 6803; Thermotoga maritima; Treponema sp.; Ureaplasma urealyticum; Vibrio cholerae; Vibrio parahaemolyticus; Xylella fastidiosa; Yersinia sp.; Zymomonas mobilis.

11. Procédé selon la revendication 10, **caractérisé en ce qu'**on transforme, pour la mise à disposition de la section génique codogène, des bactéries du genre Agrobacterium avec la section génique codogène.

12. Procédé selon la revendication 11, **caractérisé en ce que** la bactérie est l'Agrobacterium tumefaciens.

13. Procédé selon la revendication 10, **caractérisé en ce qu'**on intègre la section génique codogène dans le génome d'une plante, **en ce qu'**on
b1) laisse agir des agrobactéries, qui sont transformées avec la section génique codogène, sur la plante ou une matière biologique appropriée de celle-ci ; et
b2) extrait la plante transgénique ou la matière biologique appropriée de celle-ci.

14. Procédé selon la revendication 13, **caractérisé en ce qu'**on laisse agir les agrobactéries in planta.

15. Procédé selon la revendication 13, **caractérisé en ce que** pour l'extraction des plantes transgéniques ou de la matière biologique appropriée de celles-ci, on extrait d'abord les graines des plantes traitées avec les agrobactéries, on les sème et on les soumet à des conditions de sélection et on extrait les plantes transformées ou la matière biologique appropriée de celles-ci.

16. Matière transgénique biologique qui peut être obtenue de manière connue en soi à partir d'une population de plantes transgéniques selon l'une quelconque des revendications 1 à 9, et qui comprend au moins 50% de toutes les sections géniques codogènes de l'organisme donneur.

17. Collection de produits de construction plasmides, qui comprend un groupe de produits de construction plasmides, où dans chaque produit de construction du groupe est intégrée une section génique codogène d'un organisme donneur mais non une autre section génique codogène de l'organisme donneur, la collection pouvant être obtenue en ce qu'on
a) met à disposition au moins une section génique codogène d'un organisme donneur ;
b) intègre cette section génique codogène dans un produit de construction plasmide ; et
c) réalise les étapes a) et b) pour au moins 50% de toutes les sections géniques codogènes de l'organisme donneur sur un nombre correspondant de produits de construction plasmides, de manière telle que chacun de ces produits de construction, présente une, mais non une autre section génique codogène
et l'organisme donneur est choisi parmi Acetobacter (subgen. Acetobacter) aceti ; Acidithiobacillus ferrooxidans ; Acinetobacter sp. ; Actinobacillus sp ; Aeromonas salmonicida ; Agrobacterium tumefaciens ; Aquifex aeolicus ; Arcanobacterium pyogenes ; Aster yellows phytoplasma ; Bacillus sp. ; Bifidobacterium sp.; Borrelia burgdorferi; Brevibacterium linens; Brucella melitensis; Buchnera sp.; Butyrivibrio fibrisolvens; Campylobacter jejuni; Caulobacter crescentus; Chlamydia sp.; Chlamydophila sp.; Chlorobium limicola; Citrobacter rodentium; Clostridium sp.; Comamonas testosteroni; Corynebacterium sp.; Coxiella burnetii; Deinococcus radiodurans; Dichelobacter nodosus; Edwardsiella ictaluri; Enterobacter sp.; Erysipelothrix rhusiopathiae; Escherichia coli; Flavobacterium sp.; Francisella tularensis; Frankia sp. Cpl1; Fusobacterium nucleatum; Geobacillus stearothermophilus; Gluconobacter oxydans; Haemophilus sp.; Helicobacter pylori; Klebsiella pneumoniae; Lactobacillus sp.; Lactococcus lactis; Listeria sp.; Mannheimia haemolytica; Mesorhizobium loti; Methylophaga thalassica; Microcystis aeruginosa; Microscilla sp. PRE1; Moraxella sp. TA144; Mycobacterium sp.; Mycoplasma sp.; Neisseria sp.; Nitrosomonas sp.; Nostoc sp. PCC 7120; Novosphingobium aromaticivorans; Oenococcus oeni; Pantoea citrea; Pasteurella multocida; Pediococcus pentosaceus; Phormidium foveolarum; Phytoplasma sp.; Plectonema boryanum; Prevotella ruminicola; Propionibacterium sp.; Proteus vulgaris; Pseudomonas sp.; Ralstonia sp.; Rhizobium sp.; Rhodococcus equi; Rhodothermus marinus; Rickettsia sp.; Riemerella anatipestifer; Ruminococcus flavefaciens; Saccharomycese cerevisiae; Salmonella sp.; Selenomonas ruminantium; Serratia entomophila; Shigella sp.; Sinorhizobium meliloti; Staphylococcus sp.; Streptococcus sp.; Streptomyces sp.; Synechococcus sp.; Synechocystis sp. PCC 6803; Thermotoga maritima; Treponema sp.; Ureaplasma urealyticum; Vibrio cholerae; Vibrio parahaemolyticus; Xylella fastidiosa; Yersinia sp.; Zymomonas mobilis.

18. Collection selon la revendication 17, **caractérisée en ce que** les sections géniques codogènes sont reliées fonctionnellement avec des sections régulatrices.

19. Collection selon la revendication 18, **caractérisée en ce que** les séquences régulatrices sont végétales.

20. Collection selon l'une quelconque des revendications 17 à 19, **caractérisée en ce que** les produits de construction sont basés sur des vecteurs binaires.

21. Population d'un organisme hôte, qui comporte la collection de produits de construction plasmides selon l'une quelconque des revendications 17 à 20.

22. Procédé pour l'étude fonctionnelle d'une population selon l'une quelconque des revendications 1 à 9, dans lequel on
a) met à disposition la population;
b) soumet le cas échéant les plantes à des conditions de stress ; et
c) compare une caractéristique phénotypique ou, selon le cas, le métabolisme de toutes les plantes de la population avec le phénotype ou, selon le cas, avec le métabolisme d'une plante de référence,
où dans le génome dans les plantes de référence n'est intégrée aucune des sections géniques codogènes de l'organisme donneur comprises dans la population.

23. Procédé selon la revendication 22, où la caractéristique phénotypique est choisie parmi la croissance, la couleur, la morphologie et le comportement de floraison des plantes.

24. Procédé selon l'une quelconque des revendications 22 ou 23, où le stress est abiotique ou biotique.

25. Procédé selon l'une quelconque des revendications 22 ou 24, où le stress est un stress par le froid ou un stress par la sécheresse.

26. Utilisation d'une population de plantes transgéniques selon l'une quelconque des revendications 1 à 9 ou d'une matière biologique dérivée de celle-ci selon la revendication 16 pour la détermination de la fonction des sections géniques codogènes.
